# EUROPEAN PATENT APPLICATION

(11) **EP 2 444 504 A1**
(43) Date of publication of application: **25.04.2012**
(21) Application number: 10306143.8
(22) Date of filing: 20.10.2010
(51) Int. Cl.: C12Q 1/68

(54) **Use of specific genes or their encoded proteins for a prognosis method of classified lung cancer**

(71) Applicant: Université Joseph Fourier, 38041 Grenoble Cedex 09 (FR)
(72) Inventor: Pison-Rousseaux, Sophie, 38410 Saint Martin D'Uriage (FR); Khochbin, Saadi, 38240 Meylan (FR)
(74) Representative: Grosset-Fournier, Chantal Catherine

(57) **Abstract**

The present invention relates to the use of at least one element for the implementation of a prognosis method, preferably *in vitro,* of the survival rate of a patient afflicted by a lung cancer, said lung cancer being previously classified as a lung tumour belonging from the group consisting of ADK, SQC, BAS et LCNE.

SEQUENCE LISTING

<110>  Université Jospeh Fourier

<120>  USE OF SPECIFIC GENES OR THEIR ENCODED PROTEINS FOR A PROGNOSIS
       METHOD OF CLASSIFIED LUNG CANCER

<130>  IOB 10 BF UJF GLUN

<160>  69

<170>  PatentIn version 3.5

<210>  1
<211>  240
<212>  DNA
<213>  Homo Sapiens

<400>  1
atgtgttgca actactacgg caactcctgt ggctatggct ccggctgtgg ctgtggctat        60

ggctctggct ctggctgtgg ctgtggctat ggaactggct atggctgtgg gtatggctgt       120

gggtttggct cccattatgg ctgtggttat ggaactggct atggctgtgg atatggctct       180

ggctctggct actgtggcta ccggccattt tgctttagaa gatgctattc ttcctgctaa       240


<210>  2
<211>  1405
<212>  DNA
<213>  Homo Sapiens

<400>  2
atcttaagag gcgttccttt ttgcatagtt cccatgagca tgagagaaga agcaatgcac        60

gctccggcag attcctagga accaaatacc tctgaggagc accagatttc agcttatggg       120

atgctttgat tgctctgtgg ctgcatttag gagaaggaag ctgcagtcat gcgtcatcac       180

tgccagcctc acatctcttg acagttaaag ccttagggtg gagcaaggga aaatttaaaa       240

taacaaatga agcaaaagca agaggtgatg ttccaaagca gaggaaggct aagtttatat       300

atacaaatgt caagtgtgta tagtgcaaaa ctaggaccag ttggtggaat ctgtggtcaa       360

aaacaaaagc cttccttttt ttttttcaag gcccagtccc aagacgcaag accacttgcg       420

ccagcagcgt gcatcagcaa gatagcaaaa gcaggacgag agctgcccgg aagacatcta       480

cctggccaga agacacctac cctggccgga agacatgtac ccctgaagat agagaaagag       540

gccatcgtgt actacgtagc agtcatgtca gactgggaca cttcctgttt acagaggact       600

ataaaacccc tgtcctgtcc tcacttgggg ctgacgccat cttaggcctc agcccgcctg       660

cagccaggcg ttcgttaaaa cagcatgttg ctccacaccg ccttgtattg tttgttggtc       720

ccactctctg ggctcgaacc aatacaagca cctttcaagc agtatattct tcagtgtctt       780

gatcctccaa ataactctct tctaattcct cctgaccaca aaaagcactt atactctagg       840

atgactgatt ccagcccagt ggcctggcaa gggtgaatta caccttgcat atcacactct 900

tgacatttgt gtgcgctagc ataagaatta taattgaaac agggatttaa gtatctcctc 960

tctaggtgcc taccctcctt ggactcaggt caaatttatt aaaggaagtt ttgtttctag 1020

ataggttgtt tgaaataaaa taacagaatg ttcaagtaac acagtgtacc tacagctttt 1080

aacaaaattg aggacttggg tctcgaaaca atttcctttg attttcaggt attttatcta 1140

taaaaaggga gataaagcat tagttcatag gacagttata tgtttaaatg tgataatgta 1200

tattaaccac cttgcatgta ttcaaatgtg ttttgaaatc taacgtctac attttgatag 1260

tttaactctt ctacataagt gacttacaac aggcattaaa tattgtttgg cattttcata 1320

tatctgtaac tgtatcttaa tctacaatga gcttaatttt aagtgtagca taaaacagaa 1380

ccttcaataa agtggtaata ttagg 1405

<210> 3
<211> 857
<212> DNA
<213> Homo Sapiens

<400> 3
gcttctccca gctggagtag gtgggggagg ccagacatgg aggcccttcc tccagtcaga 60

tccagccttt tggggatcct gttgcaggtt acgaggctct cagtgctgtt ggttcagaac 120

cgagatcacc tctataattt cctgctcctc aagatcaacc tcttcaacca ctgggtgtca 180

gggctggccc aggaggcccg ggggtcctgt aactggcagg cccacctacc cctgggagct 240

gcagcctgcc ccctgggcca ggctctctgg gctgggctgg ctctgataca ggtccccgta 300

tggctggtgc tacagggacc caggctgatg tgggctggca tgtggggcag caccaagggc 360

ctgggcctgg ccttgctcag tgcctgggag cagctgggcc tgtctgtggc catctggaca 420

gatctgtttt tgtcatgtct gcacggcctg atgttggtgg ccttgctctt ggtggtagtg 480

acctggaggg tgtgtcagaa gtcccactgc ttccgactgg gcaggcagct cagtaaggcc 540

ttgcaagtga actgcgtggt aaggaagctc ctggtacagc tgagacgtct gtattggtgg 600

gtggagacta tgactgccct cacctcctgg cacctggcct atctcatcac ctggaccacc 660

tgcctggcct cccacctgct gcaggctgcc tttgagcaca cgacccagct ggccgaggcc 720

caggaggttg aaccccagga ggtctcaggg tcttccttgc tgccctcact gtctgcgtcc 780

tcggactcag agtctggaac agttttgcca gagcaagaaa ctcccagaga ataaatgtat 840

ccccatctgc ctctaaa 857

<210> 4
<211> 2521

<212>  DNA
<213>  Homo Sapiens

<400>  4
aaacgagtgg agacacgagg accagcgcga gcggtcccgg tgggctaccc tccccctgcg      60

acgacccccc ctcgctctga ccgactggtc ccctaaacgg tggcggcggt ttttggtcgt      120

tgggccccgg gatttaggac caacatttga agacccgaag gggaactgca accatgaatg     180

aagaaaatat agatggaaca aatggatgca gtaaagttcg aactggtatt cagaatgaag     240

cagcattact tgctttgatg gaaaagactg gttacaacat ggttcaggaa aatggacaaa     300

ggaaatttgg cggtcctcct ccaggttggg aaggtccacc tccacctaga ggctgtgaag     360

tttttgtagg aaaaatacct cgtgatatgt atgaagatga gttagttcct gtatttgaaa     420

gagctgggaa gatatatgaa tttcgactta tgatggaatt tagtggtgaa aatcgaggtt     480

atgcttttgt gatgtacact acaaaagaag aagcccaatt agccatcaga attcttaata     540

attatgaaat tcgaccaggg aagtttattg gtgtgtgtgt aagcctggat aattgtagat     600

tatttattgg agctattccc aaggaaaaga agaaagaaga aattttagat gaaatgaaga     660

aagttacaga aggagttgta gatgtcattg tttatccaag tgcaactgat aagaccaaaa     720

atcgtggttt tgcatttgtg gaatatgaat ctcacagagc tgctgctatg gcaaggagga     780

aactaattcc aggaacattc caactatggg gccacaccat tcaggtagat tgggctgacc     840

cagagaaaga ggtggatgag gaaaccatgc agagagttaa agttctttat gtaagaaatt     900

taatgatctc aactacagag gaaacaatta aagcagaatt caataaattt aagcctggtg     960

cagttgaacg ggtaaagaaa cttagagatt atgcttttgt tcactttttc aaccgagaag    1020

atgcagtggc tgccatgtct gttatgaatg gaaaatgcat tgatggagca agtattgagg    1080

taacactagc taaaccagta aataaagaaa acacttggag acagcatctt aatggtcaga    1140

ttagtccaaa ttctgaaaat ctgattgtgt ttgctaacaa agaagagagc cacccaaaaa    1200

ctctaggcaa gctgccaact cttcctgctc gtctcaatgg tcagcatagc ccaagtccgc    1260

ctgaagttga aagatgcact tacccttttt atcctggaac aaagcttact ccaattagta     1320

tgtattcttt aaaatccaat cattttaatt ctgcagtaat gcatttggat tattactgca    1380

acaaaaataa ctgggcacca ccagaatatt atttatattc aacaacaagt caagatggga    1440

aagtactctt ggtgtataag atagttattc ctgctattgc aaatggatcc cagagttact    1500

tcatgccaga caaactctgt actacgttag aagatgcaaa ggaactggca gcccagttta    1560

cattacttca tttggactac aatttccatc gcagctcaat aaatagtctt tcccctgtta    1620

gtgctaccct ctcttctggg actcccagcg tgcttcctta tacttcaagg ccttattctt    1680

atccaggcta tcctttgtca ccaacaatat cacttgctaa tggcagccat gttggacagc    1740

ggctatgtat ctccaatcag gcctccttct tctgaagaaa atactaacat tagtatgaaa 1800

atttgtgtaa atttgtagta tgaaaacttg caaattaaaa tattgtttta ttttagaatc 1860

gggtttgcat atttggtttt aaaaaggtat ttattccaaa gtactaaaca tcagctataa 1920

ttcagaataa catggagttg tagaatttat aaaaatgcaa agtttaaaaa gttattcagt 1980

ggtttctctt gataaaggta cagcaaacta ctattctttt taaacttcta ggattttctt 2040

ctactttctg agtgggcaat agaacctagt catttatgtt tttttttttt tttgcataat 2100

tttactaaat agtatttcac aaatattaaa gcacttgaag acaatggtta tagtagattt 2160

gattaccaag gatcactatc tgtactggag attagaacaa ttatatgacc agaagcatct 2220

aaccattatg taaaaagaaa tgatgagaca aaaagattaa gatacaaatt ttgtgcagta 2280

ctaaagaaaa agcagtctac cattgtggtc cttgaaaata actatagata tttttgttat 2340

ttgttagaca caaattataa ttttgttgtt aatgtattta agcattttat agttatgctt 2400

tgtgtttttg atattctttg tattgttaat aacaagtgtt atgggttttt aatgttgaaa 2460

tcatgtgtta atttttgtac ttgaattcaa attttttgac attaaatatg tgatgcttct 2520

a 2521

<210> 5
<211> 3305
<212> DNA
<213> Homo Sapiens

<400> 5
cacaggcagg ccgtacctcg aaatcccagc ggggacacct gtcagggaga cgcaccctca 60

caccgcccat gcatgcccga cgcatctttg ggatccttag ggatcacgtg gtgcttcctt 120

gaatctccgc ttgaggtttc ttcaggcagg ttcggccttg cccgactcct aggatcgcag 180

gaccatggcg acgatcccgc ggagagggga cgcactgcca cagacgcctg ggggccaagc 240

cgctggggcc agtcccccgg caacggtggc ggttactgtg acgcgagtcc cccctcagcg 300

ctagcgcctg gtgatcgcgc ctgggctctt cctgcgtctc caagctcagg ggctcctgct 360

tctcagcact gctgtctgga aaaggcggga acgaggacta aggcaagccc agtgtggggc 420

agagatggca acacctggaa ctagcttttc atgggggcag ataatgtgga gagtgtccca 480

attacatctc cctttattct atcccatact actaaacccc accagctatt tgcctacaaa 540

ctgtgtatca gtcaggacat caggacacca agataaagga ctagagtttg gtaatcaact 600

actggctcct ccagcagctc agaatgtggc ctgtatgcat cacttcactt aattcattca 660

agaaatgctg actgagttcc tgctgtgtgt tggacacttt cttagaagtg ggacacactg 720

cagtgtgtgg gacaaaggct gggccgctca ttgcttatgt tctagttgaa gagagagtca 780

atcaacaaca ttctactttc agacattgat aaatgctgtc atgaaaagac aaggaacatg 840

aaaaggtaag aaaagggatg gagagagatg ggatgagggg tgcctgggca gacaatgcaa 900

tgatctggca aaagagtagg acaatcatga gactgcatgt gttccagttt ctgctgctgt 960

aaaacagcat ccacaggtta catctttact attctgttac atacatacta ttatttaaat 1020

aaaaaatagt actagttaac tgtgaaaatt gcaattaata gggcagatta aacaaatcct 1080

ttaaatttaa acaaatgttt tatggttttt tatcctctga tacattgttt caactttaaa 1140

aaaccctctt tatttgatat ttaaactcta tattaatgta tattcttaag atacataaat 1200

ttaagatttt tttcgagaca gggtctcatt ctgtcacaca gtctggagtg ctgtggtgca 1260

attatggttc actgcagact ccacctctcc aggctcagat aatcctcctg cctcagactc 1320

ctgagtatct gggaccacag gtgtgcatca ccacacccag ctaatttctt gtattttttt 1380

gttgggatgg ggttttgcga tgttgcccag gctggtctca aactcctggg ctcaagtgat 1440

ctgcctgcct tggtgtctca aagtgttggg atcacaggcg tgagccacca catccagcct 1500

aatttaagat tttttaaaag cagaatgtaa atatggtcat gcattgtgta attctgtgga 1560

tactttctga gtaatgcatg gttaggtgac ttcatcatta tgtgaacatt atagagtgta 1620

catacacaaa cctagatgat gtagcctgat ggtacagcct atcacttcga ggctacaaat 1680

ccaaattgcc tggatactgt aaacaattat actcaatggt atttgtgtat ctaaacatgt 1740

ctaaacatag aaagggtaca gtaaaaatat ggtataaaag ataagaaatc gtataccggt 1800

atacagcact taccgtggat ggagcttgca agactggaag ttactccggg tgagtcagtg 1860

actgagcggt gagtgaatgt gaaggctagg gcagtactgt atactactgt agactttata 1920

tgtactgtat gcttaggcta cactaaattt atttgaattt ttttgtcggt aataaattaa 1980

ccttagctta ctataatttc tttataaacc ttttaatttt taaaaatgtt ttgactctct 2040

tgtaataaat tagcttcaaa cacattgtat agctgtacac aaatattttc tttatatcct 2100

tattctataa tcttctactt aaaaattttt ttaatttttt tattttaatt taattttatt 2160

ttattttgag acagagtctc actctctcac ccaggctgga gtgcagtggc gtgatcttaa 2220

ctcactgcaa cctccgcttc ccaggtaata ttatttaaat aaaaatagta ctagttaact 2280

gtgaaaattg caattaatag ggcagatcaa gcaattctct tgcctcagcc tccagacttt 2340

tttgttaaaa actaagacac aaacacatta gcctaggtac acacagtgcc aggatcatca 2400

aaatgtcatt aggcagtata aacttttcag ctccataaaa gttggaaaaa aaataataat 2460

aataaataca attttataaa aagaaaattt tcagctctgc tataatctta tgggaccact 2520

actgtatatg tggtccctca ttgaccaaaa catcattatg tggcacatga ttataacaaa 2580

tgatggatgg cgaggttgtg aagaaactgg aacccttgtg cattactggt tgtaatataa 2640

aaaggtacag ccactgtaaa aaaaaaaaca cagtttggtt atttcttcat aagttaatca 2700

tagaattacc atataatcca gcaatttcac ttctaggtat atgcccaaaa taattgaata 2760

aggtgtttaa acataaactt gtacaagaat gttcatagca gtacatttca caatactcag 2820

ataatcctct cattggaagc aactcaaatg tccaccaact gatggtatta cccatgcaat 2880

ggaatattat tcagtcataa aaaggaatga agtattgagg catgttacaa catgaaggaa 2940

ctttgaggac agtttgttaa tacgctatat actgtgtaat tctatttata tgaaatatcc 3000

agaataggta aatcattaga gactagagac agcagattag tggatgccag gagtttgggg 3060

aaggaagggg tgggaagaat gggaagtgac tgcttaatgg atacagggtt tctttttggg 3120

gtgatgaaaa atttctggaa ctagattgta gtgatggttg cacaacaccg taagtgtaat 3180

aatgtcactg aattatacac tttaaagtga tttaaatggt aaattgtatt atttatgtat 3240

tttaccacaa taaagaagtc acctatcaaa aaaaaaaaaa aaaaagaaaa aaaaaaaaaa 3300

aaaaa 3305

<210> 6
<211> 2499
<212> DNA
<213> Homo Sapiens

<400> 6
cttctaattc tgttattgca actgcagacc gttacctggt acgctggctg ctacctccct 60

cactcttgtc agagtcggag ctacaggcag tgccttcagc tctgagctca ggcatcccgg 120

tccctgtttt tgcggttaag gactctaaag tgttgtgtcg tgttcatcaa ctttttctca 180

acttccctgg ctctacctct tctgccacaa acgtcagcat ggtggtatct gccgaccctt 240

tgtccagcga gagggcagag atgaacatcc tagaaatcaa ccaggaattg cgctcgcagc 300

tggcagagag caatcagcag ttccgagacc tcaaagagaa attccttata actcaagcta 360

ctgcctactc cctggccaac cagctgaaga aatacaagtg tgaagagtac aaagacatca 420

tagactctgt gctgagggat gaactgcagt ccatggagaa gctggcagag aagctcaggc 480

aagctgagga gctcaggcag tataaagccc tggttcactc tcaggcaaaa gagctgaccc 540

agttacggga gaagttacgg gaagggagag atgcctcccg ctggctgaac aagcatctga 600

aaaccctcct cactcctgat gaccctgaca agtcccaggg tcaggacctc cgagagcagc 660

tggctgaggg gcacaggctg gcagagcacc ttgttcacaa gctgagccca gaaaatgatg 720

aagatgaaga tgaggatgaa gacgacaaag acgaggaggt tgagaaagta caggaatcac 780

ctgcccccag agaggtgcag aagactgaag aaaaggaagt ccctcaggac tcactggagg 840

aatgtgctgt cacttgttca aatagtcaca acccttctaa ctccaaccag cctcacagga 900

gcaccaaaat cacatttaag gaacacgaag tcgactctgc tctggttgta gagagtgaac 960

accctcatga tgaagaggag gaagctctaa acattccccc agaaaatcaa aatgaccatg 1020

aggaggagga ggggaaagcg ccagtgcccc ccagacacca tgacaagtcc aactcttacc 1080

ggcatcgtga agtctctttc ttggcattgg atgaacagaa agtttgctcc gctcaggatg 1140

ttgccaggga ttactccaat cccaaatggg atgaaacctc acttggcttc ctcgaaaagc 1200

aaagtgatct tgaagaggtg aaaggacaag aaacagttgc tcccaggctc agcaggggac 1260

cgctgagagt ggacaagcat gaaatccccc aggagtcact ggatggatgt tgcttgactc 1320

cttccatcct tcctgacctg actccctcct accaccctta ttggagcact ttgtactctt 1380

ttgaagacaa gcaagtcagc ttggctcttg tagacaaaat taaaaaggat caagaggaga 1440

tagaagacca aagcccacca tgccccaggc tcagccagga gctgccagag gtgaaggagc 1500

aggaagtccc agaggactct gtgaatgaag tttacttgac tccctcagtt caccatgacg 1560

tgtctgactg ccaccagcct tatagcagca ccttgtcctc attggaggat cagcttgcct 1620

gctctgctct ggatgtagcc tcccccaccg aggcggcctg tccccaaggg acttggagtg 1680

gagacttgag ccaccaccag tcagaggtgc aagtttcaca ggcacagctg gaaccaagca 1740

ccctggtgcc cagttgtctg cgactacagc tggatcaagg gttccactgt gggaacggct 1800

tggcccagcg gggcctttcc tccaccacct gcagcttctc agccaatgct gattctggga 1860

accaatggcc cttccaagag ctggttttag agccctctct ggggatgaag aaccctcccc 1920

agctggaaga tgatgcactt gaaggctcag caagcaacac acaagggcgt caagtcactg 1980

gccggattcg tgcctccctt gtcctgatac tgaagaccat cagaagaaga ctcccgttca 2040

gcaagtggag actggcattc agattcgctg gcccgcatgc tgagagcgca gagataccaa 2100

atactgctgg aaggacgcaa aggatggcag gatgaaagaa tgtcacaaaa agcagctttt 2160

ccacttgata aaaacaacta aaacagcaaa gcaagtttaa gtccaaacac aatactgcag 2220

gggtccttca ctgaggattg aatttcagac acagaatact cttgatgact tcaagccact 2280

atgctccttt gatttgagaa gccacattcc atccccctcc aattgtgatc aatacctagg 2340

gagaccaatg cccagatgga caaatagcat tgaccggcgt tagccctgtt tctcaattcc 2400

catcgtgtag agaacaggag tccgcagctg ctggcaggag acagcatgtc agccgggact 2460

ctgccagggc agagtatgag caatgccatg ttcttgctg 2499

<210> 7
<211> 6093
<212> DNA
<213> Homo Sapiens

<400> 7
acgccgccaa ctggcggggg tgcgggggag acaataattt gttccgcggt aataagaacg 60
gtgactgctg gccgtggatc catttcacag gcctgccttc tctcactaac gctcttccta 120
gtccccgggc caactcggac agtttgctca tttattgcaa cggtcaaggc tggcttgtgc 180
cagaacggcg cgcgcgcgcg cacgcacgca cacacacggg gggaaacttt tttaaaaatg 240
aaaggctaga agagctcagc ggcggcgcgg gcgctgcgcg agggctccgg agctgactcg 300
ccgaggcagg aaatccctcc ggtcgcgacg cccggccccg gctcggcgcc cgcgtgggat 360
ggtgcagcgc tcgccgccgg gcccgagagc tgctgcactg aaggccggcg acgatggcag 420
cgcgcccgct gcccgtgtcc cccgcccgcg ccctcctgct cgccctggcc ggtgctctgc 480
tcgcgccctg cgaggcccga ggggtgagct tatggaacca aggaagagct gatgaagttg 540
tcagtgcctc tgttgggagt ggggacctct ggatcccagt gaagagcttc gactccaaga 600
atcatccaga agtgctgaat attcgactac aacgggaaag caaagaactg atcataaatc 660
tggaaagaaa tgaaggtctc attgccagca gtttcacgga aacccactat ctgcaagacg 720
gtactgatgt ctccctcgct cgaaattaca cggtaattct gggtcactgt tactaccatg 780
gacatgtacg gggatattct gattcagcag tcagtctcag cacgtgttct ggtctcaggg 840
gacttattgt gtttgaaaat gaaagctatg tcttagaacc aatgaaaagt gcaaccaaca 900
gatacaaact cttcccagcg aagaagctga aaagcgtccg gggatcatgt ggatcacatc 960
acaacacacc aaacctcgct gcaaagaatg tgtttccacc accctctcag acatgggcaa 1020
gaaggcataa aagagagacc ctcaaggcaa ctaagtatgt ggagctggtg atcgtggcag 1080
acaaccgaga gtttcagagg caaggaaaag atctggaaaa agttaagcag cgattaatag 1140
agattgctaa tcacgttgac aagttttaca gaccactgaa cattcggatc gtgttggtag 1200
gcgtggaagt gtggaatgac atggacaaat gctctgtaag tcaggaccca ttcaccagcc 1260
tccatgaatt tctggactgg aggaagatga agcttctacc tcgcaaatcc catgacaatg 1320
cgcagcttgt cagtggggtt tatttccaag ggaccaccat cggcatggcc ccaatcatga 1380
gcatgtgcac ggcagaccag tctgggggaa ttgtcatgga ccattcagac aatccccttg 1440
gtgcagccgt gaccctggca catgagctgg gccacaattt cgggatgaat catgacacac 1500
tggacagggg ctgtagctgt caaatggcgg ttgagaaagg aggctgcatc atgaacgctt 1560
ccaccgggta cccatttccc atggtgttca gcagttgcag caggaaggac ttggagacca 1620
gcctggagaa aggaatgggg gtgtgcctgt ttaacctgcc ggaagtcagg gagtctttcg 1680
ggggccagaa gtgtgggaac agatttgtgg aagaaggaga ggagtgtgac tgtggggagc 1740
cagaggaatg tatgaatcgc tgctgcaatg ccaccacctg taccctgaag ccggacgctg 1800

tgtgcgcaca tgggctgtgc tgtgaagact gccagctgaa gcctgcagga acagcgtgca 1860

gggactccag caactcctgt gacctcccag agttctgcac aggggccagc cctcactgcc 1920

cagccaacgt gtacctgcac gatgggcact catgtcagga tgtggacggc tactgctaca 1980

atggcatctg ccagactcac gagcagcagt gtgtcacgct ctggggacca ggtgctaaac 2040

ctgcccctgg gatctgcttt gagagagtca attctgcagg tgatccttat ggcaactgtg 2100

gcaaagtctc gaagagttcc tttgccaaat gcgagatgag agatgctaaa tgtggaaaaa 2160

tccagtgtca aggaggtgcc agccggccag tcattggtac caatgccgtt tccatagaaa 2220

caaacatccc cctgcagcaa ggaggccgga ttctgtgccg ggggacccac gtgtacttgg 2280

gcgatgacat gccggaccca gggcttgtgc ttgcaggcac aaagtgtgca gatggaaaaa 2340

tctgcctgaa tcgtcaatgt caaaatatta gtgtctttgg ggttcacgag tgtgcaatgc 2400

agtgccacgg cagaggggtg tgcaacaaca ggaagaactg ccactgcgag gcccactggg 2460

cacctccctt ctgtgacaag tttggctttg gaggaagcac agacagcggc cccatccggc 2520

aagcagataa ccaaggttta accataggaa ttctggtgac catcctgtgt cttcttgctg 2580

ccggatttgt ggtttatctc aaaaggaaga ccttgatacg actgctgttt acaaataaga 2640

agaccaccat tgaaaaacta aggtgtgtgc gcccttcccg gccaccccgt ggcttccaac 2700

cctgtcaggc tcacctcggc caccttggaa aaggcctgat gaggaagccg ccagattcct 2760

acccaccgaa ggacaatccc aggagattgc tgcagtgtca gaatgttgac atcagcagac 2820

ccctcaacgg cctgaatgtc cctcagcccc agtcaactca gcgagtgctt cctcccctcc 2880

accgggctcc acgtgcacct agcgtccctg ccagacccct gccagccaag cctgcactta 2940

ggcaggccca ggggacctgt aagccaaacc cccctcagaa gcctctgcct gcagatcctc 3000

tggccagaac aactcggctc actcatgcct tggccaggac cccaggacaa tgggagactg 3060

ggctccgcct ggcacccctc agacctgctc cacaatatcc acaccaagtg cccagatcca 3120

cccacaccgc ctatattaag tgagaagccg acaccttttt tcaacagtga agacagaagt 3180

ttgcactatc tttcagctcc agttggagtt ttttgtacca acttttagga ttttttttaa 3240

tgtttaaaac atcattacta taagaacttt gagctactgc cgtcagtgct gtgctgtgct 3300

atggtgctct gtctacttgc tcaggtactt gtaaattatt aatttatgca gaatgttgat 3360

tacagtgcag tgcgctgtag taggcatttt taccatcact gagttttcca tggcaggaag 3420

gcttgttgtg cttttagtat tttagtgaac ttgaaatatc ctgcttgatg ggattctgga 3480

caggatgtgt ttgctttctg atcaaggcct tattggaaag cagtccccca actaccccca 3540

gctgtgctta tggtaccaga tgcagctcaa gagatcccaa gtagaatctc agttgatttt 3600

ctggattccc catctcaggc cagagccaag gggcttcagg tccaggctgt gtttggcttt 3660
cagggaggcc ctgtgcccct tgacaactgg caggcaggct cccagggaca cctgggagaa 3720
atctggcttc tggccaggaa gctttggtga gaacctgggt tgcagacagg aatcttaagg 3780
tgtagccaca ccaggataga gactggaaca ctagacaagc cagaacttga ccctgagctg 3840
accagccgtg agcatgtttg gaaggggtct gtagtgtcac tcaaggcggt gcttgataga 3900
aatgccaagc acttcttttt ctcgctgtcc tttctagagc actgccacca gtaggttatt 3960
tagcttggga aaggtggtgt ttctgtaaga aacctactgc ccaggcactg caaaccgcca 4020
cctccctata ctgcttggag ctgagcaaat caccacaaac tgtaatacaa tgatcctgta 4080
ttcagacaga tgaggctttc catgggacca caactatttt cagatgtgaa ccattaacca 4140
gatctagtca atcaagtctg tttactgcaa ggttcaactt attaacaatt aggcagactc 4200
tttatgcttg caaaaactac aaccaatgga atgtgatgtt catgggtata gttcatgtct 4260
gctatcatta ttcgtagata ttggacaaag aaccttctct atggggcatc ctctttttcc 4320
aacttggctg caggaatctt taaaagatgc ttttaacaga gtctgaacct atttcttaaa 4380
cacttgcaac ctacctgttg agcatcacag aatgtgataa ggaaatcaac ttgcttatca 4440
acttcctaaa tattatgaga tgctggcttg ggcagcatcc ccttgaactc ttcactcttc 4500
aaatgcctga ctagggagcc atgtttcaca aggtctttaa agtgactaat ggcatgagaa 4560
atacaaaaat actcagataa ggtaaaatgc catgatgcct ctgtcttctg gactggtttt 4620
cacattagaa gacaattgac aacagttaca taattcactc tgagtgtttt atgagaaagc 4680
cttcttttgg gggtcaacag ttttcctatg ctttgaaaca gaaaaatatg taccaagaat 4740
cttggtttgc cttccagaaa acaaaactgc atttcacttt cccggtgttc cccactgtat 4800
ctaggcaaca tagtattcat gactatggat aaactaaaca cgtgacacaa acacacacaa 4860
aagggaaccc agctctaata cattccaact cgtatagcat gcatctgttt attctatagt 4920
tattaagttc tttaaaatgt aaagccatgc tggaaaataa tactgctgag atacatacag 4980
aattactgta actgattaca cttggtaatt gtactaaagc caaacatata tatactatta 5040
aaaaggttta cagaatttta tggtgcatta cgtgggcatt gtctttttag atgcccaaat 5100
ccttagatct ggcatgttag cccttcctcc aattataaga ggatatgaac tgagtttttc 5160
ttttgttgtt tgttcttagc tgtaattcct atgcttctat ttcagagagc caggagagtt 5220
tgatattaaa ggaggttaaa actgtgatct tatgccatgt catcaatggc cacttagggg 5280
ccatggctga tgacacattc ttatctctac agtactaatg tgttattata gagccatgca 5340
ttttatttct gaataagaac atatttaaac taatattccc ttacaatatg gacagtatta 5400
atccttccaa gatgcagtat ttatcaagtg aagcatattt agcagcaaat tccattttaa 5460

cataacttag gaaccaataa ccagggtgtt ttgtggttgg gggaggcacg gggtggagta 5520

ttctttttta tatcctcaaa acaaaaaaaa tcaatactta tatttcaatg gcaatctagt 5580

atttttttaa aagactgtat aggcatgaat aatagaggtg gtttgagttt tgtagggcca 5640

tcacctggaa agtcaatgtg actagacaca aagtagccca gaggctactt ttcttcctac 5700

agcttattat agttgtaggt tctatgacct cacttcatgg gttccaggca attccgctga 5760

aaggtttgtc tcctgaaatt ttttaagttt gttttcctga cacatgtaat cagatgtgta 5820

gcaaccgagg gaaacgaagc ctaacattct ccattgtgga aatacacaca ggaggttaca 5880

tttcacagcg tggatttttc cagcttacac atgtgggatg acatcacaga aaccacaaaa 5940

gcagcaaatt aaactgtagg agagtcaata ctcctgacga gtctcggggg gggggcattt 6000

ttatgccttc ttaactttat gagaattctc aggctgaact ataggccatt gttcccaggc 6060

aaatcaatac atcaatgcat cctcaaaaaa aaa 6093

<210> 8
<211> 2714
<212> DNA
<213> Homo Sapiens

<400> 8
ggggctggca ttcagtgagg ctgggggaga agcttcctaa aagcattgcc attgaaaagc 60

agggcatacc tatctgatac tgaggatgta gaatgcagtt tggagatgct tcctagctac 120

ctaatcttcc aaggcaaacc atgcactctt catcctgata tttgtgatgc ctccctgggc 180

tacatgacag agggtgcact gcaaaggatg tgaaggaaag tcaccacagg gcaaaggtgg 240

aaacccaggg gcctttcccc agtgagactt tctggctgtt cctttggaac ttccacctgc 300

tagttgcaga tgtttgccac tggatggggc cccatgctac gtgggaactg ttcaggactg 360

tgggtactta gttccagcag agctggctcc accaggcacc caggactgca ggcttctcaa 420

ctcctggcct ggagaggcag cttcagcaac cagtgcctgg gaatccacta gccctttctc 480

tgtgagtcac gtgagggcag aatctgctca aagtcagagg gctggagcaa cctggattgt 540

gccagagtga gggcccagct cactggaggt cagagtaaga gagtcaagga tgggaagaaa 600

cagggttttg ctcagtcttg cagatactga ggcactcagg aggctcagga tggcagcaac 660

agggcatgtg ctaaacttac cctagcctct tccttttagg cagccaccac agaggacatc 720

tgtttgcctc tgtaccctac cccatctcca tgctggtaga tatagtctgg ggataccttt 780

aaaacaaaca aacaaacaaa caaaacagga tctgtctatc acctgtccaa atcctgaagc 840

aaaatgagcc ttctttatgg ccaccttgga aaggcctgag tccccagcat cacctgggat 900

acaaagtgtt ctgaggtggg ggcagagtgc cctcctgccc agatgccaca ctccacaata 960

tctcttcctg gcagacaggt ttctcttaac ttcctttggc aaatttggtt tcacttcctt 1020

ttgcaacttt agaacctttc tcaggtcagt cataaggtag tggggaagcc aggatttgaa 1080

cccattctgt ctgattccag agctcatgca ttaaccacct gactgtggtg cctccccatc 1140

atatgtgcat caagtatgat catttttgta gttctcagag tacttagcac catgcacatc 1200

tgtaataagt acagccactg cctactggga ggtctaaagg ttcaactcat aagggaccca 1260

caggccacac tgggctccag tctccttaaa agatgtggga cggagatcaa caattgtcag 1320

cagaacccca tcaggcattt gtcttctcac tgagccttgc agcaaaccac acagctgtct 1380

ataacctatc tgttcccaga ttcaagaaca caagctgctc atcagttggg tgcaggatcc 1440

actctagtac aaaagctgtg tgtctcaagg cataattcct tatgattact tcatagctct 1500

gcttttgtct tgagacaaaa gaaatttcct tcaattccta cttttgctga gatattgttt 1560

tttgaaaagc aattaaatct tgtcaatcac ttttcccacc tctttgagat acttacatgg 1620

cttttcttct ttattctctt agtgtggtaa attacattga tttattttaa atatgaaacc 1680

agccttgaat tccaggaaca aactccattt ggacatgcta gaacaacctt ttatatttgt 1740

ttagatttga tttcctgaag tggtgttaaa aggttttcat atctatgtct gtgagggata 1800

ttaatctgta cattttttta ttactatttt ctggtgttat ggtaatgcta gtctcataga 1860

atgaattagg aaaagttgcc tccttctgta ttttctgaga gtttgtgtgg cactggtaaa 1920

atattttctg tatatgtttg acataattca ccaggaaaat tatttgtacc tacaagttcc 1980

ctcctggaaa gatgttgata atcacttatt atcctttcat tgtctgccag atctgtggca 2040

ttgctttttt ctctggtatt agtaatttaa attttccctt atttcttgat caattttggc 2100

agagatttat caattttatt aatctctttt aaaaatctgt attgagcttt gttaattttc 2160

tctattacat actttttatt tctactctta cgattgctat ttctttattt ctccttcgga 2220

tttagcccat ttttctattt gtctaatttt aggatttaaa attcaatcat tatttttaga 2280

tatttttatt tttaaatata aacatgaaag ttgcaatatt accttgggca ctgttttgac 2340

tgcattccac aagtttttat ttgtcatgtt ttaatcacca ttcagttcaa aatactttct 2400

aatgctcctt gttatttctt ctttgaccca ttggttatat aaaagtacgt tttgctttat 2460

ttctaaatat gtgaagactt tctagatatc tcagttattt attactaatt taattttatt 2520

gtagccagag aaaatacact gtatgtgttc aatctttgga aatttcttga gacttatttt 2580

ttgacccagt atgtttgccc tcatggacat tccatattca ctagaaaaag aatgtgtgtt 2640

caaataatgt tggatatagt gttatgcaaa tgccactaaa tcaagataaa tcttttgcaa 2700

aaaaaaaaaa aaaa 2714

<210>  9
<211>  3280
<212>  DNA
<213>  Homo Sapiens

<400>  9
gggggaggga tgactaaaga caacggctgt aagagaactc cacaagagag tcagaacgaa      60

aatgtgcaac aagtgcggcg gctcctacca tggcaggtga ttcgaggact cagcacgagg     120

cgagagtagg gaccaggaag agccggaaaa cccgcctgtg attggccgtc cacgggtatc     180

ggtcgttgtg attgggtgag gcccagacag acagctgcgt tttgaaccgc gtagggttct     240

gggtagcaaa ggccttgcaa ggctcttaac cgaaaggggg aggggaaggt tcgccaacaa     300

acggctgagc tcacaatcct ggccggggcg tccccctccc ccatggagag agccagaccc     360

gagccgccgc ctcagccgcg cccgttgcgt cccgctccgc ccccgctgcc ggtcgagggc     420

acctcctttt gggcagcagc catggagccc cctccgtcgt ctcccacact gagcgcggca     480

gccagtgcga ccttggcctc gtcgtgcggg gaggcagtgg cgtccggctt acagcccgcg     540

gtgcggcggc tgctgcaggt gaagccagag caggtgttgc tgctaccaca gcctcaggcc     600

cagaacgagg aagccgctgc ctcgtccgcg caggcgcggc tgttgcagtt caggcccgac     660

ctgcggctcc tgcagccgcc gacagcgtca gacggcgcca cctccaggcc cgagttgcac     720

ccggtgcagc ccctggcgct gcatgtcaag gccaagaagc agaagctggg gcccagcctg     780

gatcagtcag tggggcctcg aggggccgtc gaaaccggtc ctagagcctc cagggtggtc     840

aagttggaag gccccgggcc ggccctcggc tacttccgag gggacgagaa gggcaagctg     900

gaggcggagg aggtcatgag agactcgatg caaggcgggg caggcaaaag cccggcagcc     960

atccgagaag gtgtgatcaa aacggaggaa cccgagagac tcctcgagga ctgcaggctc    1020

ggcgcggagc ccgcgtccaa tggcctggtt catggcagcg cggaggtcat cttggcccca    1080

acgtccggtg cctttgggcc gcaccagcaa gaccttagga tccctttgac gctccacacg    1140

gtcccccctg gggcccggat ccagtttcag ggagctccgc cttcagagct gataagattg    1200

accaaggtcc ccctgacacc agtgcctact aaaatgcagt ccctactgga gccttctgta    1260

aaaattgaaa ccaaagatgt cccgctcacc gtgttgccct cagatgcagg cataccagat    1320

actcccttca gtaaggacag aaatggtcat gtgaagcgac ccatgaacgc atttatggtt    1380

tgggcaagga tccaccgacc agcactagcc aaagctaacc cagcagccaa caatgcagaa    1440

atcagtgtcc agcttgggtt agagtggaac aaacttagtg aagaacaaaa gaaaccctat    1500

tacgatgaag cacaaaagat taaggaaaag cacagagagg aatttcctgg ttgggtttat    1560

cagcctcgtc cagggaagcg aaaacgattc cctctaagtg tttccaatgt attttctggt    1620

accacacaga atatcatctc tacaaatcct acaacagttt atccttaccg ctcacctacg 1680

tactctgtgg taattcccag cctacagaat cccatcactc atccagttgg tgaaacctca 1740

cctgctatcc agctgcccac acctgcagtc cagagcccaa gccctgtcac acttttccag 1800

cccagcgtct ccagtgctgc tcaggtggct gtccaggatc caagtctacc tgtctatcca 1860

gcactcccac cccaacgctt tactgggcct tcccaaacag acactcatca gctgcattct 1920

gaagccactc acactgtgaa gcaacccact cctgtctctc tagagagcgc caacaggatt 1980

tcaagtagtg caagtactgc ccatgccaga tttgcaactt cgaccatcca acctcctagg 2040

gagtattcca gcgtttcccc ttgtcccaga agtgctccaa tcccccaggc ttctcccatt 2100

ccacacccac atgtctacca gccccctccc cttggccatc cagccacact gttcgggaca 2160

ccaccaagat tctcttttca tcacccttac ttcctacccg gacctcacta cttcccatca 2220

agtacatgcc cttacagtcg gcctcccttt ggctatggaa attttccgag ttcaatgcca 2280

gaatgcctta gttattatga agacaggtac ccaaaacatg agggtatctt ttcaacttta 2340

aatagagact attcttttag agactactca agtgaatgca cacacagtga aaattctcgg 2400

agttgtgaga acatgaatgg aacttcttac tataacagtc atagccacag tggggaagaa 2460

aacttaaacc ctgtgcctca gctggacatt ggaaccttgg agaatgtctt cacagccccg 2520

acatcaactc cttctagcat ccagcaagtc aatgtcaccg acagtgatga ggaggaagaa 2580

gaaaaagtgc tcagggattt ataattttaa aacaaatatg cacagaaaat aaacatttct 2640

taaaatatat tctgggtcag ttggtatgag aaaaaaaaaa gcctagaatt ctttgttgaa 2700

agttttcagt cgtgatttga ggagttaaaa ccaaatgcaa tttatgtctt cataaaattt 2760

tgattagtga aactagagtc tggatgtttc attgtaggaa tatttaagtt attaagtagt 2820

ttaattttaa tggctgaaat ttgcatcaac atgtattatt attactttat cctggaacat 2880

gcaaaatact gaagcctcac agttgtatgt gaggggaaag gggaaataaa tctagcatag 2940

tgtgattttt attttatctc aggatacatt ttttaaatga ttttttgttt gctttttatg 3000

taatacttat ggatgttgtc aatttttgat gtaacatttt gaaagtattt tgacaactcc 3060

tagtgaactt ggacttggtt gctaaattta acttacacta ataaccaatt ataagttcca 3120

aatgtgtttt aatggcacct gggtgattct tcagctaaat ttagtcattt ctgtttctaa 3180

atatttttat cattttaaaa tatttttttt ccatttggca tacatcgttc tttgttgtaa 3240

ttaaataaac atagataaaa ttgttaaaaa aaaaaaaaaa 3280

<210> 10
<211> 2919
<212> DNA
<213> Homo Sapiens

<400> 10
   agttgagatt cagaccttga cagttgtaac cgatagactg ggctggggca acgctgatga 60
   aaggcaaatt cctgagagaa aaatcaccca gactttctag aagttgttat gagtggacct 120
   gactacctag agagagtaat acagaagctc cgtgccgatc acagctacct cattaccatg 180
   ttcctccaag atggccgccc ctgaggagaa tcatcaactg ggctttacgg aagagagaaa 240
   tccaaacgac catcagcaac aaatgaaaga ctgaagttga aaggaaatcc ataatttttg 300
   aatgctggcc gaggtctgtg acctctgact gaatggtttc caagctccga attcctaggt 360
   gatgttttca tttgttcaca cttgctgaag aagggaaagg ctctctgatt tacgtgtaag 420
   caaaacaatc taaacagtag gataaaatca tgttggaatg gagttctgcg tggatgctcc 480
   tttgaaccaa atcagttcca caactgaccc tgtcagggac atataaattt agcattttgg 540
   tgtggataca gaagacgaga ttaacacaga tcaagtaaca tggaaataaa aaaggagaca 600
   atgctggatg atacaggggc agccaacaag aggagaaaac ccaagcattg gcaaatgcct 660
   cattatccaa agaacactga ggtgtggagc aaaactgtgt tgaatgaact gtagatggaa 720
   attctctcct tcccaccgag gagccgctgc aggttgcaga ctcccagaat gccacactga 780
   gaagaggagg gctaaaattc tagagcagaa tccctggggc agcccctgct gaagaagggt 840
   gcatctgttg agaatctcat caccgccccc accccgccca gccttcaatt tagaggaatt 900
   tctagttcac gcagaatgaa gacaagatca cataagtgag gacgacgccc catttacaag 960
   tgtgatttgg aagactgaag tggaaacctt gggaaggggc ctcagagtat gtcagctggg 1020
   gtcagacggg aacaggagtc tggctcaaca cacatttatt gcactcctgt ttcatgccaa 1080
   gaaatgtacc aaaatctggg actgtgtaac acagcaaagg aaaaggctca tctccaggaa 1140
   gcccccttgt ctaggaagag aaattgaata tcagcataag gtggtgaaaa gctgtgaagg 1200
   agatgagtgc caggagtcca tggaggaagg attctagcct ggacaggtga ggaatggctt 1260
   tctggaagaa gcagatgccc cagctgaact ttaaaatgcc catgtgaaat gaactgtctg 1320
   tatcttcaat cagcaaatgg agtaggtggc cactgtttaa ttctggaatg gggaagtgta 1380
   aaggtgtggt gtgtgtgttt acccctactt agggattcaa aacagacagt aaagaacaaa 1440
   agtctagccc tttgagcatt ctccatggga catttgaggt ctagctggcc ccagagaaag 1500
   agagacagaa gcaaggtcat atccatttga gaaaatcaaa gtgtctgctc caattgagtc 1560
   tggtgtcctt attcctggaa tagccacccc tactgctctg gtgctgattt acctccggca 1620
   gttcagtgct tcctggaagg tgattgtggt gaatagtcat gagaatctgg gaactggctc 1680
   ctgctctgtc tatcttggag atgcatcttt tgtgagaagc cagggcaaga atagggaggc 1740
   ttaggcaatg ggaagtttct gagtagataa agcagatggc agagcaaagg gacagagcct 1800
   ggcgagaggc tactccctgt agcaacctgc ctaagagtga gaggaaagca aggcatggct 1860
   atgagcacgt gtagtctcag ctacttggga agctgaggct gggggatcac ttgagcccag 1920
   gagttcaaga ccagcctagg caacatagtg agaccatgtc tctttaaaaa aaaaaaaaaa 1980
   agaaaaaggg agagagagaa aaaaaggtga caattccccc aacacacata aagtacataa 2040
   gagttaacaa ggacacagtg gcagaaaata gtcagaactg agcaagcatc agtgaggaaa 2100
   tgttttcctg aggagacttg agaagtctca aaaatgaaaa gaaagagtat ttggtgtaga 2160
   aagaatggca tgagatgtga aagctcattc atccatttat tccaggtata tctttttctt 2220
   cagtactttg ggaggcaata ggtgtaatag caagaacatt caggttagtg ggttagtgtg 2280
   agtctgccat atgtatgagg gttgaataca cattaggtgt ggtgactgtc atatgggagg 2340
   cactgcaagg tgaaggagac atgtcctcac atttggtctt tgggaagcga agtaggcaga 2400
   aacccaagtg caaagtggcc atttgttttc ctaatgctag ataagtacct gatgtgttgg 2460
   aagggtggag tagaactaga aactcctgga agttctttta agaacgaatg caggacctca 2520
   tcagcccgag tctttaaata agtcacacat ttttggtcac cacattctgg aagggggaca 2580
   aaaagatctg tcacagacaa aatttggaga tatttaaggc tttgttgact ttggcaaatg 2640
   cttgattata gttctgtggt ctgtttgttt tctagagtct ccatgaattg caagggaaaa 2700
   caagtggtac actggtattt ctatttgata acctgttctc tttggaattc cggttagaaa 2760
   tcaaaggctt acttgagttt taatgctttc gggacttcaa atgcttttct atcttcctaa 2820
   taatatatta acattttctg aagacaacat atatgtggaa aacaacatta tttactcaaa 2880
   taattttttt tgaaaaatgg accaaaaaaa aaaaaaaaa 2919

<210> 11
<211> 1865
<212> DNA
<213> Homo Sapiens

<400> 11
ggtgcgcatg cgcccggaga gctgaagggc gcgcggtgcc gacagggagc ttccttggct 60

ggcatggcgg cgagcgcgct gcgaggcctg ccagtggctg gcggaggcga gagtagcgag 120

agcgaggatg atggctggga gattgggtat ctcgaccgga cgtctcagaa attgaaaagg 180

ctattaccca ttgaagaaaa gaaagaaaaa tttaagaaag caatgaccat cggagatgtt 240

tcattggtcc aggagctcct agattctggc attagtgtag attccaactt tcagtatgga 300

tggactcccc ttatgtatgc tgctagtgtt gccaatgcag agctggttcg ggtccttttg 360

gacagaggtg ctaatgcaag ctttgagaag gataagcaaa gtattttgat aactgcatgt 420

tctgctcatg gctcagagga acagatcttg aagtgtgtag aactactact ttcaagaaat 480

gctgatccaa atgttgcttg taggagactt atgaccccaa tcatgtatgc tgctcgagat 540

ggtcacaccc aggttgttgc tctccttgtt gctcatggag cagaagttaa tacccaggat 600

gagaatggtt acactgcttt aacgtgggca gcacgtcagg gtcataaaaa tatagttttg 660

aagttgcttg aacttggagc taataaaatg ctacaaacca aagatggaaa gatgccaagt 720

gagattgcaa aaagaaacaa acatcatgag atcttcaact tactttcttt tactttaaat 780

ccattggaag gaaaacttca acagctaact aaagaagaca ctatttgtaa aatattgaca 840

acagattctg atagagaaaa agatcacatt tttagttcat atacagcatt tggagatctg 900

gaagtatttt tacatggtct tggacttgaa catatgacag atttactaaa ggaaagggat 960

ataacgttaa gacatctttt gaccatgagg gaagatgaat ttacaaagaa tggaattacc 1020

agtaaagacc agcagaaaat tctggctgct cttaaagaac tacaggtaga agagatacaa 1080

tttggagagc tatctgaaga gacaaagttg gaaatcagtg gtgatgagtt cctcaacttt 1140

cttctcaaat taaataaaca gtgtggccat ttaataacag ctgtacagaa tgttattact 1200

gagttacctg taaattctca aaagataaca ctggaatggg cttctcccca gaattttact 1260

tcagtttgtg aagaattggt taataatgtt gaagatttga gtgaaaaggt ctgcaaacta 1320

aaagacctaa ttcaaaagtt gcaaaatgaa cgggaaaatg atccaactca tatacaatta 1380

agggaagaag tatctacatg gaatagtaga attttgaaga ggacagctat taccatatgc 1440

ggattcggtt ttcttctttt catttgcaag ctaactttcc agaggaaata atccaaatat 1500

ttgttttaag ttatatatac acatcttatt gaaccatcca aaaatcattg cttttaactg 1560

tttgcagcac aatataactt tacattcttt gccaatattc cataggaaaa attttgacaa 1620

tgtggagttt ttttttaaaa agtggtttgt tactttgact tataaataca atttgttatg 1680

tttatgttaa ttgtgattag gtagtaaagt ataagtaatt ttttggtttg atcgtatcta 1740

aatcacattc aaaattaaag ttatatctaa tatctttgac attttttaaa gtgtgttcat 1800

ttgtcagtga atcgtacagg atttgtaaag tgttcataaa ttgagctttc ttttgtttct 1860

ttttt 1865

<210> 12
<211> 2195
<212> DNA
<213> Homo Sapiens

<400> 12
ggctgcaggc ggggccggcg ggggcgcagc ggcccgggag gaagcagtac cttgaagaga 60

aattggagag ggagtcaatt cctaggatag cagagagatg gacaacagac agaatagatg 120

gagtttcaca atggtggcca tgtgtctgga attggtgggt tcttggtctc actgacttca 180

agaatgaagc cgcacaccct cgcagtcacc ccagctctga tctttgccat cacagttgct 240

acaatcggct ctttccagtt tggctacaac actggggtca tcaatgctcc tgagacgatc 300

ataaaggaat ttatcaataa aactttgacg gacaaggcaa atgcccctcc ctctgaggtg 360

ctgctcacga atctctggtc cttgtctgtg gccatatttt ccgtcggggg tatgatcggc 420

tccttttccg tcggactctt tgttaaccgc tttggcaggc gcaattcaat gctgattgtc 480

aacctgttgg ctgccactgg tggctgcctt atgggactgt gtaaaatagc tgagtcagtt 540

gaaatgctga tcctgggccg cttggttatt ggcctcttct gcggactctg cacaggtttt 600

gtgcccatgt acattggaga gatctcgcct actgccctga ggggtgcctt tggcactctc 660

aaccagctgg gcatagttat tggaattctg gtggcccaga tctttggtct ggaactcatc 720

cttgggtctg aagagctatg gccggtgcta ttaggcttta ccatccttcc agctatcctg 780

caaagtgcag cccttccatg ttgccctgaa agtcccagat ttttgctcat taacagaaaa 840

aaagaggaga atgctacgcg gatcctccag cggttgtggg gcacccagga tgtatcccaa 900

gacatccagg agatgaaaga tgagagtgca aggatgtcac aagaaaagca agtcaccgtg 960

ctggagctct ttagagtgtc cagctaccga cagcccatca tcatttccat tgtgctccag 1020

ctctctcagc agctctctgg gatcaatgct gtgttctatt actcaacagg aatcttcaag 1080

gatgcaggtg ttcaacagcc catctatgcc accatcagcg cgggtgtggt taatactatc 1140

ttcactttac tttctctatt tctggtggaa agggcaggaa gaaggactct gcatatgata 1200

ggccttggag ggatggcttt ttgttccacg ctcatgactg tttctttgtt attaaagaat 1260

cactataatg ggatgagctt tgtctgtatt ggggctatct tggtctttgt ggcctgtttt 1320

gaaattggac caggccccat tccctggttt attgtggccg aactcttcag ccagggcccc 1380

cgcccagctg cgatggcagt ggccggctgc tccaactgga cctccaactt cctagtcgga 1440

ttgctcttcc cctctgctgc ttactattta ggagcctacg tttttattat cttcaccggc 1500

ttcctcatta ccttcttggc ctttaccttc ttcaaagtcc ctgagacccg tggcaggact 1560

tttgaggata tcacacgggc ctttgaaggg caggcacacg gtgcagatag atctgggaag 1620

gacggcgtca tggggatgaa cagcatcgag cctgctaagg agaccaccac caatgtctaa 1680

gtcatgcctc cttccacctc cctcccggca tgggaaagcc acctctccct caacaaggga 1740

gagactttat caggatgaac ccaggactgc ttctgaatgc tgctacttga tttctttctc 1800

atcccacgca ctccatgagc accccaaggc tgcagtttgt tggatcttca atggcttttt 1860

aaattttatt tcctggacat cctcttctgc ttaggagaga ccgagtgaac ctaccttcat 1920

ttcaggaggg attggccgct tggcacatga caactttgcc agcttttcct cccttgggtt 1980

ctgatattgc cgcactagag gatataggag aggaaaagta aggtgcagtt gccccaacct 2040

cagacttacc aggaagcaga tacatatgag tgtggaagcc ggagggtgtt tatgtaagag 2100

caccttcctc acttccatac agctctacgc ggcaaattaa cttgagtttt atttatctta 2160

tcctctggtt taattacata aatatttatt tttta 2195

<210> 13
<211> 531
<212> DNA
<213> Homo Sapiens

<400> 13
acgagcactg gagcttgcgt tacttggcct cacctcacct gtgctgtcca cgcctggctt 60

tgtctcacct gacgcgatat gcctctcctg cgtgggcgct gtcctgcccg ccgccactac 120

cgccgcttgg ccctgctcgg cctgcagccc gctccccgct tcgcccactc ggggcccccg 180

cgccagcggc ccctgtctgc cgcggaaatg gctgttggac ttgtggtgtt ttttacgacc 240

ttcttaacac cagctgcata tgtgctaggc aacctgaagc agttcagaag gaattagatg 300

gaagatgatg ttgaacagct gttaacgtcc aaaaaacttt cagaaaaagc tgtgtttttg 360

ttaacgagca aaattgccta gttgagttga tgcaaccatt gtggtattca ctttcctcat 420

gtttatgatg aatattttgc acttttttag tactgtgcat tatatagatg tatagtcaaa 480

aatgttctgc ttaagtgtta aataaaacgg aaacacttat tcgtgcttgg t 531

<210> 14
<211> 2837
<212> DNA
<213> Homo Sapiens

<400> 14
agtctcgcgg gaagctccgt tgtgggcgcc ccggctggtg gctgagctca ggccttcagg 60

cagaggggag gcgagggcgg ggcggtcacg tgagagcact gccgcggtgg gttgtggggg 120

tgctgcggcg ccgtttgctt tgccaaaccg acaaaagaga gatgatggcc aacgacgcca 180

agcccgacgt gaagaccgtg caggtgctgc gggacacagc caaccgcctg cggatccatt 240

ccatcagggc cacgtgtgcc tctggttctg gccagctcac gtcgtgctgc agtgcagcgg 300

aggtcgtgtc tgtcctcttc ttccacacga tgaagtataa acagacagac ccagaacacc 360

cggacaacga ccggttcatc ctctccaggg gacatgctgc tcctatcctc tatgctgctt 420

gggtggaggt gggtgacatc agtgaatctg acttgctgaa cctgaggaaa cttcacagcg 480

acttggagag acaccctacc ccccgattgc cgtttgttga cgtggcaaca gggtccctag 540

gtcagggatt aggtactgca tgtggaatgg cttatactgg caagtacctt gacaaggcca 600

gctaccgggt gttctgcctt atgggagatg gcgaatcctc agaaggctct gtgtgggagg 660

cttttgcttt tgcctcccac tacaacttgg acaatctcgt ggcggtcttc gacgtgaacc 720

gcttgggaca aagtggccct gcaccccttg agcatggcgc agacatctac cagaattgct 780

gtgaagcctt tggatggaat acttacttag tggatggcca tgatgtggag gccttgtgcc 840

aagcattttg gcaagcaagt caagtgaaga acaagcctac tgctatagtt gccaagacct 900

tcaaaggtcg gggtattcca aatattgagg atgcagaaaa ttggcatgga aagccagtgc 960

caaaagaaag agcagatgca attgtcaaat taattgagag tcagatacag accaatgaga 1020

atctcatacc aaaatcgcct gtggaagact cacctcaaat aagcatcaca gatataaaaa 1080

tgacctcccc acctgcttac aaagttggtg acaagatagc tactcagaaa acatatggtt 1140

tggctctggc taaactgggc cgtgcaaatg aaagagttat tgttctgagt ggtgacacga 1200

tgaactccac cttttctgag atattcagga aagaacaccc tgagcgtttc atagagtgta 1260

ttattgctga acaaaacatg gtaagtgtgg cactaggctg tgctacacgt ggtcgaacca 1320

ttgcttttgc tggtgctttt gctgcctttt ttactagagc attcgatcag ctccgaatgg 1380

gagccatttc tcaagccaat atcaacctta ttggttccca ctgtggggta tccactggag 1440

aagatggagt ctcccagatg gccctggagg atctagccat gttccgaagc attcccaatt 1500

gtactgtttt ctatccaagt gatgccatct cgacagagca tgctatttat ctagccgcca 1560

ataccaaggg aatgtgcttc attcgaacca gccaaccaga aactgcagtt atttataccc 1620

cacaagaaaa ttttgagatt ggccaggcca aggtggtccg ccacggtgtc aatgataaag 1680

tcacagtaat tggagctgga gttactctcc atgaagcctt agaagctgct gaccatcttt 1740

ctcaacaagg tatttctgtc cgtgtcatcg acccatttac cattaaaccc ctggatgccg 1800

ccaccatcat ctccagtgca aaagccacag gcggccgagt tatcacagtg gaggatcact 1860

acagggaagg tggcattgga gaagctgttt gtgcagctgt ctccagggag cctgatatcc 1920

ttgttcatca actggcagtg tcaggagtgc ctcaacgtgg gaaaactagt gaattgctgg 1980

atatgtttgg aatcagtacc agacacatta tagcagccgt aacacttact ttaatgaagt 2040

aaactaggct tatttctaaa aagtcaagtc tattggcttt ggcccaaaag cactggtatc 2100

tttgtattaa attcatgttt attgtcacaa aaccattatt tatacctata cagttgtact 2160

gtttctttta aagcaaagcc atttaacatc tttcttcatt cctaatttgg aaattaaagt 2220

ttacctttct gttaatctat gtataaatgt tactctgagt tattaatgtg gattttaaaa 2280

ttgtaagcaa tagaatagga aataaaacaa ctacctaata caaatatttc tgataagact 2340

acaaatatct gactgagctg gggattaaag tagaggtaac tgtatcttaa atgagtatga 2400

tttccttgta agttaaaaaa attgaaattt aattgtagac ttcaatagtc caagttttga 2460

aggatgtttg agcttttgta taatgccatt tatacctgca gttttacaga taatgtttga 2520

ctgcagttgc cttggaaatt cctccaaagt ttgccttcat ctctcctcta cagtttggag 2580

gtgatggtgc agcagtggaa catctcttga tgcaccacac tacttgtgtt ctgtgaagtg 2640

atgaaagtat aactggttct agtttgcaca ctacacacat agttttgtga agcttcagaa 2700

atgttttttc ttttccttgt ggccaaacca gtttgttaat ctgattatat tcatctgcta 2760

atgatactaa agttaatgta ataaagcatt taaaaatcag aaaaaaaaaa aaaaaaaaaa 2820

aaaaaaaaaa aaaaaaa 2837

<210> 15
<211> 3848
<212> DNA
<213> Homo Sapiens

<400> 15
agcgttgctg ctgccttgca gtttgatctc agactgctgt gctagcaatc agcgagactc 60

cgtgggcgta ggaccctcca agccaggtga aagagcttat gatcctaagc acttccataa 120

tagggtcagt agaatcatga tcgatgatca taatgtcccc actctacggg agatggtagc 180

attctccaag gaagtgttgg agtggatggc tcaagattct gaaaacatcg tagtgattca 240

ctgtaaagga ggcaaagaat agatatgttg gatattttgc acaagtgaaa catagctaca 300

actggaatct ccctccaaga aaaacactgt ttataaaaag attcgttatt tattcgattc 360

atggtgttgg aacaggcgat ggatatgatc taaaagtcca aatagtaatg aagaaaaaga 420

ttgtcttttc ctgtacttcc ttaaagaatt gtcgggtatt tcatgacact gaaacagaca 480

gggtaataac tgatgtgttc aactgtccac ctctgtatga tgatgtgaaa gtgcaagctt 540

cctcttcaag agaagagggc agcacacctc gcagggctaa ctggaagggg gagccatcca 600

ggagacctgt gctcaactga tgggtgggga gcaatagcga gaacgaggga gggacctgag 660

agtggaagcc tttattggga tgtaaggtgt tacctgagca ggtttcctac ggggaggtct 720

aactggtgga tttaatgcaa gcagtcatga gttccatgga gtcatgctgt gactgagagg 780

tggtcattga tatatccaca tggtccatgc agagtacggg ggtctgtagg gaggttatat 840

ctagctgtcc cataatgaag tagtcaccaa cagaaggttg tataaggcag atactgggat 900

cagtcacatt gagaaacctg gaggaggtga actggaaact gtcaagggtg actgaaccct 960

gcttctgata tcagaaagtc caatttatat ttgaaaggga tgctgaggca caaaaaaatt 1020

gtaagaattc actacaaaaa tacttggcta tatataagca taggtcctta gtagattctg 1080

tttagcacta tctaaaccag attcaaattt cagcatttaa attaaatatc tatcatggaa 1140

aataaactat tccttgaaaa ttttggtaga aacagcaaga gaaagcaata gcattttctt 1200

aagcctcctc ctctgtgtct tgagtgtgtt attatagaat gcagagtgct acctattgaa 1260

tggttataat tatttgataa atatataaag gaataaagga aggaactttg atttctttgg 1320

aatgatagtt cttggcatca attttacttt taaaatattt tttttcttt ttaggatttt 1380

cctaaatact atcacaacta cccttttttc ttctggttta acacatcttt aatacaaaat 1440

aacgggtatg gatataatat caacccatag aaacaaccta atcttcaatg tctatgtata 1500

agatgtaatg gcaagtcttt tgctggttgt cataagctta atttatagaa aacaaaaaat 1560

ccttgagcca ccattgttca ttgccttact ccttttacgt tggctatttt aaaaatacag 1620

ttgttcttga gacccccagt tgcagtatcc tcaaggtcca tgccatagga ctgtgttatg 1680

agctcaaaag tattataatc agatcttaag tgtggaagta aattcctccc agagaagttc 1740

aatatgaatc tgctcagtac cttcaacatg tcaggtcctc agtaggtgct gatttaccaa 1800

tgacgaacca ccaccaaatt ttgtgctaaa gtaagggagg acctagggaa gcttcagcta 1860

gctgaaaagc tgactgacac acttatatct aggagaagtt acaagacaca gtaagtatta 1920

agaaatacag ctaaaaaatc attaaaattg gtagtctccc atttaaacat gggtttctaa 1980

taactgaatt gggaaaactt tcttaaaaac tattaattgg aggctgggtg tggtggctca 2040

tgcctgtaat cctagcactt tgggaggctg aggcgggcgg atcacctaag gttggaagtt 2100

cgagactagc ctggccaaca tggtaaaact ccgtctctac taaaaataca aaaatcagcc 2160

aggcgtggtg gcacatgcct gtaatcccag ctactcagga ggctgagcca gtagaatcgc 2220

ttgaacccag gaggcagatt gcagtgagcc gagatcgcac cactacactc cagcctgggc 2280

gacagagtga gactctgtct aaagaaaaaa gcaaaaaaac agaacaacta ttacttggat 2340

ttggagatta ttgttcccag aaaaccttct gccatatttg gaaacttatt tctcagtcta 2400

gaagttctcc actttaagta gcatttgttc tgtgctggtg aaaaactgag atttttttgt 2460

attaaccata ctcttcaata caaaaggaga aaatattttt aaaatgcttc aggtcacagt 2520

tgaggcagtt gctatgattg catgtggcat gaattggtag ttattgttac aaccagttct 2580

agtcttttct tcaaatctga gctggatcta ataactcctt aagtccagca aggcaacagt 2640

aaattaaacc tctggtctac acacttgcaa tacatacaca tttaatagat tttgatagag 2700

tgaactttgg attggatgga aattttttaa aaatttgttt cttggatgca tacaaacaat 2760

aagctttgac tcctaacatg agcaaagtcc ctcaattgtg agagctgggt ggagcttcat 2820

ttgttgctgc tcctcaaatt gattcttggt aaaggataca gatttttcct ttgaaacacc 2880

atgttcattt tggggaagca ataagttaga tcacctttat tttcactttt atataaattt 2940

ctaaagattt ctgtaatatt taaatttata tactattggt aaagctgttt ttcttagttg 3000

tgaaattgtt gtttagccaa aaatgccaac ttctgtcttt tagaacacta ggcataaatg 3060

ggttaaccaa tttatgccta gtgttccatt attggaatgc taagcatgtg ggatttattt 3120

atatcctact gctcaaggtc atcgccaagg gctgtttgca aaaattcaaa aaattgcaac 3180

ctcaggcata aattaaaaga gatatagtat tttattattg ggttttgata catgtctaat 3240

cagactgatt tctgtcacat atagaaattt agatactgta ttaaacctgg atgtcattaa 3300

ttccataaaa agcaacgtta aaagaatcag tagcatgtgt tactgatgtg ttgctgaaga 3360

ttaagatatt tttaagtctc accgaaaagg tagaaggagc caactgagac acaaaaaggg 3420

gctgaggttc tattcatggt gagcaagtct ttttttttgt ttgtttcttc aagctctaac 3480

aagggtgcct actacatggc ttttcagtta gccccaaaat aagatgtaac aatttttttt 3540

tctattctta ggctttatct acaaagaaat gaattggata atcttcataa acaaaaaaca 3600

tggaaaattt atcaaccaga atatgcagta gagatatatt ttaatgagaa atgacttaag 3660

ttatgttgta actggtagct gattaagtat agttccctgc accccttctg ggaaagaatt 3720

atgttctttc taaccctgcc acatagttat atgttctaaa tcttccttgc tggtacatct 3780

atattgatat atgtatacac atgttcttta taaatctatt aaatatatac agaaaaaaaa 3840

aaaaaaaa 3848

<210> 16
<211> 1049
<212> DNA
<213> Homo Sapiens

<400> 16
gttagacaca ggacctgctg ggccacagaa aggaggctct gggtagacgc actagattac 60

tggataaatc acttcaattt cccaatgaat tttatattgt ttatttttat acctggagtt 120

ttttccttaa aaagtagcac tttgaagcct actattgaag cattgcctaa tgtgctacct 180

ttaaatgaag atgttaataa gcaggaagaa aagaatgaag atcatactcc caattatgct 240

cctgctaatg agaaaaatgg caattattat aaagatataa aacaatatgt gttcacaaca 300

caaaatccaa atggcactga gtctgaaata tctgtgagag ccacaactga cctgaatttt 360

gctctaaaaa acgataaaac tgtcaatgca actacatatg aaaaatccac cattgaagaa 420

gaaacaacta ctagcgaacc ctctcataaa aatattcaaa gatcaacccc aaacgtgcct 480

gcattttgga caatgttagc taaagctata aatggaacag cagtggtcat ggatgataaa 540

gatcaattat ttcacccaat tccagagtct gatgtgaatg ctacacaggg agaaaatcag 600

ccagatctag aggatctgaa gatcaaaata atgctgggaa tctcgttgat gaccctcctc 660

ctctttgtgg tcctcttggc attctgtagt gctacactgt acaaactgag gcatctgagt 720

tataaaagtt gtgagagtca gtactctgtc aacccagagc tggccacgat gtcttacttt 780

catccatcag aaggtgtttc agatacatcc ttttccaaga gtgcagagag cagcacattt 840

ttgggtacca cttcttcaga tatgagaaga tcaggcacaa gaacatcaga atctaagata 900

atgacggata tcatttccat aggctcagat aatgagatgc atgaaaacga tgagtcggtt 960

acccggtgaa gaaatcaagg aacccggtga agaaatctta ttgatgaata aataacttta 1020

attattttgt catcaaaaaa aaaaaaaaa 1049

<210> 17
<211> 3591
<212> DNA
<213> Homo Sapiens

<400> 17
ttactgggcg tatggcgtac agacacgagg ccggcgcccg ggaggcggtg ttcatccgcc 60

cgggaaaaga gcgcctgttg ctcgctgccc gcgtgtccct ggctctctcg ggaacccagc 120

gccgaaggcg aggtgggcgc gggccgaagg aggtcctggg aggtcggcgg cgcggaggga 180

tctccgcggg agccgttggg gctgttggcc tcgggctgag gtgcaaggac caggactagg 240

gcgagggcag cggtccaaga aatagaaaac aatgactggg agagcccgag ccagagccag 300

aggaagggcc cgcggtcagg agacagcgca gctggtgggc tccactgcca gtcagcaacc 360

tggttatatt cagcctaggc ctcagccgcc accagcagag ggggaattat ttggccgtgg 420

acggcagaga ggaacagcag gaggaacagc caagtcacaa ggactccaga tatctgctgg 480

atttcaggag ttatcgttag cagagagagg aggtcgtcgt agagattttc atgatcttgg 540

tgtgaataca aggcagaacc tagaccatgt taaagaatca aaaacaggtt cttcaggcat 600

tatagtaagg ttaagcacta accatttccg gctgacatcc cgtccccagt gggccttata 660

tcagtatcac attgactata acccactgat ggaagccaga agactccgtt cagctcttct 720

ttttcaacac gaagatctaa ttggaaagtg tcatgctttt gatggaacga tattattttt 780

acctaaaaga ctacagcaaa aggttactga agtttttagt aagacccgga atggagagga 840

tgtgaggata acgatcactt taacaaatga acttccacct acatcaccaa cttgtttgca 900

gttctataat attattttca ggaggctttt gaaaatcatg aatttgcaac aaattggacg 960

aaattattat aacccaaatg acccaattga tattccaagt cacaggttgg tgatttggcc 1020

tggcttcact acttccatcc ttcagtatga aaacagcatc atgctctgca ctgacgttag 1080

ccataaagtc cttcgaagtg agactgtttt ggatttcatg ttcaactttt atcatcagac 1140

agaagaacat aaatttcaag aacaagtttc caaagaacta ataggtttag ttgttcttac 1200

caagtataac aataagacat acagagtgga tgatattgac tgggaccaga atcccaagag 1260

cacctttaag aaagccgacg gctctgaagt cagcttctta gaatactaca ggaagcaata 1320

caaccaagag atcaccgact tgaagcagcc tgtcttggtc agccagccca agagaaggcg 1380

gggccctggg gggacactgc cagggcctgc catgctcatt cctgagctct gctatcttac 1440

aggtctaact gataaaatgc gtaatgattt taacgtgatg aaagacttag ccgttcatac 1500

aagactaact ccagagcaaa ggcagcgtga agtgggacga ctcattgatt acattcataa 1560

aaacgataat gttcaaaggg agcttcgaga ctggggtttg agctttgatt ccaacttact 1620

gtccttctca ggaagaattt tgcaaacaga aaagattcac caaggtggaa aaacatttga 1680

ttacaatcca caatttgcag attggtccaa agaaacaaga ggtgcaccat taattagtgt 1740

taagccacta gataactggc tgttgatcta tacgcgaaga aattatgaag cagccaattc 1800

attgatacaa aatctattta aagttacacc agccatgggc atgcaaatga gaaaagcaat 1860

aatgattgaa gtggatgaca gaactgaagc ctacttaaga gtcttacagc aaaaggtcac 1920

agcagacacc cagatagttg tctgtctgtt gtcaagtaat cggaaggaca aatacgatgc 1980

tattaaaaaa tacctgtgta cagattgccc taccccaagt cagtgtgtgg tggcccgaac 2040

cttaggcaaa cagcaaactg tcatggccat tgctacaaag attgccctac agatgaactg 2100

caagatggga ggagagctct ggagggtgga catccccctg aagctcgtga tgatcgttgg 2160

catcgattgt taccatgaca tgacagctgg gcggaggtca atcgcaggat ttgttgccag 2220

catcaatgaa gggatgaccc gctggttctc acgctgcata tttcaggata gaggacagga 2280

gctggtagat gggctcaaag tctgcctgca agcggctctg agggcttgga atagctgcaa 2340

tgagtacatg cccagccgga tcatcgtgta ccgcgatggc gtaggagacg gccagctgaa 2400

aacactggtg aactacgaag tgccacagtt tttggattgt ctaaaatcca ttggtagagg 2460

ttacaaccct agactaacgg taattgtggt gaagaaaaga gtgaacacca gattttttgc 2520

tcagtctgga ggaagacttc agaatccact tcctggaaca gttattgatg tagaggttac 2580

cagaccagaa tggtatgact tttttatcgt gagccaggct gtgagaagtg gtagtgtttc 2640

tcccacacat tacaatgtca tctatgacaa cagcggcctg aagccagacc acatacagcg 2700

cttgacctac aagctgtgcc acatctatta caactggcca ggtgtcattc gtgttcctgc 2760

tccttgccag tacgcccaca agctggcttt tcttgttggc cagagtattc acagagagcc 2820

aaatctgtca ctgtcaaacc gcctttacta cctctaacct gcagaagacg atgcagccgc 2880

ttttcttttt gaaatgactt tgggattttt ttaagctttt atttactttt tttttaactg 2940

ttatctttct ggatgaaact tgggaagggg attaggagat ctagcatttt atttctagca 3000

ttgctattca ccggcttcct tattttatac gtaaaaatta agattttata ttttatcttc 3060

ttgtttctca tagatatttt gtgagcattt ttttgtttat tttgaagaaa tgtggataag 3120

atacttggta gtataaaaca gactctctga gagtatttga aatgtgtttg gagatttact 3180

taaacgtact ttcaggagtg agcaagtcct acttataaac ctatattaac tttatttttg 3240

agatacctgt tttgaattta aaggagataa gaggcgtaaa gtaggatgct cactacaacc 3300

ataggtgggg tttcagctca tatcttaaag ataaaaggta ctattatata acctatacac 3360

aagatacagg agaaaatatg cttgattttt atttggcagg ggggctaggt tgtatgggag 3420

taaaaaaaac attgaaaatt tttaaattgt ccaaagaaac attttaagac tctttaacaa 3480

aaaaggccat gagtaaatct ctatattaac attactattt attttgtttt ggaactggga 3540

catgattcta tttgttataa aataaaattg atgtgattgt caccttattt g 3591

<210> 18
<211> 819
<212> DNA
<213> Homo Sapiens

<400> 18
gtgatgctcc ctgggcctcc tgaccgcgcc ctcgcctggg aggcggggcg ggccgggttc 60

tctctgtgac gtcacaaagg ccccgccatg cctctggctt tgacccttct gctgctctcg 120

ggcttgggcg cccccggagg ctggggctgc ctgcagtgcg accccttggt gctggaggcc 180

ctgggtcacc tgcgctccgc cctcatcccc agtcgcttcc agttggagca gctgcaggcg 240

cgcgccgggg ccgtgctgat gggcatggag gggcctttct tccgggacta cgcgctgaac 300

gtgtttgtgg ggaaagtgga gacaaatcaa ctggaccttg tggcgtcctt tgtcaagaac 360

caaacgcagc acttaatggg taactctctg aaagatgagc ctctgctgga agagctggtg 420

accctcaggg cgaatgtgat caaggaattc aagaaagttt taatttcata tgaattaaaa 480

gcctgcaacc ccaaactttg ccgcttgcta aaagaagagg tgttggactg tttacattgc 540

cagaggatca ctcccaagtg tatccacaaa aagtactgct ttgtcgaccg gcaaccccgc 600

gtggccctgc agtaccagat ggacagcaaa tacccgagga accaggcgct gttgggcatc 660

ctcatttctg tgtctctggc tgtctttgtc ttcgtggtca tcgtggtctc ggcttgtaca 720

tacagacaaa accgaaaact cctgctgcag taggacggtg gtttgggggt aaggagaaag 780

gaaaataaat ttaataaaat tggtgacaaa tccaaaaaa 819

<210> 19
<211> 5120
<212> DNA
<213> Homo Sapiens

<400> 19
ggactcgcac tcggcggttg ttccagaaga aagagacagc gatggcggca gaggcttcga 60

agactgggcc ttctaggtct tcctaccagc gaatggggag gaagagtcag ccctggggtg 120

ccgctgaaat ccagtgcacc aggtgtggaa ggagggtatc cagatcatcc ggtcaccatt 180

gtgaacttca atgtggacat gctttttgtg aactatgctt gttaatgact gaagaatgca 240

ccacaattat atgccctgat tgtgaggttg ctacagctgt aaatactaga caacgctact 300

acccaatggc tggatatatt aaggaagact ccataatgga aaaactgcag cctaagacga 360

taaagaattg ttctcaggac tttaagaaga ctgctgatca gctaactact ggtttagaac 420

gttcagcctc cacagacaag actcttttga actcatcagc tgtaatgttg gacactaata 480

ctgcagaaga aattgatgaa gcattgaata cagcacacca tagtttcgaa cagttaagca 540

ttgctggaaa agcacttgaa cacatgcaga agcaaacgat agaggaaaga gaaagagtta 600

tagaagttgt ggagaaacag tttgaccaac ttttggcttt ttttgattcc aggaaaaaga 660

acctgtgtga agaatttgca agaactactg atgattatct atcaaattta ataaaggcta 720

aaagctacat tgaagagaaa aaaaataatt tgaatgcagc tatgaacata gcaagagcat 780

tacaattatc gccttctcta agaacatact gtgacctgaa tcagattatc cggactttgc 840

agttaacttc agatagtgaa ttagcacaag ttagttctcc acaactaagg aaccctccca 900

ggttgagtgt gaattgcagt gagatcatct gtatgttcaa caatatggga aagattgaat 960

ttagggactc aacaaaatgt tatccccaag aaaatgaaat tagacagaat gttcaaaaga 1020

aatataataa caaaaaggaa ctttcttgtt acgatacata cccaccgcta gaaaagaaaa 1080

aggttgacat gtctgtccta accagtgaag caccaccacc tcctttgcaa cctgagacaa 1140

atgatgtaca tttagaagca aaaaacttcc agccacagaa agacgttgca acagcatccc 1200

ctaaaaccat tgctgtgtta cctcagatgg gatctagccc tgatgtgata attgaagaaa 1260

ttattgaaga caacgtggaa agttctgcag agctagtttt tgtaagccat gtaatagatc 1320

cttgccattt ctacattcgg aagtattcac aaataaaaga cgccaaagta ctggagaaga 1380

aggtgaatga attttgcaat aggagttcac accttgatcc ttcagacatt ttggaactag 1440

gtgcaagaat atttgtcagc agtattaaaa atggaatgtg gtgtcgagga actatcacag 1500

aattaattcc aatagagggt agaaatacca gaaaaccttg tagtccaacc agattatttg 1560

tccatgaagt tgcactaata caaatattca tggtagattt tggaaattct gaagtcctga 1620

ttgtcactgg agttgttgat acccatgtga gaccagaaca ctctgctaag caacatattg 1680

cactaaatga tttatgtctg gttctaagga aatctgaacc atatactgaa gggctgctaa 1740

aagacatcca gccattagca caaccatgct cattgaaaga cattgttcca cagaattcaa 1800

atgaaggctg ggaagaggaa gctaaagtgg aatttttgaa aatggtaaat aacaaggctg 1860

tttcaatgaa agtttttaga gaagaagatg gtgtgcttat tgtagatctg caaaaaccac 1920

caccgaataa aataagcagt gatatgcctg tgtctcttag agatgcgcta gtttttatgg 1980

aactagcaaa gtttaagtca caatcactaa gaagtcactt tgaaaaaaat actactttac 2040

actatcatcc acctattttg cctaaagaaa tgacagatgt ttcagtaacg gtttgtcata 2100

taaatagtcc tggagatttc tatcttcagt tgatagaggg cctggatatt ttatttctat 2160

taaagacaat cgaggaattc tataaaagtg aagatggaga aaatctggaa atcctctgtc 2220

cagttcaaga tcaagcctgt gtagctaaat ttgaagatgg aatttggtac cgagcaaaag 2280

ttatcggatt gcctggacat caggaagttg aagttaaata tgtggacttt ggtaatactg 2340

caaaaataac aatcaaagac gtgcgtaaaa taaaggatga gtttctgaat gccccagaga 2400

aggcaattaa atgtaagttg gcctatattg aaccatataa aaggacaatg cagtggtcca 2460

aagaagctaa agaaaaattt gaagaaaagg ctcaagataa atttatgaca tgttcagtta 2520

tcaaaattct ggaagataat gtgctcttag ttgagctttt cgattctctt ggtgctcctg 2580

aaatgactac tactagtatt aatgaccagc tagttaaaga gggcctagca tcttatgaaa 2640

taggatacat cctcaaagat aattctcaaa agcatattga agtttgggat ccttctccag 2700

aagaaattat ttcaaatgaa gtacacaact taaatcctgt gtctgcaaaa tctctaccta 2760

atgagaattt tcagtcactt tataataagg aattgcctgt gcatatctgt aatgtaatat 2820

ctcctgagaa gatttatgtt cagtggttgt taactgaaaa cttacttaat agtttagaag 2880

aaaagatgat agctgcttat gaaaactcaa aatgggaacc tgttaaatgg gaaaatgata 2940

tgcactgtgc tgttaagatc caagataaaa atcagtggcg aagaggccag atcatcagaa 3000

tggttacaga cacattggta gaggtcttgc tgtatgatgt gggtgttgaa ctagtagtga 3060

atgttgactg tttaagaaaa cttgaagaaa atctaaagac aatgggaaga ctctctttgg 3120

aatgttctct ggttgacata agaccagctg gtgggagtga caagtggaca gcaacagctt 3180

gtgactgtct ttcattgtac ctgactggag ctgtagcaac tataatctta caggtggata 3240

gtgaggaaaa caacacaaca tggccattac ctgtgaaaat tttctgcaga gatgaaaaag 3300

gagagcgtgt tgatgtttct aaatatttga ttaaaaaggg tttggctttg agagaaagga 3360

gaattaataa cttagataac agccattcat tatctgagaa gtctctggaa gtccccctgg 3420

aacaggaaga ttcagtagtt actaactgta ttaaaactaa ctttgaccct gacaagaaaa 3480

ctgctgacat aatcagtgaa cagaaagtgt ctgaatttca ggagaaaatt ctagaaccaa 3540

gaaccactag agggtataag ccaccagcta ttcctaacat gaacgtattt gaggcaacag 3600

tcagctgtgt tggtgatgat ggaactatat ttgtagtacc taaactatca gaatttgagc 3660

taataaaaat gacaaatgaa attcaaagta atttaaaatg ccttggtctt ttggagcctt 3720

atttctggaa aaaaggagaa gcatgtgcag taagaggatc cgatactctg tggtatcgtg 3780

gcaaggtgat ggaggttgta ggtggcgctg tcagagtaca atatttagat catggattca 3840

ctgaaaagat tccgcagtgc catctttacc ctattttgct gtatcctgat ataccccagt 3900

tttgtattcc ttgtcagctc cataatacca cacctgttgg gaatgtctgg caaccagatg 3960

caatagaagt tcttcaacaa ctgctttcaa agagacaggt ggacattcac attatggagt 4020

tacctaaaaa tccatgggag aaattgtcta ttcacctcta ttttgatgga atgtcacttt 4080

cttattttat ggcatactat aaatactgta cttctgaaca tactgaggag atgttgaaag 4140

aaaaaccaag atcagatcat gataaaaagt atgaagagga acaatgggaa ataaggtttg 4200

aggaattgct ttcggctgaa acagacactc ctcttttacc accatatttg tcttcatctc 4260

tgccttcccc aggagaactc tatgctgttc aagttaagca cgttgtctca cctaatgaag 4320

tgtatatttg ccttgattct atagaaactt ctaaccagtc taaccagcat agtgacacag 4380

atgatagtgg agtcagcggg gaatcagaat ccgagagcct tgatgaagca ctgcagaggg 4440

ttaataagaa ggtagaggcg cttcctcctc tgacggattt tagaacagaa atgccttgcc 4500

ttgcagaata tgatgatggc ttatggtata gagcgaagat tgttgccatt aaagaattta 4560

atcctttatc tatcttagta caatttgttg attatggatc aactgcaaag ctgacattaa 4620

acagactgtg ccaaattcct tctcatctta tgcggtatcc agctcgagcc ataaaggttc 4680

tcttggcagg gtttaaacct cccttaaggg atctagggga gacaagaata ccatattgtc 4740

ccaaatggag catggaggca ctgtgggcta tgatagactg tcttcaagga aaacaactct 4800

atgctgtgtc catggctcca gcaccagaac agatagtgac attatatgac gatgaacagc 4860

atccagttca tatgccgttg gtagaaatgg ggcttgcaga taaagatgaa taagtgccta 4920

agtgtataca gtgagagcat ctatagaagc ctagaagaat tctgttatgt ttagactatg 4980

tcttatcttt agactatttc aggcttaatt ttcctaactt gttcagcact agtgctttac 5040

ctctcatttt taattgaact gttaggaatt gtgtggggaa aaaaagtaaa taaatgttcg 5100

cttccaaaaa aaaaaaaaaa 5120

<210> 20
<211> 1738
<212> DNA
<213> Homo Sapiens

<400> 20
gatgtcatca ggctttgtga gggggactgt actgcccttt gagtactact gtgtggcccc 60

gcaacccagc acaaccaggt atctgcttgg aacccagcca ccataaagcc tgctagctaa 120

aaaaaatttt acatctctca gttcattcgg cacagacccc tgcctcattc agctgtgact 180

ctgcttggaa aattcatcag ttacaaagca gccaatgcaa ttatctcaag ggaaattgaa 240

aaatggacct ttgaaaatgc tagatttaca atgagaaatg ccataattca aggtttattc 300

tatgggtcct tgacatttgg gatctggaca gctctgttat tcatatattt gcaccataat 360

catgtgagca gctggcagaa gaaaagccag gagcctctgt cagcttggtc ccctggaaaa 420

aaagtgcatc agcaaattat ctatggctca gagcaaatac caaaacctca tgtaatagtc 480

aaaaggactg atgaagataa agcaaagtct atgttaggta cagattttaa ccatacaaac 540

ccagaacttc ataaagaact tttaaaatat ggatttaatg tgattatcag tagaagcttg 600

ggcatcgaaa gagaagtgcc agataccagg agtaaaatgt gtcttcaaaa acattaccca 660

gcccgcctcc cgactgccag cattgtcatt tgcttctata atgaagaatg taatgccttg 720

tttcagacca tgtccagtgt cacgaacctc acgccacact attttcttga agaaattatt 780

ttggtagatg acatgagcaa agttgatgat ttgaaagaaa aactagacta tcacctggaa 840

acttttcggg gaaaggttaa aataataaga aacaaaaaga gagaggggct gattcgagca 900

aggctgattg gagcttctca tgcttcaggg gatgttctgg tgttcctgga cagccactgt 960

gaggtgaaca gagtatggct ggagcccctg ctgcatgcca ttgccaagga ccccaaaatg 1020

gtggtgtgcc ccctgataga tgtcattgat gatagaactc tggagtataa gccctctcct 1080

cttgtaaggg gaacttttga ttggaaccta caatttaaat gggataatgt tttctcttat 1140

gagatggatg gaccagaagg atctactaaa ccaatccggt cacctgcaat gtctggagga 1200

atttttgcta tacgtcggca ttattttaat gaaattggac agtatgacaa ggatatggat 1260

ttttggggaa gagaaaattt ggaactttca ctaaggatct ggatgtgtgg aggccaactc 1320

tttataatcc cctgctctcg agtaggacat atcagtaaga aacaaactgg aaaaccttct 1380

acaatcatca gtgctatgac acataactac ctaagactgg tgcacgtttg gctggatgaa 1440

tataaggagc agttttttct tcgaaagcct ggtctgaaat atgtcaccta cggaaatatt 1500

cgcgagcgtg ttgagttaag gaaacgactg ggttgcaagt catttcagtg gtatttggat 1560

aatgtcttcc cagagttgga ggcatctgtg aacagcctgt gaaaggaaaa caaatcactt 1620

tcattaataa agggttaaaa gtctcctagt cattcaacat agtgtcacaa gagtgtaagt 1680

ttggaacatc gtggaattac gtgaaatgca attaaaaaaa tatgaccaga cgtgaaaa 1738

<210> 21
<211> 731
<212> DNA
<213> Homo Sapiens

<400> 21
gagaactctt gagaccggga gcccagctgc ccaccctctg gacattcacc cagccaggtg 60

gtctcgtcac ctcagaggct ccgccagact cctgcccagg ccaggactga ggcaagcctc 120

aaggcacttc taggacctgc ctcttctcac caagatgaac tcactggttt cttggcagct 180

actgcttttc ctctgtgcca cccactttgg ggagccatta gaaaaggtgg cctctgtggg 240

gaattctaga cccacaggcc agcagctaga atccctgggc ctcctggccc ccggggagca 300

gagcctgccg tgcaccgaga ggaagccagc tgctactgcc aggctgagcc gtcgggggac 360

ctcgctgtcc ccgccccccg agagctccgg gagcccccag cagccgggcc tgtccgcccc 420

ccacagccgc cagatccccg caccccaggg cgcggtgctg gtgcagcggg agaaggacct 480

gccgaactac aactggaact ccttcggcct gcgcttcggc aagcgggagg cggcaccagg 540

gaaccacggc agaagcgctg ggcggggctg agggcgcagg tgcggggcag tgaacttcag 600

accccaaagg agtcagagca tgcggggcgg gggcgggggg cggggacgta gggctaaggg 660

agggggcgct ggagcttcca acccgaggca ataaaagaaa tgttgcgtaa ctcaaaaaaa 720

aaaaaaaaaa a 731

<210> 22
<211> 1660
<212> DNA
<213> Homo Sapiens

<400> 22
ggtgcactag caaaacaaac ttattttgaa cactcagctc ctagcgtgcg gcgctgccaa 60

tcattaacct cctggtgcaa gtggcgcggc ctgtgccctt tataaggtgc gcgctgtgtc 120

cagcgagcat cggccaccgc catcccatcc agcgagcatc tgccgccgcg ccgccgccac 180

cctcccagag agcactggcc accgctccac catcacttgc ccagagtttg ggccaccgcc 240

cgccgccacc agcccagaga gcatcggccc ctgtctgctg ctcgcgcctg gagatgtcag 300

aggtccccgt tgctcgcgtc tggctggtac tgctcctgct gactgtccag gtcggcgtga 360

cagccggcgc tccgtggcag tgcgcgccct gctccgccga gaagctcgcg ctctgcccgc 420

cggtgtccgc ctcgtgctcg gaggtcaccc ggtccgccgg ctgcggctgt tgcccgatgt 480

gcgccctgcc tctgggcgcc gcgtgcggcg tggcgactgc acgctgcgcc cggggactca 540

gttgccgcgc gctgccgggg gagcagcaac ctctgcacgc cctcacccgc ggccaaggcg 600

cctgcgtgca ggagtctgac gcctccgctc cccatgctgc agaggcaggg agccctgaaa 660

gcccagagag cacggagata actgaggagg agctcctgga taatttccat ctgatggccc 720

cttctgaaga ggatcattcc atcctttggg acgccatcag tacctatgat ggctcgaagg 780

ctctccatgt caccaacatc aaaaaatgga aggagccctg ccgaatagaa ctctacagag 840

tcgtagagag tttagccaag gcacaggaga catcaggaga agaaatttcc aaattttacc 900

tgccaaactg caacaagaat ggattttatc acagcagaca gtgtgagaca tccatggatg 960

gagaggcggg actctgctgg tgcgtctacc cttggaatgg gaagaggatc cctgggtctc 1020

cagagatcag gggagacccc aactgccaga tatattttaa tgtacaaaac tgaaaccaga 1080

tgaaataatg ttctgtcacg tgaaatattt aagtatatag tatatttata ctctagaaca 1140

tgcacattta tatatatatg tatatgtata tatatatagt aactactttt tatactccat 1200

acataacttg atatagaaag ctgtttattt attcactgta agtttatttt ttctacacag 1260

taaaaacttg tactatgtta ataacttgtc ctatgtcaat ttgtatatca tgaaacactt 1320

ctcatcatat tgtatgtaag taattgcatt tctgctcttc caaagctcct gcgtctgttt 1380

ttaaagagca tggaaaaata ctgcctagaa aatgcaaaat gaaataagag agagtagttt 1440

ttcagctagt ttgaaggagg acggttaact tgtatattcc accattcaca tttgatgtac 1500

atgtgtaggg aaagttaaaa gtgttgatta cataatcaaa gctacctgtg gtgatgttgc 1560

cacctgttaa aatgtacact ggatatgttg ttaaacacgt gtctataatg gaaacattta 1620

caataaatat tctgcatgga aatactgtta aaaaaaaaaa 1660

<210> 23
<211> 1949
<212> DNA
<213> Homo Sapiens

<400> 23
aataaagggg tctgagccgg tcgcctgagc ctgaaaagtg ctgtcacgtc agcggaagga 60

ggcgtcccag atcttctcag ctgtcttggt gccagccttc ctagtcttcc tacccacact 120

cctacctgct gtcacaggcc acagccatca tgcctcgggg tcacaagagt aagctccgta 180

cctgtgagaa acgccaagag accaatggtc agccacaggg tctcacgggt ccccaggcca 240

ctgcagagaa gcaggaagag tcccactctt cctcatcctc ttctcgcgct tgtctgggtg 300

attgtcgtag gtcttctgat gcctccattc ctcaggagtc tcagggagtg tcacccactg 360

ggtctcctga tgcagttgtt tcatattcaa aatccgatgt ggctgccaac ggccaagatg 420

agaaaagtcc aagcacctcc cgtgatgcct ccgttcctca ggagtctcag ggagcttcac 480

ccactggctc tcctgatgca ggtgtttcag gctcaaaata tgatgtggct gccaacggcc 540

aagatgagaa aagtccaagc acttcccatg atgtctccgt tcctcaggag tctcagggag 600

cttcacccac tggctcgcct gatgcaggtg tttcaggctc aaaatatgat gtggctgccg 660

agggtgaaga tgaggaaagt gtaagcgcct cacagaaagc catcattttt aagcgcttaa 720

gcaaagatgc tgtaaagaag aaggcgtgca cgttggcgca attcctgcag aagaagtttg 780

agaagaaaga gtccattttg aaggcagaca tgctgaagtg tgtccgcaga gagtacaagc 840

cctacttccc tcagatcctc aacagaacct cccaacattt ggtggtggcc tttggcgttg 900

aattgaaaga aatggattcc agcggcgagt cctacaccct tgtcagcaag ctaggcctcc 960

ccagtgaagg aattctgagt ggtgataatg cgctgccgaa gtcgggtctc ctgatgtcgc 1020

tcctggttgt gatcttcatg aacggcaact gtgccactga agaggaggtc tgggagttcc 1080

tgggtctgtt ggggatatat gatgggatcc tgcattcaat ctatggggat gctcggaaga 1140

tcattactga agatttggtg caagataagt acgtggttta ccggcaggtg tgcaacagtg 1200

atcctccatg ctatgagttc ctgtggggtc cacgagccta tgctgaaacc accaagatga 1260

gagtcctgcg tgttttggcc gacagcagta acaccagtcc cggtttatac ccacatctgt 1320

atgaagacgc tttgatagat gaggtagaga gagcattgag actgagagct taaggcaggg 1380

ctggcactat ttccttggcc agggtacctt atggggccat atcctacaga tcctcccatt 1440

tctagggagg tctgaagtag aattttcact ttatgttaga agagagtagt gagctttcta 1500

agtagtgcag tatagtagag gctggaggga acaagatatg tatctttctt ttgttacaca 1560

tgagtaactt gcagatttat gttttatctc tgtcagttat caacattgtt cctgttaagt 1620

gaaggtttat tttgcttcag attatacaat tatcaataac atagctctca cattcatggc 1680

tgtttaacca atctgaaagt tacggtttgg gaattaataa aacaaagtca tacaacacat 1740

tttctttgta attgagaact agataacatg gtaacagaga attgattttc atatgaatct 1800

taactccaca gtaaaatagt tgacatcata atatgaagag aaagaaaagg aaaaacagaa 1860

atgtaaaagt tgtttaattc ttggtttgcc taattcgttt tcctatttct tttcatacaa 1920

ataaaggata cctggattta tttaggtta 1949

<210> 24
<211> 79
<212> PRT
<213> Homo Sapiens

<400> 24

Met Cys Cys Asn Tyr Tyr Gly Asn Ser Cys Gly Tyr Gly Ser Gly Cys
1               5                   10                  15

Gly Cys Gly Tyr Gly Ser Gly Ser Gly Cys Gly Cys Gly Tyr Gly Thr
            20                  25                  30

Gly Tyr Gly Cys Gly Tyr Gly Cys Gly Phe Gly Ser His Tyr Gly Cys
        35                  40                  45

Gly Tyr Gly Thr Gly Tyr Gly Cys Gly Tyr Gly Ser Gly Ser Gly Tyr
    50                  55                  60

Cys Gly Tyr Arg Pro Phe Cys Phe Arg Arg Cys Tyr Ser Ser Cys
65                  70                  75

<210>  25
<211>  70
<212>  PRT
<213>  Homo Sapiens

<400>  25

Met Lys Gln Lys Gln Glu Val Met Phe Gln Ser Arg Gly Arg Leu Ser
1               5                   10                  15

Leu Tyr Ile Gln Met Ser Ser Val Tyr Ser Ala Lys Leu Gly Pro Val
            20                  25                  30

Gly Gly Ile Cys Gly Gln Lys Gln Lys Pro Ser Phe Phe Phe Phe Lys
        35                  40                  45

Ala Gln Ser Gln Asp Ala Arg Pro Leu Ala Pro Ala Ala Cys Ile Ser
    50                  55                  60

Lys Ile Ala Lys Ala Gly
65                  70

<210>  26
<211>  265
<212>  PRT
<213>  Homo Sapiens

<400>  26

Met Glu Ala Leu Pro Pro Val Arg Ser Ser Leu Leu Gly Ile Leu Leu
1               5                   10                  15

Gln Val Thr Arg Leu Ser Val Leu Leu Val Gln Asn Arg Asp His Leu
            20                  25                  30

Tyr Asn Phe Leu Leu Leu Lys Ile Asn Leu Phe Asn His Trp Val Ser
        35                  40                  45

Gly Leu Ala Gln Glu Ala Arg Gly Ser Cys Asn Trp Gln Ala His Leu
    50                  55                  60

Pro Leu Gly Ala Ala Ala Cys Pro Leu Gly Gln Ala Leu Trp Ala Gly
65                  70                  75                  80

Leu Ala Leu Ile Gln Val Pro Val Trp Leu Val Leu Gln Gly Pro Arg
                85                  90                  95

Leu Met Trp Ala Gly Met Trp Gly Ser Thr Lys Gly Leu Gly Leu Ala
            100                 105                 110

Leu Leu Ser Ala Trp Glu Gln Leu Gly Leu Ser Val Ala Ile Trp Thr
        115                 120                 125

Asp Leu Phe Leu Ser Cys Leu His Gly Leu Met Leu Val Ala Leu Leu
    130                 135                 140

Leu Val Val Val Thr Trp Arg Val Cys Gln Lys Ser His Cys Phe Arg
145                 150                 155                 160

Leu Gly Arg Gln Leu Ser Lys Ala Leu Gln Val Asn Cys Val Val Arg
                165                 170                 175

Lys Leu Leu Val Gln Leu Arg Arg Leu Tyr Trp Trp Val Glu Thr Met
            180                 185                 190

Thr Ala Leu Thr Ser Trp His Leu Ala Tyr Leu Ile Thr Trp Thr Thr
        195                 200                 205

Cys Leu Ala Ser His Leu Leu Gln Ala Ala Phe Glu His Thr Thr Gln
    210                 215                 220

Leu Ala Glu Ala Gln Glu Val Glu Pro Gln Glu Val Ser Gly Ser Ser
225                 230                 235                 240

Leu Leu Pro Ser Leu Ser Ala Ser Ser Asp Ser Glu Ser Gly Thr Val
                245                 250                 255

Leu Pro Glu Gln Glu Thr Pro Arg Glu
            260                 265

<210>  27
<211>  533
<212>  PRT
<213>  Homo Sapiens

<400>  27

Met Asn Glu Glu Asn Ile Asp Gly Thr Asn Gly Cys Ser Lys Val Arg
1               5                   10                  15

Thr Gly Ile Gln Asn Glu Ala Ala Leu Leu Ala Leu Met Glu Lys Thr
            20                  25                  30

Gly Tyr Asn Met Val Gln Glu Asn Gly Gln Arg Lys Phe Gly Gly Pro

35                  40                  45

Pro Pro Gly Trp Glu Gly Pro Pro Pro Pro Arg Gly Cys Glu Val Phe
    50                  55                  60

Val Gly Lys Ile Pro Arg Asp Met Tyr Glu Asp Glu Leu Val Pro Val
65                  70                  75                  80

Phe Glu Arg Ala Gly Lys Ile Tyr Glu Phe Arg Leu Met Met Glu Phe
                85                  90                  95

Ser Gly Glu Asn Arg Gly Tyr Ala Phe Val Met Tyr Thr Thr Lys Glu
            100                 105                 110

Glu Ala Gln Leu Ala Ile Arg Ile Leu Asn Asn Tyr Glu Ile Arg Pro
        115                 120                 125

Gly Lys Phe Ile Gly Val Cys Val Ser Leu Asp Asn Cys Arg Leu Phe
    130                 135                 140

Ile Gly Ala Ile Pro Lys Glu Lys Lys Lys Glu Glu Ile Leu Asp Glu
145                 150                 155                 160

Met Lys Lys Val Thr Glu Gly Val Val Asp Val Ile Val Tyr Pro Ser
                165                 170                 175

Ala Thr Asp Lys Thr Lys Asn Arg Gly Phe Ala Phe Val Glu Tyr Glu
            180                 185                 190

Ser His Arg Ala Ala Ala Met Ala Arg Arg Lys Leu Ile Pro Gly Thr
        195                 200                 205

Phe Gln Leu Trp Gly His Thr Ile Gln Val Asp Trp Ala Asp Pro Glu
    210                 215                 220

Lys Glu Val Asp Glu Glu Thr Met Gln Arg Val Lys Val Leu Tyr Val
225                 230                 235                 240

Arg Asn Leu Met Ile Ser Thr Thr Glu Glu Thr Ile Lys Ala Glu Phe
                245                 250                 255

Asn Lys Phe Lys Pro Gly Ala Val Glu Arg Val Lys Lys Leu Arg Asp
            260                 265                 270

Tyr Ala Phe Val His Phe Phe Asn Arg Glu Asp Ala Val Ala Ala Met
        275                 280                 285

Ser Val Met Asn Gly Lys Cys Ile Asp Gly Ala Ser Ile Glu Val Thr
    290                 295                 300

Leu Ala Lys Pro Val Asn Lys Glu Asn Thr Trp Arg Gln His Leu Asn
305                 310                 315                 320

Gly Gln Ile Ser Pro Asn Ser Glu Asn Leu Ile Val Phe Ala Asn Lys
                325                 330                 335

Glu Glu Ser His Pro Lys Thr Leu Gly Lys Leu Pro Thr Leu Pro Ala
            340                 345                 350

Arg Leu Asn Gly Gln His Ser Pro Ser Pro Pro Glu Val Glu Arg Cys
        355                 360                 365

Thr Tyr Pro Phe Tyr Pro Gly Thr Lys Leu Thr Pro Ile Ser Met Tyr
    370                 375                 380

Ser Leu Lys Ser Asn His Phe Asn Ser Ala Val Met His Leu Asp Tyr
385                 390                 395                 400

Tyr Cys Asn Lys Asn Asn Trp Ala Pro Pro Glu Tyr Tyr Leu Tyr Ser
                405                 410                 415

Thr Thr Ser Gln Asp Gly Lys Val Leu Leu Val Tyr Lys Ile Val Ile
            420                 425                 430

Pro Ala Ile Ala Asn Gly Ser Gln Ser Tyr Phe Met Pro Asp Lys Leu
        435                 440                 445

Cys Thr Thr Leu Glu Asp Ala Lys Glu Leu Ala Ala Gln Phe Thr Leu
    450                 455                 460

Leu His Leu Asp Tyr Asn Phe His Arg Ser Ser Ile Asn Ser Leu Ser
465                 470                 475                 480

Pro Val Ser Ala Thr Leu Ser Ser Gly Thr Pro Ser Val Leu Pro Tyr
                485                 490                 495

Thr Ser Arg Pro Tyr Ser Tyr Pro Gly Tyr Pro Leu Ser Pro Thr Ile
            500                 505                 510

Ser Leu Ala Asn Gly Ser His Val Gly Gln Arg Leu Cys Ile Ser Asn
        515                 520                 525

Gln Ala Ser Phe Phe
    530

<210>  28
<211>  123
<212>  PRT
<213>  Homo Sapiens

<400>  28

Met Pro Asp Ala Ser Leu Gly Ser Leu Gly Ile Thr Trp Cys Phe Leu
1               5                   10                  15

Glu Ser Pro Leu Glu Val Ser Ser Gly Arg Phe Gly Leu Ala Arg Leu
            20                  25                  30

Leu Gly Ser Gln Asp His Gly Asp Asp Pro Ala Glu Arg Gly Arg Thr
        35                  40                  45

Ala Thr Asp Ala Trp Gly Pro Ser Arg Trp Gly Gln Ser Pro Gly Asn
    50                  55                  60

Gly Gly Gly Tyr Cys Asp Ala Ser Pro Pro Ser Ala Leu Ala Pro Gly
65                  70                  75                  80

Asp Arg Ala Trp Ala Leu Pro Ala Ser Pro Ser Ser Gly Ala Pro Ala
                85                  90                  95

Ser Gln His Cys Cys Leu Glu Lys Ala Gly Thr Arg Thr Lys Ala Ser
            100                 105                 110

Pro Val Trp Gly Arg Asp Gly Asn Thr Trp Asn
        115                 120

<210>  29
<211>  638
<212>  PRT
<213>  Homo Sapiens

<400>  29

Met Val Val Ser Ala Asp Pro Leu Ser Ser Glu Arg Ala Glu Met Asn
1               5                   10                  15

Ile Leu Glu Ile Asn Gln Glu Leu Arg Ser Gln Leu Ala Glu Ser Asn
            20                  25                  30

Gln Gln Phe Arg Asp Leu Lys Glu Lys Phe Leu Ile Thr Gln Ala Thr
        35                  40                  45

Ala Tyr Ser Leu Ala Asn Gln Leu Lys Lys Tyr Lys Cys Glu Glu Tyr
    50                  55                  60

Lys Asp Ile Ile Asp Ser Val Leu Arg Asp Glu Leu Gln Ser Met Glu
65                  70                  75                  80

Lys Leu Ala Glu Lys Leu Arg Gln Ala Glu Glu Leu Arg Gln Tyr Lys
                85                  90                  95

Ala Leu Val His Ser Gln Ala Lys Glu Leu Thr Gln Leu Arg Glu Lys
            100                 105                 110

Leu Arg Glu Gly Arg Asp Ala Ser Arg Trp Leu Asn Lys His Leu Lys
        115                 120                 125

Thr Leu Leu Thr Pro Asp Asp Pro Asp Lys Ser Gln Gly Gln Asp Leu
    130                 135                 140

Arg Glu Gln Leu Ala Glu Gly His Arg Leu Ala Glu His Leu Val His
145                 150                 155                 160

Lys Leu Ser Pro Glu Asn Asp Glu Asp Glu Asp Glu Asp Glu Asp Asp
                165                 170                 175

Lys Asp Glu Glu Val Glu Lys Val Gln Glu Ser Pro Ala Pro Arg Glu
            180                 185                 190

Val Gln Lys Thr Glu Glu Lys Glu Val Pro Gln Asp Ser Leu Glu Glu
        195                 200                 205

Cys Ala Val Thr Cys Ser Asn Ser His Asn Pro Ser Asn Ser Asn Gln
    210                 215                 220

Pro His Arg Ser Thr Lys Ile Thr Phe Lys Glu His Glu Val Asp Ser
225                 230                 235                 240

Ala Leu Val Val Glu Ser Glu His Pro His Asp Glu Glu Glu Glu Ala
                245                 250                 255

Leu Asn Ile Pro Pro Glu Asn Gln Asn Asp His Glu Glu Glu Glu Gly
            260                 265                 270

Lys Ala Pro Val Pro Pro Arg His His Asp Lys Ser Asn Ser Tyr Arg
        275                 280                 285

His Arg Glu Val Ser Phe Leu Ala Leu Asp Glu Gln Lys Val Cys Ser
    290                 295                 300

Ala Gln Asp Val Ala Arg Asp Tyr Ser Asn Pro Lys Trp Asp Glu Thr
305                 310                 315                 320

Ser Leu Gly Phe Leu Glu Lys Gln Ser Asp Leu Glu Glu Val Lys Gly
                325                 330                 335

Gln Glu Thr Val Ala Pro Arg Leu Ser Arg Gly Pro Leu Arg Val Asp
            340                 345                 350

Lys His Glu Ile Pro Gln Glu Ser Leu Asp Gly Cys Cys Leu Thr Pro
        355                 360                 365

Ser Ile Leu Pro Asp Leu Thr Pro Ser Tyr His Pro Tyr Trp Ser Thr
    370                 375                 380

Leu Tyr Ser Phe Glu Asp Lys Gln Val Ser Leu Ala Leu Val Asp Lys
385                 390                 395                 400

Ile Lys Lys Asp Gln Glu Glu Ile Glu Asp Gln Ser Pro Pro Cys Pro
                405                 410                 415

Arg Leu Ser Gln Glu Leu Pro Glu Val Lys Glu Gln Glu Val Pro Glu
            420                 425                 430

Asp Ser Val Asn Glu Val Tyr Leu Thr Pro Ser Val His His Asp Val
        435                 440                 445

Ser Asp Cys His Gln Pro Tyr Ser Ser Thr Leu Ser Ser Leu Glu Asp
    450                 455                 460

Gln Leu Ala Cys Ser Ala Leu Asp Val Ala Ser Pro Thr Glu Ala Ala
465                 470                 475                 480

Cys Pro Gln Gly Thr Trp Ser Gly Asp Leu Ser His His Gln Ser Glu
                485                 490                 495

Val Gln Val Ser Gln Ala Gln Leu Glu Pro Ser Thr Leu Val Pro Ser
            500                 505                 510

Cys Leu Arg Leu Gln Leu Asp Gln Gly Phe His Cys Gly Asn Gly Leu
        515                 520                 525

Ala Gln Arg Gly Leu Ser Ser Thr Thr Cys Ser Phe Ser Ala Asn Ala
    530                 535                 540

Asp Ser Gly Asn Gln Trp Pro Phe Gln Glu Leu Val Leu Glu Pro Ser
545                 550                 555                 560

Leu Gly Met Lys Asn Pro Pro Gln Leu Glu Asp Asp Ala Leu Glu Gly
                565                 570                 575

Ser Ala Ser Asn Thr Gln Gly Arg Gln Val Thr Gly Arg Ile Arg Ala
            580                 585                 590

Ser Leu Val Leu Ile Leu Lys Thr Ile Arg Arg Arg Leu Pro Phe Ser
        595                 600                 605

Lys Trp Arg Leu Ala Phe Arg Phe Ala Gly Pro His Ala Glu Ser Ala
    610                 615                 620

Glu Ile Pro Asn Thr Ala Gly Arg Thr Gln Arg Met Ala Gly
625                 630                 635

<210>  30
<211>  909
<212>  PRT
<213>  Homo Sapiens

<400>  30

Met Ala Ala Arg Pro Leu Pro Val Ser Pro Ala Arg Ala Leu Leu Leu
1               5                   10                  15

Ala Leu Ala Gly Ala Leu Leu Ala Pro Cys Glu Ala Arg Gly Val Ser
            20                  25                  30

Leu Trp Asn Gln Gly Arg Ala Asp Glu Val Val Ser Ala Ser Val Gly
        35                  40                  45

Ser Gly Asp Leu Trp Ile Pro Val Lys Ser Phe Asp Ser Lys Asn His
    50                  55                  60

Pro Glu Val Leu Asn Ile Arg Leu Gln Arg Glu Ser Lys Glu Leu Ile
65                  70                  75                  80

Ile Asn Leu Glu Arg Asn Glu Gly Leu Ile Ala Ser Ser Phe Thr Glu
                85                  90                  95

Thr His Tyr Leu Gln Asp Gly Thr Asp Val Ser Leu Ala Arg Asn Tyr
            100                 105                 110

Thr Val Ile Leu Gly His Cys Tyr Tyr His Gly His Val Arg Gly Tyr
        115                 120                 125

Ser Asp Ser Ala Val Ser Leu Ser Thr Cys Ser Gly Leu Arg Gly Leu
    130                 135                 140

Ile Val Phe Glu Asn Glu Ser Tyr Val Leu Glu Pro Met Lys Ser Ala
145                 150                 155                 160

Thr Asn Arg Tyr Lys Leu Phe Pro Ala Lys Lys Leu Lys Ser Val Arg
                165                 170                 175

Gly Ser Cys Gly Ser His His Asn Thr Pro Asn Leu Ala Ala Lys Asn
            180                 185                 190

Val Phe Pro Pro Pro Ser Gln Thr Trp Ala Arg Arg His Lys Arg Glu
        195                 200                 205

Thr Leu Lys Ala Thr Lys Tyr Val Glu Leu Val Ile Val Ala Asp Asn
    210                 215                 220

Arg Glu Phe Gln Arg Gln Gly Lys Asp Leu Glu Lys Val Lys Gln Arg
225                 230                 235                 240

Leu Ile Glu Ile Ala Asn His Val Asp Lys Phe Tyr Arg Pro Leu Asn
                245                 250                 255

Ile Arg Ile Val Leu Val Gly Val Glu Val Trp Asn Asp Met Asp Lys
            260                 265                 270

Cys Ser Val Ser Gln Asp Pro Phe Thr Ser Leu His Glu Phe Leu Asp
        275                 280                 285

Trp Arg Lys Met Lys Leu Leu Pro Arg Lys Ser His Asp Asn Ala Gln
    290                 295                 300

Leu Val Ser Gly Val Tyr Phe Gln Gly Thr Thr Ile Gly Met Ala Pro
305                 310                 315                 320

Ile Met Ser Met Cys Thr Ala Asp Gln Ser Gly Gly Ile Val Met Asp
                325                 330                 335

His Ser Asp Asn Pro Leu Gly Ala Ala Val Thr Leu Ala His Glu Leu
            340                 345                 350

Gly His Asn Phe Gly Met Asn His Asp Thr Leu Asp Arg Gly Cys Ser
        355                 360                 365

Cys Gln Met Ala Val Glu Lys Gly Gly Cys Ile Met Asn Ala Ser Thr
    370                 375                 380

Gly Tyr Pro Phe Pro Met Val Phe Ser Ser Cys Ser Arg Lys Asp Leu
385                 390                 395                 400

Glu Thr Ser Leu Glu Lys Gly Met Gly Val Cys Leu Phe Asn Leu Pro
                405                 410                 415

Glu Val Arg Glu Ser Phe Gly Gly Gln Lys Cys Gly Asn Arg Phe Val
            420                 425                 430

Glu Glu Gly Glu Glu Cys Asp Cys Gly Glu Pro Glu Glu Cys Met Asn
        435                 440                 445

Arg Cys Cys Asn Ala Thr Thr Cys Thr Leu Lys Pro Asp Ala Val Cys
    450                 455                 460

Ala His Gly Leu Cys Cys Glu Asp Cys Gln Leu Lys Pro Ala Gly Thr
465                 470                 475                 480

Ala Cys Arg Asp Ser Ser Asn Ser Cys Asp Leu Pro Glu Phe Cys Thr
                485                 490                 495

Gly Ala Ser Pro His Cys Pro Ala Asn Val Tyr Leu His Asp Gly His
            500                 505                 510

Ser Cys Gln Asp Val Asp Gly Tyr Cys Tyr Asn Gly Ile Cys Gln Thr
        515                 520                 525

His Glu Gln Gln Cys Val Thr Leu Trp Gly Pro Gly Ala Lys Pro Ala
    530                 535                 540

Pro Gly Ile Cys Phe Glu Arg Val Asn Ser Ala Gly Asp Pro Tyr Gly
545                 550                 555                 560

Asn Cys Gly Lys Val Ser Lys Ser Ser Phe Ala Lys Cys Glu Met Arg
                565                 570                 575

Asp Ala Lys Cys Gly Lys Ile Gln Cys Gln Gly Gly Ala Ser Arg Pro
            580                 585                 590

Val Ile Gly Thr Asn Ala Val Ser Ile Glu Thr Asn Ile Pro Leu Gln
        595                 600                 605

Gln Gly Gly Arg Ile Leu Cys Arg Gly Thr His Val Tyr Leu Gly Asp
    610                 615                 620

Asp Met Pro Asp Pro Gly Leu Val Leu Ala Gly Thr Lys Cys Ala Asp
625                 630                 635                 640

Gly Lys Ile Cys Leu Asn Arg Gln Cys Gln Asn Ile Ser Val Phe Gly
                645                 650                 655

Val His Glu Cys Ala Met Gln Cys His Gly Arg Gly Val Cys Asn Asn
            660                 665                 670

Arg Lys Asn Cys His Cys Glu Ala His Trp Ala Pro Pro Phe Cys Asp
        675                 680                 685

Lys Phe Gly Phe Gly Gly Ser Thr Asp Ser Gly Pro Ile Arg Gln Ala
    690                 695                 700

Asp Asn Gln Gly Leu Thr Ile Gly Ile Leu Val Thr Ile Leu Cys Leu
705                 710                 715                 720

Leu Ala Ala Gly Phe Val Val Tyr Leu Lys Arg Lys Thr Leu Ile Arg
                725                 730                 735

Leu Leu Phe Thr Asn Lys Lys Thr Thr Ile Glu Lys Leu Arg Cys Val
            740                 745                 750

Arg Pro Ser Arg Pro Pro Arg Gly Phe Gln Pro Cys Gln Ala His Leu
        755                 760                 765

Gly His Leu Gly Lys Gly Leu Met Arg Lys Pro Pro Asp Ser Tyr Pro
    770                 775                 780

Pro Lys Asp Asn Pro Arg Arg Leu Leu Gln Cys Gln Asn Val Asp Ile
785                 790                 795                 800

Ser Arg Pro Leu Asn Gly Leu Asn Val Pro Gln Pro Gln Ser Thr Gln
                805                 810                 815

Arg Val Leu Pro Pro Leu His Arg Ala Pro Arg Ala Pro Ser Val Pro
            820                 825                 830

Ala Arg Pro Leu Pro Ala Lys Pro Ala Leu Arg Gln Ala Gln Gly Thr

835                 840                 845

Cys Lys Pro Asn Pro Pro Gln Lys Pro Leu Pro Ala Asp Pro Leu Ala
    850                 855                 860

Arg Thr Thr Arg Leu Thr His Ala Leu Ala Arg Thr Pro Gly Gln Trp
865                 870                 875                 880

Glu Thr Gly Leu Arg Leu Ala Pro Leu Arg Pro Ala Pro Gln Tyr Pro
                885                 890                 895

His Gln Val Pro Arg Ser Thr His Thr Ala Tyr Ile Lys
            900                 905

<210>  31
<211>  0
<212>  PRT
<213>  Homo Sapiens

<400>  31
000
<210>  32
<211>  753
<212>  PRT
<213>  Homo Sapiens

<400>  32

<210>  33
<211>  113
<212>  PRT
<213>  Homo Sapiens

<400>  33

Met Glu Ile Lys Lys Glu Thr Met Leu Asp Asp Thr Gly Ala Asp Asn
1               5                   10                  15

Lys Arg Arg Glu Pro Lys His Trp Gln Met Arg His Tyr Pro Lys Asn
            20                  25                  30

Thr Gly Pro Glu Tyr Val Cys Gln Leu Gly Ser Asp Arg Asn Arg Ser
        35                  40                  45

Leu Thr Gln His Thr Phe Ile Ala Leu Leu Leu His Ala Lys Lys Cys
    50                  55                  60

Thr Lys Ile Trp Asp Cys Val Thr Gln Arg Arg Lys Arg Leu Ile Ser
65                  70                  75                  80

Arg Arg Pro Pro Cys Leu Gly Arg Glu Thr Glu His Gln His Lys Val
                85                  90                  95

Val Lys Ser Tyr Glu Gly Gly Glu Cys His Glu Ser Thr Glu Glu Gly
            100                 105                 110

Phe

<210>  34
<211>  475
<212>  PRT
<213>  Homo Sapiens

<400>  34

Met Ala Ala Ser Ala Leu Arg Gly Leu Pro Val Ala Gly Gly Gly Glu
1               5                   10                  15

Ser Ser Glu Ser Glu Asp Asp Gly Trp Glu Ile Gly Tyr Leu Asp Arg
            20                  25                  30

Thr Ser Gln Lys Leu Lys Arg Leu Leu Pro Ile Glu Glu Lys Lys Glu
        35                  40                  45

Lys Phe Lys Lys Ala Met Thr Ile Gly Asp Val Ser Leu Val Gln Glu
    50                  55                  60

Leu Leu Asp Ser Gly Ile Ser Val Asp Ser Asn Phe Gln Tyr Gly Trp
65                  70                  75                  80

Thr Pro Leu Met Tyr Ala Ala Ser Val Ala Asn Ala Glu Leu Val Arg
                85                  90                  95

Val Leu Leu Asp Arg Gly Ala Asn Ala Ser Phe Glu Lys Asp Lys Gln
            100                 105                 110

Ser Ile Leu Ile Thr Ala Cys Ser Ala His Gly Ser Glu Glu Gln Ile
        115                 120                 125

Leu Lys Cys Val Glu Leu Leu Leu Ser Arg Asn Ala Asp Pro Asn Val
    130                 135                 140

Ala Cys Arg Arg Leu Met Thr Pro Ile Met Tyr Ala Ala Arg Asp Gly
145                 150                 155                 160

His Thr Gln Val Val Ala Leu Leu Val Ala His Gly Ala Glu Val Asn
                165                 170                 175

Thr Gln Asp Glu Asn Gly Tyr Thr Ala Leu Thr Trp Ala Ala Arg Gln
            180                 185                 190

Gly His Lys Asn Ile Val Leu Lys Leu Leu Glu Leu Gly Ala Asn Lys
        195                 200                 205

Met Leu Gln Thr Lys Asp Gly Lys Met Pro Ser Glu Ile Ala Lys Arg
    210                 215                 220

Asn Lys His His Glu Ile Phe Asn Leu Leu Ser Phe Thr Leu Asn Pro
225                 230                 235                 240

Leu Glu Gly Lys Leu Gln Gln Leu Thr Lys Glu Asp Thr Ile Cys Lys
                245                 250                 255

Ile Leu Thr Thr Asp Ser Asp Arg Glu Lys Asp His Ile Phe Ser Ser
            260                 265                 270

Tyr Thr Ala Phe Gly Asp Leu Glu Val Phe Leu His Gly Leu Gly Leu
        275                 280                 285

Glu His Met Thr Asp Leu Leu Lys Glu Arg Asp Ile Thr Leu Arg His
    290                 295                 300

Leu Leu Thr Met Arg Glu Asp Glu Phe Thr Lys Asn Gly Ile Thr Ser
305                 310                 315                 320

Lys Asp Gln Gln Lys Ile Leu Ala Ala Leu Lys Glu Leu Gln Val Glu
                325                 330                 335

Glu Ile Gln Phe Gly Glu Leu Ser Glu Glu Thr Lys Leu Glu Ile Ser
            340                 345                 350

Gly Asp Glu Phe Leu Asn Phe Leu Leu Lys Leu Asn Lys Gln Cys Gly
        355                 360                 365

His Leu Ile Thr Ala Val Gln Asn Val Ile Thr Glu Leu Pro Val Asn
    370                 375                 380

Ser Gln Lys Ile Thr Leu Glu Trp Ala Ser Pro Gln Asn Phe Thr Ser
385                 390                 395                 400

Val Cys Glu Glu Leu Val Asn Asn Val Glu Asp Leu Ser Glu Lys Val
                405                 410                 415

Cys Lys Leu Lys Asp Leu Ile Gln Lys Leu Gln Asn Glu Arg Glu Asn
            420                 425                 430

Asp Pro Thr His Ile Gln Leu Arg Glu Glu Val Ser Thr Trp Asn Ser
        435                 440                 445

Arg Ile Leu Lys Arg Thr Ala Ile Thr Ile Cys Gly Phe Gly Phe Leu
    450                 455                 460

Leu Phe Ile Cys Lys Leu Thr Phe Gln Arg Lys
465                 470                 475

<210>  35
<211>  520
<212>  PRT
<213>  Homo Sapiens

<400>  35

Met Glu Phe His Asn Gly Gly His Val Ser Gly Ile Gly Gly Phe Leu
1               5                   10                  15

Val Ser Leu Thr Ser Arg Met Lys Pro His Thr Leu Ala Val Thr Pro
            20                  25                  30

Ala Leu Ile Phe Ala Ile Thr Val Ala Thr Ile Gly Ser Phe Gln Phe
        35                  40                  45

Gly Tyr Asn Thr Gly Val Ile Asn Ala Pro Glu Thr Ile Ile Lys Glu
    50                  55                  60

Phe Ile Asn Lys Thr Leu Thr Asp Lys Ala Asn Ala Pro Pro Ser Glu
65                  70                  75                  80

Val Leu Leu Thr Asn Leu Trp Ser Leu Ser Val Ala Ile Phe Ser Val
                85                  90                  95

Gly Gly Met Ile Gly Ser Phe Ser Val Gly Leu Phe Val Asn Arg Phe
            100                 105                 110

Gly Arg Arg Asn Ser Met Leu Ile Val Asn Leu Leu Ala Ala Thr Gly

115                 120                 125

Gly Cys Leu Met Gly Leu Cys Lys Ile Ala Glu Ser Val Glu Met Leu
    130                 135                 140

Ile Leu Gly Arg Leu Val Ile Gly Leu Phe Cys Gly Leu Cys Thr Gly
145                 150                 155                 160

Phe Val Pro Met Tyr Ile Gly Glu Ile Ser Pro Thr Ala Leu Arg Gly
                165                 170                 175

Ala Phe Gly Thr Leu Asn Gln Leu Gly Ile Val Ile Gly Ile Leu Val
            180                 185                 190

Ala Gln Ile Phe Gly Leu Glu Leu Ile Leu Gly Ser Glu Glu Leu Trp
        195                 200                 205

Pro Val Leu Leu Gly Phe Thr Ile Leu Pro Ala Ile Leu Gln Ser Ala
    210                 215                 220

Ala Leu Pro Cys Cys Pro Glu Ser Pro Arg Phe Leu Leu Ile Asn Arg
225                 230                 235                 240

Lys Lys Glu Glu Asn Ala Thr Arg Ile Leu Gln Arg Leu Trp Gly Thr
                245                 250                 255

Gln Asp Val Ser Gln Asp Ile Gln Glu Met Lys Asp Glu Ser Ala Arg
            260                 265                 270

Met Ser Gln Glu Lys Gln Val Thr Val Leu Glu Leu Phe Arg Val Ser
        275                 280                 285

Ser Tyr Arg Gln Pro Ile Ile Ile Ser Ile Val Leu Gln Leu Ser Gln
    290                 295                 300

Gln Leu Ser Gly Ile Asn Ala Val Phe Tyr Tyr Ser Thr Gly Ile Phe
305                 310                 315                 320

Lys Asp Ala Gly Val Gln Gln Pro Ile Tyr Ala Thr Ile Ser Ala Gly
                325                 330                 335

Val Val Asn Thr Ile Phe Thr Leu Leu Ser Leu Phe Leu Val Glu Arg
            340                 345                 350

Ala Gly Arg Arg Thr Leu His Met Ile Gly Leu Gly Gly Met Ala Phe
        355                 360                 365

Cys Ser Thr Leu Met Thr Val Ser Leu Leu Leu Lys Asn His Tyr Asn
    370                 375                 380

Gly Met Ser Phe Val Cys Ile Gly Ala Ile Leu Val Phe Val Ala Cys
385                 390                 395                 400

Phe Glu Ile Gly Pro Gly Pro Ile Pro Trp Phe Ile Val Ala Glu Leu
                405                 410                 415

Phe Ser Gln Gly Pro Arg Pro Ala Ala Met Ala Val Ala Gly Cys Ser
            420                 425                 430

Asn Trp Thr Ser Asn Phe Leu Val Gly Leu Leu Phe Pro Ser Ala Ala
        435                 440                 445

Tyr Tyr Leu Gly Ala Tyr Val Phe Ile Ile Phe Thr Gly Phe Leu Ile
    450                 455                 460

Thr Phe Leu Ala Phe Thr Phe Phe Lys Val Pro Glu Thr Arg Gly Arg
465                 470                 475                 480

Thr Phe Glu Asp Ile Thr Arg Ala Phe Glu Gly Gln Ala His Gly Ala
                485                 490                 495

Asp Arg Ser Gly Lys Asp Gly Val Met Gly Met Asn Ser Ile Glu Pro
            500                 505                 510

Ala Lys Glu Thr Thr Thr Asn Val
        515                 520


<210>  36
<211>  72
<212>  PRT
<213>  Homo Sapiens

<400>  36

Met Pro Leu Leu Arg Gly Arg Cys Pro Ala Arg Arg His Tyr Arg Arg
1               5                   10                  15

Leu Ala Leu Leu Gly Leu Gln Pro Ala Pro Arg Phe Ala His Ser Gly
            20                  25                  30

Pro Pro Arg Gln Arg Pro Leu Ser Ala Ala Glu Met Ala Val Gly Leu
        35                  40                  45

Val Val Phe Phe Thr Thr Phe Leu Thr Pro Ala Ala Tyr Val Leu Gly
    50                  55                  60

Asn Leu Lys Gln Phe Arg Arg Asn
65                  70

<210>  37
<211>  626
<212>  PRT
<213>  Homo Sapiens

<400>  37

Met Met Ala Asn Asp Ala Lys Pro Asp Val Lys Thr Val Gln Val Leu
1               5                   10                  15

Arg Asp Thr Ala Asn Arg Leu Arg Ile His Ser Ile Arg Ala Thr Cys
            20                  25                  30

Ala Ser Gly Ser Gly Gln Leu Thr Ser Cys Cys Ser Ala Ala Glu Val
        35                  40                  45

Val Ser Val Leu Phe Phe His Thr Met Lys Tyr Lys Gln Thr Asp Pro
    50                  55                  60

Glu His Pro Asp Asn Asp Arg Phe Ile Leu Ser Arg Gly His Ala Ala
65                  70                  75                  80

Pro Ile Leu Tyr Ala Ala Trp Val Glu Val Gly Asp Ile Ser Glu Ser
                85                  90                  95

Asp Leu Leu Asn Leu Arg Lys Leu His Ser Asp Leu Glu Arg His Pro
            100                 105                 110

Thr Pro Arg Leu Pro Phe Val Asp Val Ala Thr Gly Ser Leu Gly Gln
        115                 120                 125

Gly Leu Gly Thr Ala Cys Gly Met Ala Tyr Thr Gly Lys Tyr Leu Asp
    130                 135                 140

Lys Ala Ser Tyr Arg Val Phe Cys Leu Met Gly Asp Gly Glu Ser Ser
145                 150                 155                 160

Glu Gly Ser Val Trp Glu Ala Phe Ala Phe Ala Ser His Tyr Asn Leu
                165                 170                 175

Asp Asn Leu Val Ala Val Phe Asp Val Asn Arg Leu Gly Gln Ser Gly
            180                 185                 190

Pro Ala Pro Leu Glu His Gly Ala Asp Ile Tyr Gln Asn Cys Cys Glu
        195                 200                 205

Ala Phe Gly Trp Asn Thr Tyr Leu Val Asp Gly His Asp Val Glu Ala
    210                 215                 220

Leu Cys Gln Ala Phe Trp Gln Ala Ser Gln Val Lys Asn Lys Pro Thr
225                 230                 235                 240

Ala Ile Val Ala Lys Thr Phe Lys Gly Arg Gly Ile Pro Asn Ile Glu
                245                 250                 255

Asp Ala Glu Asn Trp His Gly Lys Pro Val Pro Lys Glu Arg Ala Asp
            260                 265                 270

Ala Ile Val Lys Leu Ile Glu Ser Gln Ile Gln Thr Asn Glu Asn Leu
        275                 280                 285

Ile Pro Lys Ser Pro Val Glu Asp Ser Pro Gln Ile Ser Ile Thr Asp
    290                 295                 300

Ile Lys Met Thr Ser Pro Pro Ala Tyr Lys Val Gly Asp Lys Ile Ala
305                 310                 315                 320

Thr Gln Lys Thr Tyr Gly Leu Ala Leu Ala Lys Leu Gly Arg Ala Asn
                325                 330                 335

Glu Arg Val Ile Val Leu Ser Gly Asp Thr Met Asn Ser Thr Phe Ser
            340                 345                 350

Glu Ile Phe Arg Lys Glu His Pro Glu Arg Phe Ile Glu Cys Ile Ile
        355                 360                 365

Ala Glu Gln Asn Met Val Ser Val Ala Leu Gly Cys Ala Thr Arg Gly
    370                 375                 380

Arg Thr Ile Ala Phe Ala Gly Ala Phe Ala Ala Phe Phe Thr Arg Ala
385                 390                 395                 400

Phe Asp Gln Leu Arg Met Gly Ala Ile Ser Gln Ala Asn Ile Asn Leu
                405                 410                 415

Ile Gly Ser His Cys Gly Val Ser Thr Gly Glu Asp Gly Val Ser Gln
            420                 425                 430

Met Ala Leu Glu Asp Leu Ala Met Phe Arg Ser Ile Pro Asn Cys Thr
        435                 440                 445

Val Phe Tyr Pro Ser Asp Ala Ile Ser Thr Glu His Ala Ile Tyr Leu
    450                 455                 460

Ala Ala Asn Thr Lys Gly Met Cys Phe Ile Arg Thr Ser Gln Pro Glu
465                 470                 475                 480

Thr Ala Val Ile Tyr Thr Pro Gln Glu Asn Phe Glu Ile Gly Gln Ala
                485                 490                 495

Lys Val Val Arg His Gly Val Asn Asp Lys Val Thr Val Ile Gly Ala
            500                 505                 510

Gly Val Thr Leu His Glu Ala Leu Glu Ala Ala Asp His Leu Ser Gln
        515                 520                 525

Gln Gly Ile Ser Val Arg Val Ile Asp Pro Phe Thr Ile Lys Pro Leu
    530                 535                 540

Asp Ala Ala Thr Ile Ile Ser Ser Ala Lys Ala Thr Gly Gly Arg Val
545                 550                 555                 560

Ile Thr Val Glu Asp His Tyr Arg Glu Gly Gly Ile Gly Glu Ala Val
                565                 570                 575

Cys Ala Ala Val Ser Arg Glu Pro Asp Ile Leu Val His Gln Leu Ala
            580                 585                 590

Val Ser Gly Val Pro Gln Arg Gly Lys Thr Ser Glu Leu Leu Asp Met
        595                 600                 605

Phe Gly Ile Ser Thr Arg His Ile Ile Ala Ala Val Thr Leu Thr Leu
    610                 615                 620

Met Lys
625

<210>  38
<211>  522
<212>  PRT
<213>  Homo Sapiens

<400>  38

<210>  39
<211>  294
<212>  PRT
<213>  Homo Sapiens

<400>  39

Met Asn Phe Ile Leu Phe Ile Phe Ile Pro Gly Val Phe Ser Leu Lys
1               5                   10                  15

Ser Ser Thr Leu Lys Pro Thr Ile Glu Ala Leu Pro Asn Val Leu Pro
            20                  25                  30

Leu Asn Glu Asp Val Asn Lys Gln Glu Glu Lys Asn Glu Asp His Thr
        35                  40                  45

Pro Asn Tyr Ala Pro Ala Asn Glu Lys Asn Gly Asn Tyr Tyr Lys Asp
    50                  55                  60

Ile Lys Gln Tyr Val Phe Thr Thr Gln Asn Pro Asn Gly Thr Glu Ser
65                  70                  75                  80

Glu Ile Ser Val Arg Ala Thr Thr Asp Leu Asn Phe Ala Leu Lys Asn
                85                  90                  95

Asp Lys Thr Val Asn Ala Thr Thr Tyr Glu Lys Ser Thr Ile Glu Glu
            100                 105                 110

Glu Thr Thr Thr Ser Glu Pro Ser His Lys Asn Ile Gln Arg Ser Thr
        115                 120                 125

Pro Asn Val Pro Ala Phe Trp Thr Met Leu Ala Lys Ala Ile Asn Gly
    130                 135                 140

Thr Ala Val Val Met Asp Asp Lys Asp Gln Leu Phe His Pro Ile Pro
145                 150                 155                 160

Glu Ser Asp Val Asn Ala Thr Gln Gly Glu Asn Gln Pro Asp Leu Glu
                165                 170                 175

Asp Leu Lys Ile Lys Ile Met Leu Gly Ile Ser Leu Met Thr Leu Leu

180                 185                 190

Leu Phe Val Val Leu Leu Ala Phe Cys Ser Ala Thr Leu Tyr Lys Leu
        195                 200                 205

Arg His Leu Ser Tyr Lys Ser Cys Glu Ser Gln Tyr Ser Val Asn Pro
    210                 215                 220

Glu Leu Ala Thr Met Ser Tyr Phe His Pro Ser Glu Gly Val Ser Asp
225                 230                 235                 240

Thr Ser Phe Ser Lys Ser Ala Glu Ser Ser Thr Phe Leu Gly Thr Thr
                245                 250                 255

Ser Ser Asp Met Arg Arg Ser Gly Thr Arg Thr Ser Glu Ser Lys Ile
            260                 265                 270

Met Thr Asp Ile Ile Ser Ile Gly Ser Asp Asn Glu Met His Glu Asn
        275                 280                 285

Asp Glu Ser Val Thr Arg
    290

<210>  40
<211>  861
<212>  PRT
<213>  Homo Sapiens

<400>  40

Met Thr Gly Arg Ala Arg Ala Arg Ala Arg Gly Arg Ala Arg Gly Gln
1               5                   10                  15

Glu Thr Ala Gln Leu Val Gly Ser Thr Ala Ser Gln Gln Pro Gly Tyr
            20                  25                  30

Ile Gln Pro Arg Pro Gln Pro Pro Pro Ala Glu Gly Glu Leu Phe Gly
        35                  40                  45

Arg Gly Arg Gln Arg Gly Thr Ala Gly Gly Thr Ala Lys Ser Gln Gly
    50                  55                  60

Leu Gln Ile Ser Ala Gly Phe Gln Glu Leu Ser Leu Ala Glu Arg Gly
65                  70                  75                  80

Gly Arg Arg Arg Asp Phe His Asp Leu Gly Val Asn Thr Arg Gln Asn
                85                  90                  95

Leu Asp His Val Lys Glu Ser Lys Thr Gly Ser Ser Gly Ile Ile Val
            100                 105                 110

Arg Leu Ser Thr Asn His Phe Arg Leu Thr Ser Arg Pro Gln Trp Ala
        115                 120                 125

Leu Tyr Gln Tyr His Ile Asp Tyr Asn Pro Leu Met Glu Ala Arg Arg
    130                 135                 140

Leu Arg Ser Ala Leu Leu Phe Gln His Glu Asp Leu Ile Gly Lys Cys
145                 150                 155                 160

His Ala Phe Asp Gly Thr Ile Leu Phe Leu Pro Lys Arg Leu Gln Gln
                165                 170                 175

Lys Val Thr Glu Val Phe Ser Lys Thr Arg Asn Gly Glu Asp Val Arg
            180                 185                 190

Ile Thr Ile Thr Leu Thr Asn Glu Leu Pro Pro Thr Ser Pro Thr Cys
        195                 200                 205

Leu Gln Phe Tyr Asn Ile Ile Phe Arg Arg Leu Leu Lys Ile Met Asn
    210                 215                 220

Leu Gln Gln Ile Gly Arg Asn Tyr Tyr Asn Pro Asn Asp Pro Ile Asp
225                 230                 235                 240

Ile Pro Ser His Arg Leu Val Ile Trp Pro Gly Phe Thr Thr Ser Ile
                245                 250                 255

Leu Gln Tyr Glu Asn Ser Ile Met Leu Cys Thr Asp Val Ser His Lys
            260                 265                 270

Val Leu Arg Ser Glu Thr Val Leu Asp Phe Met Phe Asn Phe Tyr His
        275                 280                 285

Gln Thr Glu Glu His Lys Phe Gln Glu Gln Val Ser Lys Glu Leu Ile
    290                 295                 300

Gly Leu Val Val Leu Thr Lys Tyr Asn Asn Lys Thr Tyr Arg Val Asp
305                 310                 315                 320

Asp Ile Asp Trp Asp Gln Asn Pro Lys Ser Thr Phe Lys Lys Ala Asp
                325                 330                 335

Gly Ser Glu Val Ser Phe Leu Glu Tyr Tyr Arg Lys Gln Tyr Asn Gln
            340                 345                 350

Glu Ile Thr Asp Leu Lys Gln Pro Val Leu Val Ser Gln Pro Lys Arg
        355                 360                 365

Arg Arg Gly Pro Gly Gly Thr Leu Pro Gly Pro Ala Met Leu Ile Pro
    370                 375                 380

Glu Leu Cys Tyr Leu Thr Gly Leu Thr Asp Lys Met Arg Asn Asp Phe
385                 390                 395                 400

Asn Val Met Lys Asp Leu Ala Val His Thr Arg Leu Thr Pro Glu Gln
                405                 410                 415

Arg Gln Arg Glu Val Gly Arg Leu Ile Asp Tyr Ile His Lys Asn Asp
            420                 425                 430

Asn Val Gln Arg Glu Leu Arg Asp Trp Gly Leu Ser Phe Asp Ser Asn
        435                 440                 445

Leu Leu Ser Phe Ser Gly Arg Ile Leu Gln Thr Glu Lys Ile His Gln
    450                 455                 460

Gly Gly Lys Thr Phe Asp Tyr Asn Pro Gln Phe Ala Asp Trp Ser Lys
465                 470                 475                 480

Glu Thr Arg Gly Ala Pro Leu Ile Ser Val Lys Pro Leu Asp Asn Trp
                485                 490                 495

Leu Leu Ile Tyr Thr Arg Arg Asn Tyr Glu Ala Ala Asn Ser Leu Ile
            500                 505                 510

Gln Asn Leu Phe Lys Val Thr Pro Ala Met Gly Met Gln Met Arg Lys
        515                 520                 525

Ala Ile Met Ile Glu Val Asp Asp Arg Thr Glu Ala Tyr Leu Arg Val
    530                 535                 540

Leu Gln Gln Lys Val Thr Ala Asp Thr Gln Ile Val Val Cys Leu Leu
545                 550                 555                 560

Ser Ser Asn Arg Lys Asp Lys Tyr Asp Ala Ile Lys Lys Tyr Leu Cys
                565                 570                 575

Thr Asp Cys Pro Thr Pro Ser Gln Cys Val Val Ala Arg Thr Leu Gly

            580                 585                 590

Lys Gln Gln Thr Val Met Ala Ile Ala Thr Lys Ile Ala Leu Gln Met
        595                 600                 605

Asn Cys Lys Met Gly Gly Glu Leu Trp Arg Val Asp Ile Pro Leu Lys
    610                 615                 620

Leu Val Met Ile Val Gly Ile Asp Cys Tyr His Asp Met Thr Ala Gly
625                 630                 635                 640

Arg Arg Ser Ile Ala Gly Phe Val Ala Ser Ile Asn Glu Gly Met Thr
                645                 650                 655

Arg Trp Phe Ser Arg Cys Ile Phe Gln Asp Arg Gly Gln Glu Leu Val
            660                 665                 670

Asp Gly Leu Lys Val Cys Leu Gln Ala Ala Leu Arg Ala Trp Asn Ser
        675                 680                 685

Cys Asn Glu Tyr Met Pro Ser Arg Ile Ile Val Tyr Arg Asp Gly Val
    690                 695                 700

Gly Asp Gly Gln Leu Lys Thr Leu Val Asn Tyr Glu Val Pro Gln Phe
705                 710                 715                 720

Leu Asp Cys Leu Lys Ser Ile Gly Arg Gly Tyr Asn Pro Arg Leu Thr
                725                 730                 735

Val Ile Val Val Lys Lys Arg Val Asn Thr Arg Phe Phe Ala Gln Ser
            740                 745                 750

Gly Gly Arg Leu Gln Asn Pro Leu Pro Gly Thr Val Ile Asp Val Glu
        755                 760                 765

Val Thr Arg Pro Glu Trp Tyr Asp Phe Phe Ile Val Ser Gln Ala Val
    770                 775                 780

Arg Ser Gly Ser Val Ser Pro Thr His Tyr Asn Val Ile Tyr Asp Asn
785                 790                 795                 800

Ser Gly Leu Lys Pro Asp His Ile Gln Arg Leu Thr Tyr Lys Leu Cys
                805                 810                 815

His Ile Tyr Tyr Asn Trp Pro Gly Val Ile Arg Val Pro Ala Pro Cys
            820                 825                 830

Gln Tyr Ala His Lys Leu Ala Phe Leu Val Gly Gln Ser Ile His Arg
        835                 840                 845

Glu Pro Asn Leu Ser Leu Ser Asn Arg Leu Tyr Tyr Leu
    850                 855                 860

<210>  41
<211>  221
<212>  PRT
<213>  Homo Sapiens

<400>  41

Met Pro Leu Ala Leu Thr Leu Leu Leu Leu Ser Gly Leu Gly Ala Pro
1               5                   10                  15

Gly Gly Trp Gly Cys Leu Gln Cys Asp Pro Leu Val Leu Glu Ala Leu
            20                  25                  30

Gly His Leu Arg Ser Ala Leu Ile Pro Ser Arg Phe Gln Leu Glu Gln
        35                  40                  45

Leu Gln Ala Arg Ala Gly Ala Val Leu Met Gly Met Glu Gly Pro Phe
    50                  55                  60

Phe Arg Asp Tyr Ala Leu Asn Val Phe Val Gly Lys Val Glu Thr Asn
65                  70                  75                  80

Gln Leu Asp Leu Val Ala Ser Phe Val Lys Asn Gln Thr Gln His Leu
                85                  90                  95

Met Gly Asn Ser Leu Lys Asp Glu Pro Leu Leu Glu Glu Leu Val Thr
            100                 105                 110

Leu Arg Ala Asn Val Ile Lys Glu Phe Lys Lys Val Leu Ile Ser Tyr
        115                 120                 125

Glu Leu Lys Ala Cys Asn Pro Lys Leu Cys Arg Leu Leu Lys Glu Glu
    130                 135                 140

Val Leu Asp Cys Leu His Cys Gln Arg Ile Thr Pro Lys Cys Ile His
145                 150                 155                 160

Lys Lys Tyr Cys Phe Val Asp Arg Gln Pro Arg Val Ala Leu Gln Tyr
                165                 170                 175

Gln Met Asp Ser Lys Tyr Pro Arg Asn Gln Ala Leu Leu Gly Ile Leu
            180                 185                 190

Ile Ser Val Ser Leu Ala Val Phe Val Phe Val Val Ile Val Val Ser
        195                 200                 205

Ala Cys Thr Tyr Arg Gln Asn Arg Lys Leu Leu Leu Gln
    210                 215                 220

<210>  42
<211>  1623
<212>  PRT
<213>  Homo Sapiens

<400>  42

Met Ala Ala Glu Ala Ser Lys Thr Gly Pro Ser Arg Ser Ser Tyr Gln
1               5                   10                  15

Arg Met Gly Arg Lys Ser Gln Pro Trp Gly Ala Ala Glu Ile Gln Cys
            20                  25                  30

Thr Arg Cys Gly Arg Arg Val Ser Arg Ser Ser Gly His His Cys Glu
        35                  40                  45

Leu Gln Cys Gly His Ala Phe Cys Glu Leu Cys Leu Leu Met Thr Glu
    50                  55                  60

Glu Cys Thr Thr Ile Ile Cys Pro Asp Cys Glu Val Ala Thr Ala Val
65                  70                  75                  80

Asn Thr Arg Gln Arg Tyr Tyr Pro Met Ala Gly Tyr Ile Lys Glu Asp
                85                  90                  95

Ser Ile Met Glu Lys Leu Gln Pro Lys Thr Ile Lys Asn Cys Ser Gln
            100                 105                 110

Asp Phe Lys Lys Thr Ala Asp Gln Leu Thr Thr Gly Leu Glu Arg Ser
        115                 120                 125

Ala Ser Thr Asp Lys Thr Leu Leu Asn Ser Ser Ala Val Met Leu Asp
    130                 135                 140

Thr Asn Thr Ala Glu Glu Ile Asp Glu Ala Leu Asn Thr Ala His His
145                 150                 155                 160

Ser Phe Glu Gln Leu Ser Ile Ala Gly Lys Ala Leu Glu His Met Gln
                165                 170                 175

Lys Gln Thr Ile Glu Glu Arg Glu Arg Val Ile Glu Val Val Glu Lys
            180                 185                 190

Gln Phe Asp Gln Leu Leu Ala Phe Phe Asp Ser Arg Lys Lys Asn Leu
        195                 200                 205

Cys Glu Glu Phe Ala Arg Thr Thr Asp Asp Tyr Leu Ser Asn Leu Ile
    210                 215                 220

Lys Ala Lys Ser Tyr Ile Glu Glu Lys Lys Asn Asn Leu Asn Ala Ala
225                 230                 235                 240

Met Asn Ile Ala Arg Ala Leu Gln Leu Ser Pro Ser Leu Arg Thr Tyr
                245                 250                 255

Cys Asp Leu Asn Gln Ile Ile Arg Thr Leu Gln Leu Thr Ser Asp Ser
            260                 265                 270

Glu Leu Ala Gln Val Ser Ser Pro Gln Leu Arg Asn Pro Pro Arg Leu
        275                 280                 285

Ser Val Asn Cys Ser Glu Ile Ile Cys Met Phe Asn Asn Met Gly Lys
    290                 295                 300

Ile Glu Phe Arg Asp Ser Thr Lys Cys Tyr Pro Gln Glu Asn Glu Ile
305                 310                 315                 320

Arg Gln Asn Val Gln Lys Lys Tyr Asn Asn Lys Lys Glu Leu Ser Cys
                325                 330                 335

Tyr Asp Thr Tyr Pro Pro Leu Glu Lys Lys Lys Val Asp Met Ser Val
            340                 345                 350

Leu Thr Ser Glu Ala Pro Pro Pro Pro Leu Gln Pro Glu Thr Asn Asp
        355                 360                 365

Val His Leu Glu Ala Lys Asn Phe Gln Pro Gln Lys Asp Val Ala Thr
    370                 375                 380

Ala Ser Pro Lys Thr Ile Ala Val Leu Pro Gln Met Gly Ser Ser Pro
385                 390                 395                 400

Asp Val Ile Ile Glu Glu Ile Ile Glu Asp Asn Val Glu Ser Ser Ala
                405                 410                 415

Glu Leu Val Phe Val Ser His Val Ile Asp Pro Cys His Phe Tyr Ile
            420                 425                 430

Arg Lys Tyr Ser Gln Ile Lys Asp Ala Lys Val Leu Glu Lys Lys Val
        435                 440                 445

Asn Glu Phe Cys Asn Arg Ser Ser His Leu Asp Pro Ser Asp Ile Leu
    450                 455                 460

Glu Leu Gly Ala Arg Ile Phe Val Ser Ser Ile Lys Asn Gly Met Trp
465                 470                 475                 480

Cys Arg Gly Thr Ile Thr Glu Leu Ile Pro Ile Glu Gly Arg Asn Thr
                485                 490                 495

Arg Lys Pro Cys Ser Pro Thr Arg Leu Phe Val His Glu Val Ala Leu
            500                 505                 510

Ile Gln Ile Phe Met Val Asp Phe Gly Asn Ser Glu Val Leu Ile Val
        515                 520                 525

Thr Gly Val Val Asp Thr His Val Arg Pro Glu His Ser Ala Lys Gln
    530                 535                 540

His Ile Ala Leu Asn Asp Leu Cys Leu Val Leu Arg Lys Ser Glu Pro
545                 550                 555                 560

Tyr Thr Glu Gly Leu Leu Lys Asp Ile Gln Pro Leu Ala Gln Pro Cys
                565                 570                 575

Ser Leu Lys Asp Ile Val Pro Gln Asn Ser Asn Glu Gly Trp Glu Glu
            580                 585                 590

Glu Ala Lys Val Glu Phe Leu Lys Met Val Asn Asn Lys Ala Val Ser
        595                 600                 605

Met Lys Val Phe Arg Glu Glu Asp Gly Val Leu Ile Val Asp Leu Gln
    610                 615                 620

Lys Pro Pro Pro Asn Lys Ile Ser Ser Asp Met Pro Val Ser Leu Arg
625                 630                 635                 640

Asp Ala Leu Val Phe Met Glu Leu Ala Lys Phe Lys Ser Gln Ser Leu
                645                 650                 655

Arg Ser His Phe Glu Lys Asn Thr Thr Leu His Tyr His Pro Pro Ile
            660                 665                 670

Leu Pro Lys Glu Met Thr Asp Val Ser Val Thr Val Cys His Ile Asn
        675                 680                 685

Ser Pro Gly Asp Phe Tyr Leu Gln Leu Ile Glu Gly Leu Asp Ile Leu
    690                 695                 700

Phe Leu Leu Lys Thr Ile Glu Glu Phe Tyr Lys Ser Glu Asp Gly Glu
705                 710                 715                 720

Asn Leu Glu Ile Leu Cys Pro Val Gln Asp Gln Ala Cys Val Ala Lys
                725                 730                 735

Phe Glu Asp Gly Ile Trp Tyr Arg Ala Lys Val Ile Gly Leu Pro Gly
            740                 745                 750

His Gln Glu Val Glu Val Lys Tyr Val Asp Phe Gly Asn Thr Ala Lys
        755                 760                 765

Ile Thr Ile Lys Asp Val Arg Lys Ile Lys Asp Glu Phe Leu Asn Ala
    770                 775                 780

Pro Glu Lys Ala Ile Lys Cys Lys Leu Ala Tyr Ile Glu Pro Tyr Lys
785                 790                 795                 800

Arg Thr Met Gln Trp Ser Lys Glu Ala Lys Glu Lys Phe Glu Glu Lys
                805                 810                 815

Ala Gln Asp Lys Phe Met Thr Cys Ser Val Ile Lys Ile Leu Glu Asp
            820                 825                 830

Asn Val Leu Leu Val Glu Leu Phe Asp Ser Leu Gly Ala Pro Glu Met
        835                 840                 845

Thr Thr Thr Ser Ile Asn Asp Gln Leu Val Lys Glu Gly Leu Ala Ser
    850                 855                 860

Tyr Glu Ile Gly Tyr Ile Leu Lys Asp Asn Ser Gln Lys His Ile Glu
865                 870                 875                 880

Val Trp Asp Pro Ser Pro Glu Glu Ile Ile Ser Asn Glu Val His Asn
                885                 890                 895

Leu Asn Pro Val Ser Ala Lys Ser Leu Pro Asn Glu Asn Phe Gln Ser

900                 905                 910

Leu Tyr Asn Lys Glu Leu Pro Val His Ile Cys Asn Val Ile Ser Pro
        915                 920                 925

Glu Lys Ile Tyr Val Gln Trp Leu Leu Thr Glu Asn Leu Leu Asn Ser
    930                 935                 940

Leu Glu Glu Lys Met Ile Ala Ala Tyr Glu Asn Ser Lys Trp Glu Pro
945                 950                 955                 960

Val Lys Trp Glu Asn Asp Met His Cys Ala Val Lys Ile Gln Asp Lys
                965                 970                 975

Asn Gln Trp Arg Arg Gly Gln Ile Ile Arg Met Val Thr Asp Thr Leu
            980                 985                 990

Val Glu Val Leu Leu Tyr Asp Val  Gly Val Glu Leu Val  Val Asn Val
        995                 1000                1005

Asp Cys  Leu Arg Lys Leu Glu  Glu Asn Leu Lys Thr  Met Gly Arg
    1010                1015                1020

Leu Ser  Leu Glu Cys Ser Leu  Val Asp Ile Arg Pro  Ala Gly Gly
    1025                1030                1035

Ser Asp  Lys Trp Thr Ala Thr  Ala Cys Asp Cys Leu  Ser Leu Tyr
    1040                1045                1050

Leu Thr  Gly Ala Val Ala Thr  Ile Ile Leu Gln Val  Asp Ser Glu
    1055                1060                1065

Glu Asn  Asn Thr Thr Trp Pro  Leu Pro Val Lys Ile  Phe Cys Arg
    1070                1075                1080

Asp Glu  Lys Gly Glu Arg Val  Asp Val Ser Lys Tyr  Leu Ile Lys
    1085                1090                1095

Lys Gly  Leu Ala Leu Arg Glu  Arg Arg Ile Asn Asn  Leu Asp Asn
    1100                1105                1110

Ser His  Ser Leu Ser Glu Lys  Ser Leu Glu Val Pro  Leu Glu Gln
    1115                1120                1125

Glu Asp  Ser Val Val Thr Asn  Cys Ile Lys Thr Asn  Phe Asp Pro
    1130                1135                1140

Asp Lys Lys Thr Ala Asp Ile Ile Ser Glu Gln Lys Val Ser Glu
    1145                1150                1155

Phe Gln Glu Lys Ile Leu Glu Pro Arg Thr Thr Arg Gly Tyr Lys
    1160                1165                1170

Pro Pro Ala Ile Pro Asn Met Asn Val Phe Glu Ala Thr Val Ser
    1175                1180                1185

Cys Val Gly Asp Asp Gly Thr Ile Phe Val Val Pro Lys Leu Ser
    1190                1195                1200

Glu Phe Glu Leu Ile Lys Met Thr Asn Glu Ile Gln Ser Asn Leu
    1205                1210                1215

Lys Cys Leu Gly Leu Leu Glu Pro Tyr Phe Trp Lys Lys Gly Glu
    1220                1225                1230

Ala Cys Ala Val Arg Gly Ser Asp Thr Leu Trp Tyr Arg Gly Lys
    1235                1240                1245

Val Met Glu Val Val Gly Gly Ala Val Arg Val Gln Tyr Leu Asp
    1250                1255                1260

His Gly Phe Thr Glu Lys Ile Pro Gln Cys His Leu Tyr Pro Ile
    1265                1270                1275

Leu Leu Tyr Pro Asp Ile Pro Gln Phe Cys Ile Pro Cys Gln Leu
    1280                1285                1290

His Asn Thr Thr Pro Val Gly Asn Val Trp Gln Pro Asp Ala Ile
    1295                1300                1305

Glu Val Leu Gln Gln Leu Leu Ser Lys Arg Gln Val Asp Ile His
    1310                1315                1320

Ile Met Glu Leu Pro Lys Asn Pro Trp Glu Lys Leu Ser Ile His
    1325                1330                1335

Leu Tyr Phe Asp Gly Met Ser Leu Ser Tyr Phe Met Ala Tyr Tyr
    1340                1345                1350

Lys Tyr Cys Thr Ser Glu His Thr Glu Glu Met Leu Lys Glu Lys
    1355                1360                1365

Pro Arg Ser Asp His Asp Lys Lys Tyr Glu Glu Glu Gln Trp Glu
    1370                1375                1380

Ile Arg Phe Glu Glu Leu Leu Ser Ala Glu Thr Asp Thr Pro Leu
    1385                1390                1395

Leu Pro Pro Tyr Leu Ser Ser Ser Leu Pro Ser Pro Gly Glu Leu
    1400                1405                1410

Tyr Ala Val Gln Val Lys His Val Val Ser Pro Asn Glu Val Tyr
    1415                1420                1425

Ile Cys Leu Asp Ser Ile Glu Thr Ser Asn Gln Ser Asn Gln His
    1430                1435                1440

Ser Asp Thr Asp Asp Ser Gly Val Ser Gly Glu Ser Glu Ser Glu
    1445                1450                1455

Ser Leu Asp Glu Ala Leu Gln Arg Val Asn Lys Lys Val Glu Ala
    1460                1465                1470

Leu Pro Pro Leu Thr Asp Phe Arg Thr Glu Met Pro Cys Leu Ala
    1475                1480                1485

Glu Tyr Asp Asp Gly Leu Trp Tyr Arg Ala Lys Ile Val Ala Ile
    1490                1495                1500

Lys Glu Phe Asn Pro Leu Ser Ile Leu Val Gln Phe Val Asp Tyr
    1505                1510                1515

Gly Ser Thr Ala Lys Leu Thr Leu Asn Arg Leu Cys Gln Ile Pro
    1520                1525                1530

Ser His Leu Met Arg Tyr Pro Ala Arg Ala Ile Lys Val Leu Leu
    1535                1540                1545

Ala Gly Phe Lys Pro Pro Leu Arg Asp Leu Gly Glu Thr Arg Ile
    1550                1555                1560

Pro Tyr Cys Pro Lys Trp Ser Met Glu Ala Leu Trp Ala Met Ile
    1565                1570                1575

Asp Cys Leu Gln Gly Lys Gln Leu Tyr Ala Val Ser Met Ala Pro
    1580                1585                1590

Ala Pro Glu Gln Ile Val Thr Leu Tyr Asp Asp Glu Gln His Pro
    1595                1600                1605

Val His Met Pro Leu Val Glu Met Gly Leu Ala Asp Lys Asp Glu
    1610                1615                1620

<210> 43
<211> 443
<212> PRT
<213> Homo Sapiens

<400> 43

Met Arg Asn Ala Ile Ile Gln Gly Leu Phe Tyr Gly Ser Leu Thr Phe
1               5                   10                  15

Gly Ile Trp Thr Ala Leu Leu Phe Ile Tyr Leu His His Asn His Val
            20                  25                  30

Ser Ser Trp Gln Lys Lys Ser Gln Glu Pro Leu Ser Ala Trp Ser Pro
        35                  40                  45

Gly Lys Lys Val His Gln Gln Ile Ile Tyr Gly Ser Glu Gln Ile Pro
    50                  55                  60

Lys Pro His Val Ile Val Lys Arg Thr Asp Glu Asp Lys Ala Lys Ser
65                  70                  75                  80

Met Leu Gly Thr Asp Phe Asn His Thr Asn Pro Glu Leu His Lys Glu
                85                  90                  95

Leu Leu Lys Tyr Gly Phe Asn Val Ile Ile Ser Arg Ser Leu Gly Ile
            100                 105                 110

Glu Arg Glu Val Pro Asp Thr Arg Ser Lys Met Cys Leu Gln Lys His
        115                 120                 125

Tyr Pro Ala Arg Leu Pro Thr Ala Ser Ile Val Ile Cys Phe Tyr Asn
    130                 135                 140

Glu Glu Cys Asn Ala Leu Phe Gln Thr Met Ser Ser Val Thr Asn Leu
145                 150                 155                 160

Thr Pro His Tyr Phe Leu Glu Glu Ile Ile Leu Val Asp Asp Met Ser
                165                 170                 175

Lys Val Asp Asp Leu Lys Glu Lys Leu Asp Tyr His Leu Glu Thr Phe
            180                 185                 190

Arg Gly Lys Val Lys Ile Ile Arg Asn Lys Lys Arg Glu Gly Leu Ile
        195                 200                 205

Arg Ala Arg Leu Ile Gly Ala Ser His Ala Ser Gly Asp Val Leu Val
    210                 215                 220

Phe Leu Asp Ser His Cys Glu Val Asn Arg Val Trp Leu Glu Pro Leu
225                 230                 235                 240

Leu His Ala Ile Ala Lys Asp Pro Lys Met Val Val Cys Pro Leu Ile
                245                 250                 255

Asp Val Ile Asp Asp Arg Thr Leu Glu Tyr Lys Pro Ser Pro Leu Val
            260                 265                 270

Arg Gly Thr Phe Asp Trp Asn Leu Gln Phe Lys Trp Asp Asn Val Phe
        275                 280                 285

Ser Tyr Glu Met Asp Gly Pro Glu Gly Ser Thr Lys Pro Ile Arg Ser
    290                 295                 300

Pro Ala Met Ser Gly Gly Ile Phe Ala Ile Arg Arg His Tyr Phe Asn
305                 310                 315                 320

Glu Ile Gly Gln Tyr Asp Lys Asp Met Asp Phe Trp Gly Arg Glu Asn
                325                 330                 335

Leu Glu Leu Ser Leu Arg Ile Trp Met Cys Gly Gly Gln Leu Phe Ile
            340                 345                 350

Ile Pro Cys Ser Arg Val Gly His Ile Ser Lys Lys Gln Thr Gly Lys
        355                 360                 365

Pro Ser Thr Ile Ile Ser Ala Met Thr His Asn Tyr Leu Arg Leu Val
    370                 375                 380

His Val Trp Leu Asp Glu Tyr Lys Glu Gln Phe Phe Leu Arg Lys Pro
385                 390                 395                 400

Gly Leu Lys Tyr Val Thr Tyr Gly Asn Ile Arg Glu Arg Val Glu Leu
                405                 410                 415

Arg Lys Arg Leu Gly Cys Lys Ser Phe Gln Trp Tyr Leu Asp Asn Val
            420                 425                 430

Phe Pro Glu Leu Glu Ala Ser Val Asn Ser Leu
        435                 440

<210>  44
<211>  138
<212>  PRT
<213>  Homo Sapiens

<400>  44

Met Asn Ser Leu Val Ser Trp Gln Leu Leu Leu Phe Leu Cys Ala Thr
1               5                   10                  15

His Phe Gly Glu Pro Leu Glu Lys Val Ala Ser Val Gly Asn Ser Arg
            20                  25                  30

Pro Thr Gly Gln Gln Leu Glu Ser Leu Gly Leu Leu Ala Pro Gly Glu
        35                  40                  45

Gln Ser Leu Pro Cys Thr Glu Arg Lys Pro Ala Ala Thr Ala Arg Leu
    50                  55                  60

Ser Arg Arg Gly Thr Ser Leu Ser Pro Pro Pro Glu Ser Ser Gly Ser
65                  70                  75                  80

Pro Gln Gln Pro Gly Leu Ser Ala Pro His Ser Arg Gln Ile Pro Ala
                85                  90                  95

Pro Gln Gly Ala Val Leu Val Gln Arg Glu Lys Asp Leu Pro Asn Tyr
            100                 105                 110

Asn Trp Asn Ser Phe Gly Leu Arg Phe Gly Lys Arg Glu Ala Ala Pro
        115                 120                 125

Gly Asn His Gly Arg Ser Ala Gly Arg Gly
    130                 135

<210>  45
<211>  259
<212>  PRT
<213>  Homo Sapiens

<400>  45

Met Ser Glu Val Pro Val Ala Arg Val Trp Leu Val Leu Leu Leu Leu
1               5                   10                  15

Thr Val Gln Val Gly Val Thr Ala Gly Ala Pro Trp Gln Cys Ala Pro
            20                  25                  30

Cys Ser Ala Glu Lys Leu Ala Leu Cys Pro Pro Val Ser Ala Ser Cys
        35                  40                  45

Ser Glu Val Thr Arg Ser Ala Gly Cys Gly Cys Cys Pro Met Cys Ala
    50                  55                  60

Leu Pro Leu Gly Ala Ala Cys Gly Val Ala Thr Ala Arg Cys Ala Arg
65                  70                  75                  80

Gly Leu Ser Cys Arg Ala Leu Pro Gly Glu Gln Gln Pro Leu His Ala
                85                  90                  95

Leu Thr Arg Gly Gln Gly Ala Cys Val Gln Glu Ser Asp Ala Ser Ala
            100                 105                 110

Pro His Ala Ala Glu Ala Gly Ser Pro Glu Ser Pro Glu Ser Thr Glu
        115                 120                 125

Ile Thr Glu Glu Glu Leu Leu Asp Asn Phe His Leu Met Ala Pro Ser
    130                 135                 140

Glu Glu Asp His Ser Ile Leu Trp Asp Ala Ile Ser Thr Tyr Asp Gly
145                 150                 155                 160

Ser Lys Ala Leu His Val Thr Asn Ile Lys Lys Trp Lys Glu Pro Cys
                165                 170                 175

Arg Ile Glu Leu Tyr Arg Val Val Glu Ser Leu Ala Lys Ala Gln Glu
            180                 185                 190

Thr Ser Gly Glu Glu Ile Ser Lys Phe Tyr Leu Pro Asn Cys Asn Lys
        195                 200                 205

Asn Gly Phe Tyr His Ser Arg Gln Cys Glu Thr Ser Met Asp Gly Glu
    210                 215                 220

Ala Gly Leu Cys Trp Cys Val Tyr Pro Trp Asn Gly Lys Arg Ile Pro
225                 230                 235                 240

Gly Ser Pro Glu Ile Arg Gly Asp Pro Asn Cys Gln Ile Tyr Phe Asn
                245                 250                 255

Val Gln Asn

<210>  46
<211>  407
<212>  PRT
<213>  Homo Sapiens

<400>  46
   Met Pro Arg Gly His Lys Ser Lys Leu Arg Thr Cys Glu Lys Arg Gln
   1               5                   10                  15
   Glu Thr Asn Gly Gln Pro Gln Gly Leu Thr Gly Pro Gln Ala Thr Ala
               20                  25                  30
   Glu Lys Gln Glu Glu Ser His Ser Ser Ser Ser Ser Ser Arg Ala Cys
           35                  40                  45
   Leu Gly Asp Cys Arg Arg Ser Ser Asp Ala Ser Ile Pro Gln Glu Ser
       50                  55                  60
   Gln Gly Val Ser Pro Thr Gly Ser Pro Asp Ala Val Val Ser Tyr Ser
   65                  70                  75                  80
   Lys Ser Asp Val Ala Ala Asn Gly Gln Asp Glu Lys Ser Pro Ser Thr
                   85                  90                  95
   Ser Arg Asp Ala Ser Val Pro Gln Glu Ser Gln Gly Ala Ser Pro Thr
               100                 105                 110
   Gly Ser Pro Asp Ala Gly Val Ser Gly Ser Lys Tyr Asp Val Ala Ala
           115                 120                 125
   Asn Gly Gln Asp Glu Lys Ser Pro Ser Thr Ser His Asp Val Ser Val
       130                 135                 140
   Pro Gln Glu Ser Gln Gly Ala Ser Pro Thr Gly Ser Pro Asp Ala Gly
   145                 150                 155                 160
   Val Ser Gly Ser Lys Tyr Asp Val Ala Ala Glu Gly Glu Asp Glu Glu
                   165                 170                 175
   Ser Val Ser Ala Ser Gln Lys Ala Ile Ile Phe Lys Arg Leu Ser Lys
               180                 185                 190
   Asp Ala Val Lys Lys Lys Ala Cys Thr Leu Ala Gln Phe Leu Gln Lys
           195                 200                 205
   Lys Phe Glu Lys Lys Glu Ser Ile Leu Lys Ala Asp Met Leu Lys Cys
   210                 215                 220
   Val Arg Arg Glu Tyr Lys Pro Tyr Phe Pro Gln Ile Leu Asn Arg Thr
   225                 230                 235                 240
   Ser Gln His Leu Val Val Ala Phe Gly Val Glu Leu Lys Glu Met Asp
                   245                 250                 255
   Ser Ser Gly Glu Ser Tyr Thr Leu Val Ser Lys Leu Gly Leu Pro Ser
               260                 265                 270
   Glu Gly Ile Leu Ser Gly Asp Asn Ala Leu Pro Lys Ser Gly Leu Leu
           275                 280                 285
   Met Ser Leu Leu Val Val Ile Phe Met Asn Gly Asn Cys Ala Thr Glu
       290                 295                 300
   Glu Glu Val Trp Glu Phe Leu Gly Leu Leu Gly Ile Tyr Asp Gly Ile
   305                 310                 315                 320
   Leu His Ser Ile Tyr Gly Asp Ala Arg Lys Ile Ile Thr Glu Asp Leu
                   325                 330                 335
   Val Gln Asp Lys Tyr Val Val Tyr Arg Gln Val Cys Asn Ser Asp Pro
               340                 345                 350
   Pro Cys Tyr Glu Phe Leu Trp Gly Pro Arg Ala Tyr Ala Glu Thr Thr
           355                 360                 365
   Lys Met Arg Val Leu Arg Val Leu Ala Asp Ser Ser Asn Thr Ser Pro
       370                 375                 380
   Gly Leu Tyr Pro His Leu Tyr Glu Asp Ala Leu Ile Asp Glu Val Glu
   385                 390                 395                 400
   Arg Ala Leu Arg Leu Arg Ala
                   405

<210>  47
<211>  568
<212>  DNA
<213>  Artificial Sequence

<220>
<223>  probe derived from KRTAP21-1

<220>

<221>  misc_feature
<222>  (537)..(538)
<223>  n is a, c, g, or t

<220>
<221>  misc_feature
<222>  (541)..(543)
<223>  n is a, c, g, or t

<400>  47
ctaacttagt gctttgagtt taaattcttt tttcagccac tgtggtatgt tgattactat      60

attaataatg tgtctccttt atgtgttttt aaatagttac ttggtcatga actatttata     120

agctttggaa acatgatttc atttattctg caggataact tcaggaagtg ggtattaatt     180

ctcactgtat aagtgaagac attaagcacc agagtttaag tagcttgatc cactgaaaga     240

gattcaactg tgtggaacct atgccgcgga gaattgcttt ctgccattgt tattgtccag     300

cagttgccga atagctctga cctgagcaaa gcagtgaaac gcggtgctgt tgagtgggtt     360

gccactgcac ctttttcaac tactctagtc ttgtggagaa aacacccctt ctaaattttt     420

tcttcattga atactctccc tcagctccag ggtaccatat agagttttct tgtattttag     480

agttactgtt ttatcaaagt gtaccagtct tttgtattac atttctcctg tctaaannaa     540

nnntgtggtt tctactcatt atggatat                                        568


<210>  48
<211>  417
<212>  DNA
<213>  Artificial Sequence

<220>
<223>  probe derived from C12orf37

<400>  48
ttcctcctga ccacaaaaag cacttatact ctaggatgac tgattccagc ccagtggcct      60

ggcaagggtg aattacacct tgcatatcac actcttgaca tttgtgtgcg ctagcataag     120

aattataatt gaaacaggga tttaagtatc tcctctctag gtgcctaccc tccttggact     180

caggtcaaat ttattaaagg aagttttgtt tctagatagg ttgtttgaaa taaaataaca     240

gaatgttcaa gtaacacagt gtacctacag cttttaacaa aattgaggac ttgggtctcg     300

aaacaatttc ctttgatttt caggtatttt atctataaaa agggagataa agcattagtt     360

cataggacag ttatatgttt aaatgtgata atgtatatta accaccttgc atgtatt        417


<210>  49
<211>  287
<212>  DNA
<213>  Artificial Sequence

<220>

<223>  probe derived from WBSCR28


<220>
<221>  misc_feature
<222>  (118)..(118)
<223>  n is a, c, g, or t

<220>
<221>  misc_feature
<222>  (177)..(178)
<223>  n is a, c, g, or t

<220>
<221>  misc_feature
<222>  (240)..(240)
<223>  n is a, c, g, or t

<400>  49
taaggccttg caagtgaact gcgtggtaag gaagctcctg gtacagctga gacgtctgta       60

ttggtgggtg gagactatga ctgccctcac ctcctggcac ctggcctatc tcatcacntg      120

gaccacctgc ctggcctccc acctgctgca ggctgccttt gagcacacga cccagcnngc      180

cgaggcccag gaggttgaac cccaggaggt ctcagggtct tccttgctgc cctcactgtn      240

tgcgtcctcg gactcagagt ctggaacagt tttgccagag caagaaa                    287


<210>  50
<211>  355
<212>  DNA
<213>  Artificial Sequence

<220>
<223>  probe derived from RBM46


<220>
<221>  misc_feature
<222>  (41)..(41)
<223>  n is a, c, g, or t

<220>
<221>  misc_feature
<222>  (56)..(56)
<223>  n is a, c, g, or t

<400>  50
agcacttgaa gacaatggtt atagtagatt tgattaccaa ngatcactat ctgtanctgg       60

agattagaac aattatatga ccagaagcat ctaaccatta tgtaaaaaga aatgatgaga      120

caaaaagatt aagatacaaa ttttgtgcag tactaaagaa aaagcagtct accattgtgg      180

tccttgaaaa taactataga tatttttgtt atttgttaga cacaaattat aattttgttg      240

ttaatgtatt taagcatttt atagttatgc tttgtgtttt tgatattctt tgtattgtta      300

ataacaagtg ttatgggttt ttaatgttga aatcatgtgt taatttttgt acttg           355

<210>  51
<211>  381
<212>  DNA
<213>  Artificial Sequence

<220>
<223>  probe derived from DKFZp761D1

<400>  51
gcagtacatt tcacaatact cagataatcc tctcattgga agcaactcaa atgtccacca      60

actgatggta ttacccatgc aatggaatat tattcagtca taaaaaggaa tgaagtattg     120

aggcatgtta caacatgaag gaactttgag gacagtttgt taatacgcta tatactgtgt     180

aattctattt atatgaaata tccagaatag gtaaatcatt agagactaga gacagcagat     240

tagtggatgc caggagtttg gggaaggaag gggtgggaag aatgggaagt gactgcttaa     300

tggatacagg gtttcttttt ggggtgatga aaaatttctg gaactagatt gtagtgatgg     360

ttgcacaaca ccgtaagtgt a                                               381


<210>  52
<211>  497
<212>  DNA
<213>  Artificial Sequence

<220>
<223>  probe derived from NBPF4


<220>
<221>  misc_feature
<222>  (284)..(284)
<223>  n is a, c, g, or t

<220>
<221>  misc_feature
<222>  (393)..(393)
<223>  n is a, c, g, or t

<220>
<221>  misc_feature
<222>  (438)..(438)
<223>  n is a, c, g, or t

<400>  52
gcagagaagg cccagtgtgt ccatccccag tgcggtgata ctaggatggt cacttggtta      60

aggaggggtc taggagctct gtcccttgta aagacatctt atttgtaagt aatttggaaa     120

gtggtttgaa atagtataaa tatcctgtat tctagtgatc ttcttcagaa cattttatca     180

ccaattaatc accccgtctg tgtcagttat tatatttaag tttgtacatt gaaaattgtc     240

tatctcaaaa tcttacctta tacttgcttt tgctggcatt cttngtaaaa aagatcattc     300

cctgcccaaa ttttaacttt catccaaaat taattttaat ttctttttgc tggcattctg 360

ttgtgaaaaa gaatattctc tgccccaatt atnactttca tccaaaatta attttagtcc 420

atcagttaaa attttaantt ttaaatctgt ttaattaaaa catttcttgc ctctcactct 480

ggactattgg attttttt 497

<210> 53
<211> 500
<212> DNA
<213> Artificial Sequence

<220>
<223> probe derived from ADAM12

<400> 53
gtcttctgga ctggttttca cattagaaga caattgacaa cagttacata attcactctg 60

agtgttttat gagaaagcct tcttttgggg tcaacagttt tcctatgctt tgaaacagaa 120

aaatatgtac caagaatctt ggtttgcctt ccagaaaaca aaactgcatt tcactttccc 180

ggtgttcccc actgtatcta ggcaacatag tattcatgac tatggataaa ctaaacacgt 240

gacacaaaca cacacaaaag ggaacccagc tctaatacat tccaactcgt atagcatgca 300

tctgtttatt ctatagttat taagttcttt aaaatgtaaa gccatgctgg aaaataatac 360

tgctgagata catacagaat tactgtaact gattacactt ggtaattgta ctaaagccaa 420

acatatatat actattaaaa aggtttacag aattttatgg tgcattacgt gggcattgtc 480

tttttagatg cccaaatcct 500

<210> 54
<211> 420
<212> DNA
<213> Artificial Sequence

<220>
<223> probe derived from CDNA clone IMAGE:5299000

<400> 54
gttgcaatat taccttgggc actgttttga ctgcattcca caagttttta tttgtcatgt 60

tttaatcacc attcagttca aaatactttc taatgctcct tgttatttct tctttgaccc 120

attggttata taaaagtacg ttttgcttta tttctaaata tgtgaagact ttctagatat 180

ctcagttatt tattactaat ttaattttat tgtagccaga gaaaatacac tgtatgtgtt 240

caatctttgg aaatttcttg agacttattt tttgacccag tatgtttgcc ctcatgaaca 300

ttccatattc actagaaaaa gaatgtgtgt tcaaataatg ttggatatag tgttatgcaa 360

atgccactaa atcaagataa atcttttgca ctcttgtttt ttgcctgctt gttctaacaa 420

<210>  55
<211>  544
<212>  DNA
<213>  Artificial Sequence

<220>
<223>  probe direved from SOX30

<400>  55
gaaagttttc agtcgtgatt tgaggagtta aaaccaaatg caatttatgt cttcataaaa        60

ttttgattag tgaaactaga gtctggatgt ttcattgtag gaatatttaa gttattaagt       120

agtttaattt taatggctga aatttgcatc aacatgtatt attattactt tatcctggaa       180

catgcaaaat actgaagcct cacagttgta tgtgagggga aaggggaaat aaatctagca       240

tagtgtgatt tttattttat ctcaggatac attttttaaa tgattttttg tttgcttttt       300

atgtaatact tatggatgtt gtcaattttt gatgtaacat tttgaaagta ttttgacaac       360

tcctagtgaa cttggacttg gttgctaaat ttaacttaca ctaataacca attataagtt       420

ccaaatgtgt tttaatggca cctgggtgat tcttcagcta aatttagtca tttctgtttc       480

taaatatttt tatcatttta aaatattttt tttccatttg gcatacatcg ttctttgttg       540

taat                                                                    544


<210>  56
<211>  493
<212>  DNA
<213>  Artificial Sequence

<220>
<223>  probe direved from CDNA clone IMAGE:5296886

<400>  56
tgactgtcat atgggaggca ctgcaaggtg aaggagacat gtcctcacat ttggtctttg        60

ggaagcgaag taggcagaaa cccaagtgca aagtggccat ttgttttcct aatgctagat       120

aagtacctga tgtgttggaa gggtggagta gaactagaaa ctcctggaag ttcttttaag       180

aacgaatgca ggacctcatc agcccgagtc tttaaataag tcacacattt ttggtcacca       240

cattctggaa gggggacaaa aagatctgtc acagacaaaa tttggagata tttaaggctt       300

tgttgacttt ggcaaatgct tgattatagt tctgtggtct gtttgttttc tagagtctcc       360

atgaattgca agggaaaaca agtggtacac tggtatttct atttgataac ctgttctctt       420

tggaattccg gttagaaatc aaaggcttac ttgagtttta atgctttcgg gacttcaaat       480

gcttttctat ctt                                                          493


<210>  57
<211>  375
<212>  DNA

<213>  Artificial Sequence

<220>
<223>  probe derived from ASZ1

<400>  57
gataacactg gaatgggctt ctccccagaa ttttacttca gtttgtgaag aattggttaa       60

taatgttgaa gatttgagtg aaaaggtctg caaactaaaa gacctaattc aaaagttgca      120

aaatgaacgg gaaaatgatc caactcatat acaattaagg gaagaagtat ctacatggaa      180

tagtagaatt ttgaagagga cagctattac catatgcgga ttcggttttc ttcttttcat      240

ttgcaagcta actttccaga ggaaataatc caaatatttg ttttaagtta tatatacaca      300

tcttattgaa ccatccaaaa atcattgctt ttaactgttt gcagcacaat ataactttac      360

attctttgcc aatat                                                       375


<210>  58
<211>  504
<212>  DNA
<213>  Artificial Sequence

<220>
<223>  probe derived from SLC2A14


<220>
<221>  misc_feature
<222>  (171)..(171)
<223>  n is a, c, g, or t

<220>
<221>  misc_feature
<222>  (184)..(184)
<223>  n is a, c, g, or t

<220>
<221>  misc_feature
<222>  (186)..(186)
<223>  n is a, c, g, or t

<220>
<221>  misc_feature
<222>  (192)..(192)
<223>  n is a, c, g, or t

<220>
<221>  misc_feature
<222>  (264)..(264)
<223>  n is a, c, g, or t

<220>
<221>  misc_feature
<222>  (324)..(324)
<223>  n is a, c, g, or t

<220>

<221>  misc_feature
<222>  (326)..(326)
<223>  n is a, c, g, or t

<220>
<221>  misc_feature
<222>  (330)..(330)
<223>  n is a, c, g, or t

<220>
<221>  misc_feature
<222>  (342)..(342)
<223>  n is a, c, g, or t

<220>
<221>  misc_feature
<222>  (344)..(344)
<223>  n is a, c, g, or t

<400>  58
agagaccgag tgaacctacc ttcatttcag gagggattgg ccgcttggca catgacaact      60

ttgccagctt ttcctccctt gggttctgat attgccgcac tagaggatat aggagaggaa     120

aagtaaggtg cagttgcccc aacctcagac ttaccaggaa gcagatacat ntgagtgtgg     180

aagncngagg gngtttatgt aagagcacct tcctcacttc catacagctc tacgcggcaa     240

attaacttga gttttattta tctnatcctc tggtttaatt acataaatat ttatttttta      300

agtgtaattt tgccaaataa taanancagn aaggaaattg anantagagg gaggtgttta     360

aagagaggtt atagagtaaa agatttgatg ctggagaggt taaggtgcaa taagaattca     420

gggagaaatg ttgttcatta ttggagggta aatgatgtgg tgcctgaggt ctgtacatta     480

cctcttaaca atttctgtcc ttca                                            504


<210>  59
<211>  313
<212>  DNA
<213>  Artificial Sequence

<220>
<223>  probe derived from COX8C


<220>
<221>  misc_feature
<222>  (47)..(47)
<223>  n is a, c, g, or t

<400>  59
   ccctgtctgc cgcggaaatg gctgttggac ttgtggtgtt ttttacnacc ttcttaacac      60
   cagctgcata tgtgctaggc aacctgaagc agttcagaag gaattagatg gaagatgatg     120
   ttgaacagct gttaacgtcc aaaaaacttt cagaaaaagc tgtgtttttg ttaacgagca     180
   aaattgccta gttgagttga tgcaaccatt gtggtattca ctttcctcat gtttatgatg     240
   aatattttgc acttttttag tactgtgcat tatatagatg tatagtcaaa aatgttctgc 300
   ttaagtgtta aat 313

<210> 60
<211> 353
<212> DNA
<213> Artificial Sequence

<220>
<223> probe derived from TKTL2

<400> 60
gactacaaat atctgactga gctggggatt aaagtagagg taactgtatc ttaaatgagt 60

atgatttcct tgtaagttaa aaaaattgaa atttaattgt agacttcaat agtccaagtt 120

ttgaaggatg tttgagcttt tgtataatgc catttatacc tgcagtttta cagataatgt 180

ttgactgcag ttgccttgga aattcctcca aagtttgcct tcatctctcc tctacagttt 240

ggaggtgatg gtgcagcagt ggaacatctc ttgatgcacc acactacttg tgttctgtga 300

agtgatgaaa gtataactgg ttctagtttg cacactacac acatagtttt gtg 353

<210> 61
<211> 364
<212> DNA
<213> Artificial Sequence

<220>
<223> probe derived from TPTE2P2

<220>
<221> misc_feature
<222> (37)..(37)
<223> n is a, c, g, or t

<220>
<221> misc_feature
<222> (90)..(90)
<223> n is a, c, g, or t

<220>
<221> misc_feature
<222> (92)..(92)
<223> n is a, c, g, or t

<220>
<221> misc_feature
<222> (102)..(102)
<223> n is a, c, g, or t

<220>
<221> misc_feature
<222> (107)..(107)
<223> n is a, c, g, or t

<220>
<221>  misc_feature
<222>  (120)..(120)
<223>  n is a, c, g, or t

<220>
<221>  misc_feature
<222>  (179)..(179)
<223>  n is a, c, g, or t

<220>
<221>  misc_feature
<222>  (214)..(214)
<223>  n is a, c, g, or t

<220>
<221>  misc_feature
<222>  (222)..(222)
<223>  n is a, c, g, or t

<220>
<221>  misc_feature
<222>  (277)..(277)
<223>  n is a, c, g, or t

<400>  61
ctgaggttct attcatggtg agcaagtctt ttttttngtt tgtttcttca agctctaaca       60

agggtgccta ctacatggct tttcagttan cnccaaaata anatgtnaca attttttttn      120

ctattcttag gctttatcta caaagaaatg aattggataa tcttcataaa caaaaaacnt      180

ggaaaattta tcaaccagaa tatgcagtag aganatattt tnatgagaaa tgacttaagt      240

tatgttgtaa ctggtagctg attaagtata gttcccngca ccccttctgg gaaagaatta      300

tgttctttct aaccctgcca catagttata tgttctaaat cttccttgct ggtacatcta      360

tatt                                                                   364


<210>  62
<211>  217
<212>  DNA
<213>  Artificial Sequence

<220>
<223>  probe derived from C9orf11


<220>
<221>  misc_feature
<222>  (55)..(55)
<223>  n is a, c, g, or t

<220>
<221>  misc_feature
<222>  (59)..(60)
<223>  n is a, c, g, or t

<220>
<221>  misc_feature
<222>  (62)..(64)
<223>  n is a, c, g, or t

<220>
<221>  misc_feature
<222>  (66)..(70)
<223>  n is a, c, g, or t

<220>
<221>  misc_feature
<222>  (72)..(72)
<223>  n is a, c, g, or t

<220>
<221>  misc_feature
<222>  (74)..(74)
<223>  n is a, c, g, or t

<220>
<221>  misc_feature
<222>  (77)..(79)
<223>  n is a, c, g, or t

<220>
<221>  misc_feature
<222>  (81)..(84)
<223>  n is a, c, g, or t

<220>
<221>  misc_feature
<222>  (88)..(88)
<223>  n is a, c, g, or t

<220>
<221>  misc_feature
<222>  (90)..(93)
<223>  n is a, c, g, or t

<220>
<221>  misc_feature
<222>  (95)..(95)
<223>  n is a, c, g, or t

<220>
<221>  misc_feature
<222>  (99)..(104)
<223>  n is a, c, g, or t

<220>
<221>  misc_feature
<222>  (106)..(110)
<223>  n is a, c, g, or t

<400>  62
gtatttaaat tactacactg tattcatacc tggagaatat cattatgtga cttanaaann       60

tnnntnnnnn tncnctnnnc nnnncatnan nnnanagcnn nnnnannnnn tttatactgc      120

taccttttaa agtcactaaa atcttttaaa tggtgaatgt ctaagagtca gtcttcaggt      180

atacctgaca aaggatgaga aatgagcttt tgtagaa 217

<210> 63
<211> 546
<212> DNA
<213> Artificial Sequence

<220>
<223> probe derived from PIWIL1

<220>
<221> misc_feature
<222> (148)..(148)
<223> n is a, c, g, or t

<400> 63
attcaccggc ttccttattt tatatgtaaa aattaagatt ttatatttta tcttcttgtt 60

tctcatagat attttgtgag catttttttg tttattttga agaaatgtgg ataagatact 120

tggtagtata aaacagactc tctgagangt atttgaaatg tgtttggaga tttacttaaa 180

cgtactttca ggagtgagca agtcctactt ataaacctat attaacttta tttttgagat 240

acctgttttg aatttaaagg agataagagg cgtaaagtag gatgctcact acaaccatag 300

gtggggtttc agctcatatc ttaaagataa aaggtactat tatataacct atacacaaga 360

tacaggagaa aatatgcttg atttttattt ggcagggggg ctaggttgta tgggagtaaa 420

aaaaacattg aaaattttta aattgtccaa agaaacattt taagactctt taacaaaaaa 480

ggccatgagt aaatctctat attaacatta ctatttattt tgttttggaa ctgggacatg 540

attcta 546

<210> 64
<211> 521
<212> DNA
<213> Artificial Sequence

<220>
<223> probe derived from C19orf41

<400> 64
ggccgtgcgt gatgggcatg gaggggcctt tcttccggga ctacgcgctg aacgtgtttg 60

tggggaaagt ggagacaaat caactggacc ttgtggcgtc ctttgtcaag aaccaaacgc 120

agcacttaat gggtaactct ctgaaagatg agcctctgct ggaagagctg gtgaccctca 180

gggcgaatgt gatcaaggaa ttcaagaaag ttttaatttc atatgaatta aaagcctgca 240

accccaaact ttgccgcttg ctaaaagaag aggtgttgga ctgtttacat tgccagagga 300

tcactcccaa gtgtatccac aaaaagtact gctttgtcga ccggcaaccc cgcgtggccc 360

tgcagtacca gatggacagc aaatacccga ggaaccaggc gctgttgggc atcctcattt 420

ctgtgtctct ggctgtcttt gtcttcgtgg tcatcgtggt ctcggcttgt acatacagac 480

aaaaccgaaa actcctgctg cagtaggacg gtggtttggg g 521

<210> 65
<211> 486
<212> DNA
<213> Artificial Sequence

<220>
<223> probe derived from RNF17

<400> 65
gaatttaatc ctttatctat cttagtacaa tttgttgatt atggatcaac tgcaaagctg 60

acattaaaca gactgtgcca aattccttct catcttatgc ggtatccagc tcgagccata 120

aaggttctct tggcagggtt taaacctccc ttaagggatc taggggagac aagaatacca 180

tattgtccca aatggagcat ggaggcactg tgggctatga tagactgtct tcaaggaaaa 240

caactctatg ctgtgtccat ggctccagca ccagaacaga tagtgacatt atatgacgat 300

gaacagcatc cagttcatat gccgttggta gaaatggggc ttgcagataa agatgaataa 360

gtgcctaagt gtatacagtg agagcatcta tagaagccta gaagaattct gttatgttta 420

gactatgtct tatctttaga ctatttcagg cttaattttc ctaacttgtt cagccctagt 480

gcttta 486

<210> 66
<211> 306
<212> DNA
<213> Artificial Sequence

<220>
<223> probe derived from GALNTL5

<220>
<221> misc_feature
<222> (44)..(44)
<223> n is a, c, g, or t

<400> 66
ggaactttca ctaaggatct ggatgtgtgg aggccaactc tttnataatc ccctgctctc 60

gagtaggaca tatcagtaag aaacaaactg gaaaaccttc tacaatcatc agtgctatga 120

cacataacta cctaagactg gtgcacgttt ggctggatga atataaggag cagttttttc 180

ttcgaaagcc tggtctgaaa tatgtcacct acggaaatat tcgcgagcgt gttgagttaa 240

ggaaacgact gggttgcaag tcatttcagt ggtatttgga taatgtcttc ccagagttgg 300

aggcat 306

<210> 67
<211> 511
<212> DNA
<213> Artificial Sequence

<220>
<223> probe derived from KISS1

<400> 67
aagcctcaag gcacttctag gacctggctc ttctcaccaa gatgaactca ctggtttctt 60

ggcagctact gcttttcctc tgtgccaccc actttgggga gccattagaa aaggtggcct 120

ctgtggggaa ttctagaccc acaggccagc agctagaatc cctgggcctc ctggcccccg 180

gggagcagag cctgccgtgc accgagagga agccagctgc tactgccagg ctgagccgtc 240

gggggacctc gctgtccccg ccccccgaga gctccgggag ccgccagcag ccgggcctgt 300

ccgcccccca cagccgccag atccccgcac cccagggcgc ggtgctggtg cagcgggaga 360

aggacctgcc gaactacaac tggaactcct tcggcctgcg cttcggcaag cgggaggcgg 420

caccagggaa ccacggcaga agcgctgggc ggggctgggg cgcaggtgcg gggcagtgaa 480

cttcagaccc caaaggagtc agagcatgcg g 511


<210> 68
<211> 526
<212> DNA
<213> Artificial Sequence

<220>
<223> probe derived from IGFBP1

<400> 68
aataatgttc tgtcacgtga aatatttaag tatatagtat atttatactc tagaacatgc 60

acatttatat atatatgtat atgtatatat atatagtaac tactttttat actccataca 120

taacttgata tagaaagctg tttatttatt cactgtaagt ttattttttc tacacagtaa 180

aaacttgtac tatgttaata acttgtccta tgtcaatttg tatatcatga aacacttctc 240

atcatattgt atgtaagtaa ttgcatttct gctcttccaa agctcctgcg tctgttttta 300

aagagcatgg aaaaatactg cctagaaaat gcaaaatgaa ataagagaga gtagtttttc 360

agctagtttg aaggaggacg gttaacttgt atattccacc attcacattt gatgtacatg 420

tgtagggaaa gttaaaagtg ttgattacat aatcaaagct acctgtggtg atgttgccac 480

ctgttaaaat gtacactgga tatgttgtta aacacgtgtc gataat 526


<210> 69
<211> 279
<212> DNA

<213>  Artificial Sequence

<220>
<223>  probe derived from MAGEB6


<220>
<221>  misc_feature
<222>  (138)..(138)
<223>  n is a, c, g, or t

<220>
<221>  misc_feature
<222>  (167)..(167)
<223>  n is a, c, g, or t

<400>  69
tcacattcat ggctgtttaa ccaatctgaa agttacggtt tgggaattaa taaaacaaag       60

tcatacaaca cattttcttt gtaattgaga actagataac atggtaacag agaattgatt      120

ttcatatgaa tcttaacncc acagtaaaat agttgacatc ataatangaa gagaaagaaa      180

aggaaaaaca gaaatgtaaa agttgtttaa ttcttggttt gcctaattcg ttttcctatt      240

tcttttcata caaataaagg atacctggat ttatttagg                             279

## Description

The present invention relates to a prognosis method of lung cancer aby using specific genes.

Lung cancer is a disease of uncontrolled cell growth in tissues of the lung. This growth may lead to metastasis, which is the invasion of adjacent tissues and infiltration beyond the lungs. The vast majority of primary lung cancers are carcinomas of the lung, derived from epithelial cells. Lung cancer, the most common cause of cancer-related death in men and women, is responsible for 1.3 million deaths worldwide annually, as of 2004. The most common symptoms are shortness of breath, coughing (including coughing up blood), and weight loss.

Due to the high prevalence of this type of tumors, there is a need to efficiently diagnose lung cancer. Moreover, it is important to propose a prognosis method that allows the pathologist to determine, when a patient is afflicted by lung tumors, the survival rate during a short and a long period, and consequently to propose an adapted therapy. Presently, several clinical and pathological parameters help defining the prognosis, including histological subtypes, TNM stages (tumour size, presence of tumour cells in lymph nodes, presence of distant metastasis).

Cancer Testis (CT) genes are genes that are expressed in testis cells, but not expressed in somatic non pathologic cells. In cancers, CT genes are deregulated and are expressed ectopically in somatic cells. They appear as good candidate for cancer diagnosis. Some works have intended to identify a "general" strategy for diagnosing lung cancer, by detecting cancer testis gene expression.

For instance, the international application WO 2009/121878 discloses the use of a minimal group of CT genes for identifying any somatic or ovarian cancer. However, even if specific genes, or combinations of genes, can be used for diagnosing cancer, there is no indication that these genes can be used to establish a reliable prognosis during a short or a long period.

Therefore, there is a need to provide prognosis marker that can give specific evolution of tumoral progression, and patient survival, for instance by using CT genes.

Upon pathological diagnosis, the pathologist is able to define the histological subtype of lung cancer. Of all lung tumours, Small cell lung cancers (SCLC) represent the least frequent tumours with the worse outcome. Among Non Small Cell Lung cancers (NSCLC), the most frequent histological subtypes are squamous cell carcinoma (SQC), adenocarcinoma (ADK), large cell neuro-endocrine tumours (LCNE) and basaloid tumours (BAS), the former two being of better prognosis than the latter two. However, although the histological subtype of lung cancer represents one of the parameters influencing prognosis after surgery in operable lung cancer, patients with the same histological type of tumours have variable outcome, some die rapidly with signs of tumour aggressivity (local relapse after surgery, metastasis...) whereas others survive longer and/or die of other causes. Hence tumour aggressivity is not specifically defined by its histological subtype, and there is a need for molecular markers defining particularly aggressive tumours within each given histological subtype, in order to adapt therapeutic approaches accordingly.

One aim of the invention is to a simple, rapid, easy-to-use and effective method for giving a prognosis of lung cancer.

Another aim of the invention is to provide a specfic prognosis method of lung tumor, that have been histologically classified.

The present invention relates to the use of at least one element chosen among:
- at least 4 genes chosen among a group of 23 genes comprising or consisting of the nucleic acid sequences SEQ ID NO 1 to 23,
- or fragments of said genes
- or complementary sequences of said genes
- or sequences having at least 80% homology with said genes or fragment thereof,
- or protein coded by said genes, said proteins comprising or consisting of the amino acid sequences SEQ ID NO 24 to 46
- or fragments of said proteins,
- or antibodies directed against said proteins,
   said at least 4 genes being such that
   a. at least one gene belongs to a first set A of 4 genes comprising or consisting of the nucleic acid sequences SEQ ID NO: 1 to 4,
   b. at least one gene belongs to a second set B of 3 genes comprising or consisting of the nucleic acid sequences SEQ ID NO: 5 to 7
   c. at least one gene belongs to a third set C of 6 genes comprising or consisting of the nucleic acid sequences SEQ ID NO: 8 to 13 , and
   d. at least one gene belongs to a fourth set D of 9 genes comprising or consisting of the nucleic acid sequences SEQ ID NO: 14-23,
   for the implementation of a prognosis method, preferably *in vitro,* of the survival rate of a patient afflicted by a lung cancer, said lung cancer being previously classified as a lung tumour belonging from the group consisting of adenocarcinma (ADK), Squamous cell carcinoma (SQC), Basaloid tumours (BAS) and Large Cell Neuroendocrine (LCNE), wherein
   - the nucleic acid sequences SEQ ID NO: 1 to 4 are expressed in BAS,
   - the nucleic acid sequences SEQ ID NO: 5 to 7 are expressed in ADK,
   - the nucleic acid sequences SEQ ID NO: 8 to 13 are expressed in SQC,
   - the nucleic acid sequences SEQ ID NO: 14 to 23 are expressed in LCNE,
   said prognosis being such that:
   ■ if none of the genes of said set A, B, C and D is expressed, the patient survival rate during a period of time from 30 to 120 months after the diagnosis of said lung cancer is from about 19% to about 88% , and
   ■ if at least one gene of one of said sets A, B, C and D is expressed, the patient survival rate during a period of time from 30 to 120 months after the diagnosis of said lung cancer is from about 0% to about 73%.

The present invention is based on the unexpected observation made by the Inventors that the expression of at least 4 genes of a group of 23 determined genes is sufficient to determine the survival rate of a patient afflicted by a lung cancer, said lung cancer being classified among the following histological tumors: (ADK adenocarcinoma), Squamous cell carcinoma (SQC), Basaloid tumours (BAS) and Large Cell Neuroendocrine (LCNE) In other words, and as explained and exemplified hereafter, according to the invention the determination of the gene expression status on a ON/OFF basis of at least 4 genes chosen among a set of 23 genes allow to estimate at 30 months, or 60 months or 120 months after the diagnosis of a lung cancer, the probability of survival of an individual afflicted by one of the category of lung cancer belonguing to the group of ADK, SQC, BAS and LCNE.

The prognosis method proposed by the Inventors can also be carried out by detecting the expression of proteins expressed by the above mentioned at least 4 genes chosen among proteins coded by said 23 genes, also on a absence/presence basis.

Another aspect of the invention is that the diagnosis can also be carried out by determining the presence, of at least 4 specific antibodies specifically recognising at least 4 proteins coded by said at least 4 genes mentioned above. The above antibodies are specific of one protein, each protein being coded by one gene of the set of 23 genes.

A key aspect of the invention is the concept of ON/OFF for gene expression and presence/absence for the encoded proteins and antibodies detecting these proteins. This specific approach has the advantage of simplifying the analyses and making them independent of complex statistical tests to measure variations in expression levels applied to the majority of the existing tests.

The ON/OFF status of gene expression is established by determination of a threshold of gene expression allowing them to decide on the ON/OFF status of a gene such that:
a. if a gene is expressed at a level lower than the threshold, the gene is considered as not expressed or weakly expressed (defined as OFF), and
b. if a gene is expressed at a level upper to the threshold, the gene is considered as being expressed (defined as ON).

The Inventors have identified 23 genes comprising or being constituted by the nucleic acid sequences SEQ ID NO 1 to 23, as being cancer testis genes (CT genes) that can be used to carry out the prognosis method according to the invention.

The above 23 genes have been identified as being liable to be "expressed" (form here by, expressed refers to the ON status and not expressed to the OFF status) in lung cancer cells, but not in healthy samples. In other words, the above 23 genes are such as
a. they are not expressed, or weakly expressed in lung cells, and
b. they maybe expressed in lung tumor cells.

By "not expressed" it is defined in the invention the fact that the transcription of a gene is either not carried out, or is not detectable by common techniques known in the art, such as Quantitative RT-PCR, Northern blot or when microarrays data are considered.

By "weakly expressed", it is defined in the invention that a gene is expressed at a low level, meaning that the values are within the range of those measured for healthy tissue samples by Q-RT-PCR and by Northern blots or below the threshold when microarray data are considered. These values are considered as false-positive expressions, due to probe cross hybridization for instance. All the expression falling in these categories are considered as "OFF"

The difference between the absence of expression, or weak expression, and the expression determines its ON/OFF status, which is a key step of the invention. Indeed, the Inventors have identified that the ON status of the above 23 genes is a key step to determine the prognosis of lung cancer.

On microarrays, the expression level of the above mentioned genes is determined by the fact that a threshold of expression has been identified by the Inventors allowing to determine expression (ON) and non-expression (OFF) of said genes. The threshold determination is detailed hereafter, in the Example section.

For the microarrays, the threshold enabling to determine the expression status of a gene (ON versus OFF) is calculated by using the signal mean value and distribution obtained from transcriptomic data (in the same technology) with the corresponding probes in a large number of somatic tissues (which do not express the genes).

A similar strategy enables determining a threshold for the presence/absence of the encoded proteins or antibodies. For each protein or antibody, the mean value and distribution of the signal intensities obtained in an appropriate number of control somatic tissues serves as a basis for calculating the threshold.

According to the invention, the prognosis is carried out as described hereafter:
By "expressed", the invention defined that the transcript of a gene is detectable by the above known techniques while it is not detectable in healthy tissues or determined as being above the threshold when microarrays data are considered.

The 23 genes according to the invention have been classified by the Inventors in four sets:
a. a first set of 4 genes,
b. a second set of 3 genes
c. a third set of 6 genes, and
d. a fourth set of 9 genes.

The first set, also called in the invention set A, of 4 genes consists of the genes comprising or constituted by the nucleic acid sequences SEQ ID NO: 1, SEQ ID NO: 2, SEQ ID NO: 3, SEQ ID NO: 4.

The second set, also called in the invention set B, of 3 genes consists of the genes comprising or constituted by the nucleic acid sequences SEQ ID NO: 5, SEQ ID NO: 6 and SEQ ID NO: 7.

The third set, also called in the invention set C, of 6 genes consists of the genes comprising or constituted by the nucleic acid sequences SEQ ID NO: 8, SEQ ID NO: 9, SEQ ID NO: 10, SEQ ID NO: 11, SEQ ID NO: 12 and SEQ ID NO: 13.

The fourth set also called in the invention set D, of 9 genes consists of the genes comprising or constituted by the nucleic acid sequences SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, and SEQ ID NO: 23.

Set A is specific of BAS tumors, set B is specific of ADK tumors, set C is specific of SQC tumors and set D is specific of LCNE tumors.

The inventors have also defined subsets of each of the above sets A and B as follows:
Set A is divided into 4 subsets A1, A4 and A5, said subset being such that :
   a. subset A1 consists of the genes comprising or being constituted by the nucleic acid sequences SEQ ID NO: 1 and SEQ ID NO: 2,
   b. subset A4 consists of the gene comprising or being constituted by the nucleic acid sequence SEQ ID NO: 3, and
   c. subset A5 consists of the genes comprising or being constituted by the nucleic acid sequence SEQ ID NO: 4,
Set A can also be divided into the following subsets:
   a. subset A1 consists of the genes comprising or being constituted by the nucleic acid sequences SEQ ID NO: 1 and SEQ ID NO: 2,
   b. subset A2 consists of the genes comprising or being constituted by the nucleic acid sequences SEQ ID NO: 1, SEQ ID NO: 2 and SEQ ID NO: 3, and
   c. subset A3 consists of the genes comprising or being constituted by the nucleic acid sequences SEQ ID NO: 1, SEQ ID NO: 2, SEQ ID NO: 3 and SEQ ID NO: 4.
Set B is divided into 2 subsets Bland B3, said subset being such that:
   a. subset B1 consists of the genes comprising or being constituted by the nucleic acid sequences SEQ ID NO: 5 and SEQ ID NO: 6, and
   b. subset B3 consists of the gene comprising or being constituted by the nucleic acid sequence SEQ ID NO: 7.
Set B can also be divided into the following subsets:
   c. subset B1 consists of the genes comprising or being constituted by the nucleic acid sequences SEQ ID NO: 5, and SEQ ID NO: 6,
   d. subset B2 consists of the genes comprising or being constituted by the nucleic acid sequences SEQ ID NO: 5, SEQ ID NO: 6 and SEQ ID NO: 7.
Set C is divided into 3 subsets C1, C4 and C5, said subset being such that :
   a. subset C1 consists of the genes comprising or being constituted by the nucleic acid sequences SEQ ID NO: 8 and SEQ ID NO: 9,
   b. subset C4 consists of the genes comprising or being constituted by the nucleic acid sequences SEQ ID NO: 10 and SEQ ID NO: 11, and
   c. subset C5 consists of the genes comprising or being constituted by the nucleic acid sequences SEQ ID NO: 12 and SEQ ID NO: 13.
Set C can also be divided into the following subsets:
   a. subset C1 consists of the genes comprising or being constituted by the nucleic acid sequences SEQ ID NO: 8, and SEQ ID NO: 9,
   b. subset C2 consists of the genes comprising or being constituted by the nucleic acid sequences SEQ ID NO: 8, SEQ ID NO: 9, SEQ ID NO: 10 and SEQ ID NO: 11, and
   c. subset C3 consists of the genes comprising or being constituted by the nucleic acid sequences SEQ ID NO: 8, SEQ ID NO: 9, SEQ ID NO: 10, SEQ ID NO: 11, SEQ ID NO: 12 and SEQ ID NO: 13.
Set D is divided into 3 subsets D1, D5, D6 and D7, said subset being such that :
   a. subset D1 consists of the genes comprising or being constituted by the nucleic acid sequences SEQ ID NO: 14 and SEQ ID NO: 15,
   b. subset D5 consists of the genes comprising or being constituted by the nucleic acid sequences SEQ ID NO: 16, SEQ ID NO: 17, SEQ ID NO: 18, SEQ ID NO: 19 and SEQ ID NO: 20,
   c. subset D6 consists of the gene comprising or being constituted by the nucleic acid sequence SEQ ID NO: 21 and
   d. subset D7 consists of the genes comprising or being constituted by the nucleic acid sequences SEQ ID NO: 22 and SEQ ID NO: 23.
Set D can also be divided into the following subsets:
   a. subset D1 consists of the genes comprising or being constituted by the nucleic acid sequences SEQ ID NO: 14 and SEQ ID NO: 15,
   b. subset D2 consists of the genes comprising or being constituted by the nucleic acid sequences SEQ ID NO: 14 and SEQ ID NO: 15, SEQ ID NO: 16, SEQ ID NO: 17, SEQ ID NO: 18, SEQ ID NO: 19 and SEQ ID NO: 20,
   c. subset D3 consists of the genes comprising or being constituted by the nucleic acid sequences SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21, and
   d. subset D3 consists of the genes comprising or being constituted by the nucleic acid sequences SEQ ID NO: 14, SEQ ID NO: 15, SEQ ID NO: 16, SEQ ID NO: 17, SEQ ID NO: 18, SEQ ID NO: 19, SEQ ID NO: 20 , SEQ ID NO: 21, SEQ ID NO: 22 and SEQ ID NO: 23.

Thus, the prognosis method according to the invention is such that, when determining the expression status of 4 genes on a ON/OFF basis, belonging to the set of 23 genes comprising or consisting of nucleic acid sequences SEQ ID NO : 1 to 23,
a. if none of said at least 4 genes are expressed , the patient survival rate at 30 months, 60 months or 120 months following the diagnosis is from about 19% to about 88% or more, and
b. if at least one gene of the sets A, B, C or D is expressed, according to the defined expression threshold, the patient survival rate at 30 months, 60 months or 120 months following the diagnosis is from about 0% to about 73%.

Therefore, the prognosis method according to the invention can be carried out as by measuring at least the expression of the following 720 combinations of genes:
SEQ ID NO: 1+5+8+14, SEQ ID NO: 1+5+9+14, SEQ ID NO: 1+5+10+14, SEQ ID NO: 1+5+11+14, SEQ ID NO: 1+5+12+14, SEQ ID NO: 1+5+12+14, SEQ ID NO: 1+5+8+15, SEQ ID NO: 1+5+9+15, SEQ ID NO: 1+5+10+15, SEQ ID NO: 1+5+11+15, SEQ ID NO: 1+5+12+15, SEQ ID NO: 1+5+12+15, SEQ ID NO: 1+5+8+16, SEQ ID NO: 1+5+9+16, SEQ ID NO: 1+5+10+16, SEQ ID NO: 1+5+11+16, SEQ ID NO: 1+5+12+16, SEQ ID NO: 1+5+12+16, SEQ ID NO: 1+5+8+17, SEQ ID NO: 1+5+9+17, SEQ ID NO: 1+5+10+17, SEQ ID NO: 1+5+11+17, SEQ ID NO: 1+5+12+17, SEQ ID NO: 1+5+12+17, SEQ ID NO: 1+5+8+18, SEQ ID NO: 1+5+9+18, SEQ ID NO: 1+5+10+18, SEQ ID NO: 1+5+11+18, SEQ ID NO: 1+5+12+18, SEQ ID NO: 1+5+12+18, SEQ ID NO: 1+5+8+19, SEQ ID NO: 1+5+9+19, SEQ ID NO: 1+5+10+19, SEQ ID NO: 1+5+11+19, SEQ ID NO: 1+5+12+19, SEQ ID NO: 1+5+12+19, SEQ ID NO: 1+5+8+20, SEQ ID NO: 1+5+9+20, SEQ ID NO: 1+5+10+20, SEQ ID NO: 1+5+11+20, SEQ ID NO: 1+5+12+20, SEQ ID NO: 1+5+12+20, SEQ ID NO: 1+5+8+21, SEQ ID NO: 1+5+9+21, SEQ ID NO: 1+5+10+21, SEQ ID NO: 1+5+11+21, SEQ ID NO: 1+5+12+21, SEQ ID NO: 1+5+12+21, SEQ ID NO: 1+5+8+22, SEQ ID NO: 1+5+9+22, SEQ ID NO: 1+5+10+22, SEQ ID NO: 1+5+11+22, SEQ ID NO: 1+5+12+22, SEQ ID NO: 1+5+12+22, SEQ ID NO: 1+5+8+23, SEQ ID NO: 1+5+9+23, SEQ ID NO: 1+5+10+23, SEQ ID NO: 1+5+11+23, SEQ ID NO: 1+5+12+23, SEQ ID NO: 1+5+12+23, SEQ ID NO: 1+6+8+14, SEQ ID NO: 1+6+9+14, SEQ ID NO: 1+6+10+14, SEQ ID NO: 1+6+11+14, SEQ ID NO: 1+6+12+14, SEQ ID NO: 1+6+12+14, SEQ ID NO: 1+6+8+15, SEQ ID NO: 1+6+9+15, SEQ ID NO: 1+6+10+15, SEQ ID NO: 1+6+11+15, SEQ ID NO: 1+6+12+15, SEQ ID NO: 1+6+12+15, SEQ ID NO: 1+6+8+16, SEQ ID NO: 1+6+9+16, SEQ ID NO: 1+6+10+16, SEQ ID NO: 1+6+11+16, SEQ ID NO: 1+6+12+16, SEQ ID NO: 1+6+12+16, SEQ ID NO: 1+6+8+17, SEQ ID NO: 1+6+9+17, SEQ ID NO: 1+6+10+17, SEQ ID NO: 1+6+11+17, SEQ ID NO: 1+6+12+17, SEQ ID NO: 1+6+12+17, SEQ ID NO: 1+6+8+18, SEQ ID NO: 1+6+9+18, SEQ ID NO: 1+6+10+18, SEQ ID NO: 1+6+11+18, SEQ ID NO: 1+6+12+18, SEQ ID NO: 1+6+12+18, SEQ ID NO: 1+6+8+19, SEQ ID NO: 1+6+9+19, SEQ ID NO: 1+6+10+19, SEQ ID NO: 1+6+11+19, SEQ ID NO: 1+6+12+19, SEQ ID NO: 1+6+12+19, SEQ ID NO: 1+6+8+20, SEQ ID NO: 1+6+9+20, SEQ ID NO: 1+6+10+20, SEQ ID NO: 1+6+11+20, SEQ ID NO: 1+6+12+20, SEQ ID NO: 1+6+12+20, SEQ ID NO: 1+6+8+21, SEQ ID NO: 1+6+9+21, SEQ ID NO: 1+6+10+21, SEQ ID NO: 1+6+11+21, SEQ ID NO: 1+6+12+21, SEQ ID NO: 1+6+12+21, SEQ ID NO: 1+6+8+22, SEQ ID NO: 1+6+9+22, SEQ ID NO: 1+6+10+22, SEQ ID NO: 1+6+11+22, SEQ ID NO: 1+6+12+22, SEQ ID NO: 1+6+12+22, SEQ ID NO: 1+6+8+23, SEQ ID NO: 1+6+9+23, SEQ ID NO: 1+6+10+23, SEQ ID NO: 1+6+11+23, SEQ ID NO: 1+6+12+23, SEQ ID NO: 1+6+12+23, SEQ ID NO: 1+7+8+14, SEQ ID NO: 1+7+9+14, SEQ ID NO: 1+7+10+14, SEQ ID NO: 1+7+11+14, SEQ ID NO: 1+7+12+14, SEQ ID NO: 1+7+12+14, SEQ ID NO: 1+7+8+15, SEQ ID NO: 1+7+9+15, SEQ ID NO: 1+7+10+15, SEQ ID NO: 1+7+11+15, SEQ ID NO: 1+7+12+15, SEQ ID NO: 1+7+12+15, SEQ ID NO: 1+7+8+16, SEQ ID NO: 1+7+9+16, SEQ ID NO: 1+7+10+16, SEQ ID NO: 1+7+11+16, SEQ ID NO: 1+7+12+16, SEQ ID NO: 1+7+12+16, SEQ ID NO: 1+7+8+17, SEQ ID NO: 1+7+9+17, SEQ ID NO: 1+7+10+17, SEQ ID NO: 1+7+11+17, SEQ ID NO: 1+7+12+17, SEQ ID NO: 1+7+12+17, SEQ ID NO: 1+7+8+18, SEQ ID NO: 1+7+9+18, SEQ ID NO: 1+7+10+18, SEQ ID NO: 1+7+11+18, SEQ ID NO: 1+7+12+18, SEQ ID NO: 1+7+12+18, SEQ ID NO: 1+7+8+19, SEQ ID NO: 1+7+9+19, SEQ ID NO: 1+7+10+19, SEQ ID NO: 1+7+11+19, SEQ ID NO: 1+7+12+19, SEQ ID NO: 1+7+12+19, SEQ ID NO: 1+7+8+20, SEQ ID NO: 1+7+9+20, SEQ ID NO: 1+7+10+20, SEQ ID NO: 1+7+11+20, SEQ ID NO: 1+7+12+20, SEQ ID NO: 1+7+12+20, SEQ ID NO: 1+7+8+21, SEQ ID NO: 1+7+9+21, SEQ ID NO: 1+7+10+21, SEQ ID NO: 1+7+11+21, SEQ ID NO: 1+7+12+21, SEQ ID NO: 1+7+12+21, SEQ ID NO: 1+7+8+22, SEQ ID NO: 1+7+9+22, SEQ ID NO: 1+7+10+22, SEQ ID NO: 1+7+11+22, SEQ ID NO: 1+7+12+22, SEQ ID NO: 1+7+12+22, SEQ ID NO: 1+7+8+23, SEQ ID NO: 1+7+9+23, SEQ ID NO: 1+7+10+23, SEQ ID NO: 1+7+11+23, SEQ ID NO: 1+7+12+23, SEQ ID NO: 1+7+12+23, SEQ ID NO: 2+5+8+14, SEQ ID NO: 2+5+9+14, SEQ ID NO: 1+5+10+14, SEQ ID NO: 2+5+11+14, SEQ ID NO: 2+5+12+14, SEQ ID NO: 2+5+12+14, SEQ ID NO: 2+5+8+15, SEQ ID NO: 2+5+9+15, SEQ ID NO: 1+5+10+15, SEQ ID NO: 2+5+11+15, SEQ ID NO: 2+5+12+15, SEQ ID NO: 2+5+12+15, SEQ ID NO: 2+5+8+16, SEQ ID NO: 2+5+9+16, SEQ ID NO: 1+5+10+16, SEQ ID NO: 2+5+11+16, SEQ ID NO: 2+5+12+16, SEQ ID NO: 2+5+12+16, SEQ ID NO: 2+5+8+17, SEQ ID NO: 2+5+9+17, SEQ ID NO: 1+5+10+17, SEQ ID NO: 2+5+11+17, SEQ ID NO: 2+5+12+17, SEQ ID NO: 2+5+12+17, SEQ ID NO: 2+5+8+18, SEQ ID NO: 2+5+9+18, SEQ ID NO: 1+5+10+18, SEQ ID NO: 2+5+11+18, SEQ ID NO: 2+5+12+18, SEQ ID NO: 2+5+12+18, SEQ ID NO: 2+5+8+19, SEQ ID NO: 2+5+9+19, SEQ ID NO: 1+5+10+19, SEQ ID NO: 2+5+11+19, SEQ ID NO: 2+5+12+19, SEQ ID NO: 2+5+12+19, SEQ ID NO: 2+5+8+20, SEQ ID NO: 2+5+9+20, SEQ ID NO: 1+5+10+20, SEQ ID NO: 2+5+11+20, SEQ ID NO: 2+5+12+20, SEQ ID NO: 2+5+12+20, SEQ ID NO: 2+5+8+21, SEQ ID NO: 2+5+9+21, SEQ ID NO: 1+5+10+21, SEQ ID NO: 2+5+11+21, SEQ ID NO: 2+5+12+21, SEQ ID NO: 2+5+12+21, SEQ ID NO: 2+5+8+22, SEQ ID NO: 2+5+9+22, SEQ ID NO: 1+5+10+22, SEQ ID NO: 2+5+11+22, SEQ ID NO: 2+5+12+22, SEQ ID NO: 2+5+12+22, SEQ ID NO: 2+5+8+23, SEQ ID NO: 2+5+9+23, SEQ ID NO: 1+5+10+23, SEQ ID NO: 2+5+11+23, SEQ ID NO: 2+5+12+23, SEQ ID NO: 2+5+12+23, SEQ ID NO: 2+6+8+14, SEQ ID NO: 2+6+9+14, SEQ ID NO: 2+6+10+14, SEQ ID NO: 2+6+11+14, SEQ ID NO: 2+6+12+14, SEQ ID NO: 2+6+12+14, SEQ ID NO: 2+6+8+15, SEQ ID NO: 2+6+9+15, SEQ ID NO: 2+6+10+15, SEQ ID NO: 2+6+11+15, SEQ ID NO: 2+6+12+15, SEQ ID NO: 2+6+12+15, SEQ ID NO: 2+6+8+16, SEQ ID NO: 2+6+9+16, SEQ ID NO: 2+6+10+16, SEQ ID NO: 2+6+11+16, SEQ ID NO: 2+6+12+16, SEQ ID NO: 2+6+12+16, SEQ ID NO: 2+6+8+17, SEQ ID NO: 2+6+9+17, SEQ ID NO: 2+6+10+17, SEQ ID NO: 2+6+11+17, SEQ ID NO: 2+6+12+17, SEQ ID NO: 2+6+12+17, SEQ ID NO: 2+6+8+18, SEQ ID NO: 2+6+9+18, SEQ ID NO: 2+6+10+18, SEQ ID NO: 2+6+11+18, SEQ ID NO: 2+6+12+18, SEQ ID NO: 2+6+12+18, SEQ ID NO: 2+6+8+19, SEQ ID NO: 2+6+9+19, SEQ ID NO: 2+6+10+19, SEQ ID NO: 2+6+11+19, SEQ ID NO: 2+6+12+19, SEQ ID NO: 2+6+12+19, SEQ ID NO: 2+6+8+20, SEQ ID NO: 2+6+9+20, SEQ ID NO: 2+6+10+20, SEQ ID NO: 2+6+11+20, SEQ ID NO: 2+6+12+20, SEQ ID NO: 2+6+12+20, SEQ ID NO: 2+6+8+21, SEQ ID NO: 2+6+9+21, SEQ ID NO: 2+6+10+21, SEQ ID NO: 2+6+11+21, SEQ ID NO: 2+6+12+21, SEQ ID NO: 2+6+12+21, SEQ ID NO: 2+6+8+22, SEQ ID NO: 2+6+9+22, SEQ ID NO: 2+6+10+22, SEQ ID NO: 2+6+11+22, SEQ ID NO: 2+6+12+22, SEQ ID NO: 2+6+12+22, SEQ ID NO: 2+6+8+23, SEQ ID NO: 2+6+9+23, SEQ ID NO: 2+6+10+23, SEQ ID NO: 2+6+11+23, SEQ ID NO: 2+6+12+23, SEQ ID NO: 2+6+12+23, SEQ ID NO: 2+7+8+14, SEQ ID NO: 2+7+9+14, SEQ ID NO: 2+7+10+14, SEQ ID NO: 2+7+11+14, SEQ ID NO: 2+7+12+14, SEQ ID NO: 2+7+12+14, SEQ ID NO: 2+7+8+15, SEQ ID NO: 2+7+9+15, SEQ ID NO: 2+7+10+15, SEQ ID NO: 2+7+11+15, SEQ ID NO: 2+7+12+15, SEQ ID NO: 2+7+12+15, SEQ ID NO: 2+7+8+16, SEQ ID NO: 2+7+9+16, SEQ ID NO: 2+7+10+16, SEQ ID NO: 2+7+11+16, SEQ ID NO: 2+7+12+16, SEQ ID NO: 2+7+12+16, SEQ ID NO: 2+7+8+17, SEQ ID NO: 2+7+9+17, SEQ ID NO: 2+7+10+17, SEQ ID NO: 2+7+11+17, SEQ ID NO: 2+7+12+17, SEQ ID NO: 2+7+12+17, SEQ ID NO: 2+7+8+18, SEQ ID NO: 2+7+9+18, SEQ ID NO: 2+7+10+18, SEQ ID NO: 2+7+11+18, SEQ ID NO: 2+7+12+18, SEQ ID NO: 2+7+12+18, SEQ ID NO: 2+7+8+19, SEQ ID NO: 2+7+9+19, SEQ ID NO: 2+7+10+19, SEQ ID NO: 2+7+11+19, SEQ ID NO: 2+7+12+19, SEQ ID NO: 2+7+12+19, SEQ ID NO: 2+7+8+20, SEQ ID NO: 2+7+9+20, SEQ ID NO: 2+7+10+20, SEQ ID NO: 2+7+11+20, SEQ ID NO: 2+7+12+20, SEQ ID NO: 2+7+12+20, SEQ ID NO: 2+7+8+21, SEQ ID NO: 2+7+9+21, SEQ ID NO: 2+7+10+21, SEQ ID NO: 2+7+11+21, SEQ ID NO: 2+7+12+21, SEQ ID NO: 2+7+12+21, SEQ ID NO: 2+7+8+22, SEQ ID NO: 2+7+9+22, SEQ ID NO: 2+7+10+22, SEQ ID NO: 2+7+11+22, SEQ ID NO: 2+7+12+22, SEQ ID NO: 2+7+12+22, SEQ ID NO: 2+7+8+23, SEQ ID NO: 2+7+9+23, SEQ ID NO: 2+7+10+23, SEQ ID NO: 2+7+11+23, SEQ ID NO: 2+7+12+23, SEQ ID NO: 2+7+12+23, SEQ ID NO: 3+5+8+14, SEQ ID NO: 3+5+9+14, SEQ ID NO: 3+5+10+14, SEQ ID NO: 3+5+11+14, SEQ ID NO: 3+5+12+14, SEQ ID NO: 3+5+12+14, SEQ ID NO: 3+5+8+15, SEQ ID NO: 3+5+9+15, SEQ ID NO: 3+5+10+15, SEQ ID NO: 3+5+11+15, SEQ ID NO: 3+5+12+15, SEQ ID NO: 3+5+12+15, SEQ ID NO: 3+5+8+16, SEQ ID NO: 3+5+9+16, SEQ ID NO: 3+5+10+16, SEQ ID NO: 3+5+11+16, SEQ ID NO: 3+5+12+16, SEQ ID NO: 3+5+12+16, SEQ ID NO: 3+5+8+17, SEQ ID NO: 3+5+9+17, SEQ ID NO: 3+5+10+17, SEQ ID NO: 3+5+11+17, SEQ ID NO: 3+5+12+17, SEQ ID NO: 3+5+12+17, SEQ ID NO: 3+5+8+18, SEQ ID NO: 3+5+9+18, SEQ ID NO: 3+5+10+18, SEQ ID NO: 3+5+11+18, SEQ ID NO: 3+5+12+18, SEQ ID NO: 3+5+12+18, SEQ ID NO: 3+5+8+19, SEQ ID NO: 3+5+9+19, SEQ ID NO: 3+5+10+19, SEQ ID NO: 3+5+11+19, SEQ ID NO: 3+5+12+19, SEQ ID NO: 3+5+12+19, SEQ ID NO: 3+5+8+20, SEQ ID NO: 3+5+9+20, SEQ ID NO: 3+5+10+20, SEQ ID NO: 3+5+11+20, SEQ ID NO: 3+5+12+20, SEQ ID NO: 3+5+12+20, SEQ ID NO: 3+5+8+21, SEQ ID NO: 3+5+9+21, SEQ ID NO: 3+5+10+21, SEQ ID NO: 3+5+11+21, SEQ ID NO: 3+5+12+21, SEQ ID NO: 3+5+12+21, SEQ ID NO: 3+5+8+22, SEQ ID NO: 3+5+9+22, SEQ ID NO: 3+5+10+22, SEQ ID NO: 3+5+11+22, SEQ ID NO: 3+5+12+22, SEQ ID NO: 3+5+12+22, SEQ ID NO: 3+5+8+23, SEQ ID NO: 3+5+9+23, SEQ ID NO: 3+5+10+23, SEQ ID NO: 3+5+11+23, SEQ ID NO: 3+5+12+23, SEQ ID NO: 3+5+12+23, SEQ ID NO: 3+6+8+14, SEQ ID NO: 3+6+9+14, SEQ ID NO: 3+6+10+14, SEQ ID NO: 3+6+11+14, SEQ ID NO: 3+6+12+14, SEQ ID NO: 3+6+12+14, SEQ ID NO: 3+6+8+15, SEQ ID NO: 3+6+9+15, SEQ ID NO: 3+6+10+15, SEQ ID NO: 3+6+11+15, SEQ ID NO: 3+6+12+15, SEQ ID NO: 3+6+12+15, SEQ ID NO: 3+6+8+16, SEQ ID NO: 3+6+9+16, SEQ ID NO: 3+6+10+16, SEQ ID NO: 3+6+11+16, SEQ ID NO: 3+6+12+16, SEQ ID NO: 3+6+12+16, SEQ ID NO: 3+6+8+17, SEQ ID NO: 3+6+9+17, SEQ ID NO: 3+6+10+17, SEQ ID NO: 3+6+11+17, SEQ ID NO: 3+6+12+17, SEQ ID NO: 3+6+12+17, SEQ ID NO: 3+6+8+18, SEQ ID NO: 3+6+9+18, SEQ ID NO: 3+6+10+18, SEQ ID NO: 3+6+11+18, SEQ ID NO: 3+6+12+18, SEQ ID NO: 3+6+12+18, SEQ ID NO: 3+6+8+19, SEQ ID NO: 3+6+9+19, SEQ ID NO: 3+6+10+19, SEQ ID NO: 3+6+11+19, SEQ ID NO: 3+6+12+19, SEQ ID NO: 3+6+12+19, SEQ ID NO: 3+6+8+20, SEQ ID NO: 3+6+9+20, SEQ ID NO: 3+6+10+20, SEQ ID NO: 3+6+11+20, SEQ ID NO: 3+6+12+20, SEQ ID NO: 3+6+12+20, SEQ ID NO: 3+6+8+21, SEQ ID NO: 3+6+9+21, SEQ ID NO: 3+6+10+21, SEQ ID NO: 3+6+11+21, SEQ ID NO: 3+6+12+21, SEQ ID NO: 3+6+12+21, SEQ ID NO: 3+6+8+22, SEQ ID NO: 3+6+9+22, SEQ ID NO: 3+6+10+22, SEQ ID NO: 3+6+11+22, SEQ ID NO: 3+6+12+22, SEQ ID NO: 3+6+12+22, SEQ ID NO: 3+6+8+23, SEQ ID NO: 3+6+9+23, SEQ ID NO: 3+6+10+23, SEQ ID NO: 3+6+11+23, SEQ ID NO: 3+6+12+23, SEQ ID NO: 3+6+12+23, SEQ ID NO: 3+7+8+14, SEQ ID NO: 3+7+9+14, SEQ ID NO: 3+7+10+14, SEQ ID NO: 3+7+11+14, SEQ ID NO: 3+7+12+14, SEQ ID NO: 3+7+12+14, SEQ ID NO: 3+7+8+15, SEQ ID NO: 3+7+9+15, SEQ ID NO: 3+7+10+15, SEQ ID NO: 3+7+11+15, SEQ ID NO: 3+7+12+15, SEQ ID NO: 3+7+12+15, SEQ ID NO: 3+7+8+16, SEQ ID NO: 3+7+9+16, SEQ ID NO: 3+7+10+16, SEQ ID NO: 3+7+11+16, SEQ ID NO: 3+7+12+16, SEQ ID NO: 3+7+12+16, SEQ ID NO: 3+7+8+17, SEQ ID NO: 3+7+9+17, SEQ ID NO: 3+7+10+17, SEQ ID NO: 3+7+11+17, SEQ ID NO: 3+7+12+17, SEQ ID NO: 3+7+12+17, SEQ ID NO: 3+7+8+18, SEQ ID NO: 3+7+9+18, SEQ ID NO: 3+7+10+18, SEQ ID NO: 3+7+11+18, SEQ ID NO: 3+7+12+18, SEQ ID NO: 3+7+12+18, SEQ ID NO: 3+7+8+19, SEQ ID NO: 3+7+9+19, SEQ ID NO: 3+7+10+19, SEQ ID NO: 3+7+11+19, SEQ ID NO: 3+7+12+19, SEQ ID NO: 3+7+12+19, SEQ ID NO: 3+7+8+20, SEQ ID NO: 3+7+9+20, SEQ ID NO: 3+7+10+20, SEQ ID NO: 3+7+11+20, SEQ ID NO: 3+7+12+20, SEQ ID NO: 3+7+12+20, SEQ ID NO: 3+7+8+21, SEQ ID NO: 3+7+9+21, SEQ ID NO: 3+7+10+21, SEQ ID NO: 3+7+11+21, SEQ ID NO: 3+7+12+21, SEQ ID NO: 3+7+12+21, SEQ ID NO: 3+7+8+22, SEQ ID NO: 3+7+9+22, SEQ ID NO: 3+7+10+22, SEQ ID NO: 3+7+11+22, SEQ ID NO: 3+7+12+22, SEQ ID NO: 3+7+12+22, SEQ ID NO: 3+7+8+23, SEQ ID NO: 3+7+9+23, SEQ ID NO: 3+7+10+23, SEQ ID NO: 3+7+11+23, SEQ ID NO: 3+7+12+23, SEQ ID NO: 3+7+12+23, SEQ ID NO: 4+5+8+14, SEQ ID NO: 4+5+9+14, SEQ ID NO: 4+5+10+14, SEQ ID NO: 4+5+11+14, SEQ ID NO: 4+5+12+14, SEQ ID NO: 4+5+12+14, SEQ ID NO: 4+5+8+15, SEQ ID NO: 4+5+9+15, SEQ ID NO: 4+5+10+15, SEQ ID NO: 4+5+11+15, SEQ ID NO: 4+5+12+15, SEQ ID NO: 4+5+12+15, SEQ ID NO: 4+5+8+16, SEQ ID NO: 4+5+9+16, SEQ ID NO: 4+5+10+16, SEQ ID NO: 4+5+11+16, SEQ ID NO: 4+5+12+16, SEQ ID NO: 4+5+12+16, SEQ ID NO: 4+5+8+17, SEQ ID NO: 4+5+9+17, SEQ ID NO: 4+5+10+17, SEQ ID NO: 4+5+11+17, SEQ ID NO: 4+5+12+17, SEQ ID NO: 4+5+12+17, SEQ ID NO: 4+5+8+18, SEQ ID NO: 4+5+9+18, SEQ ID NO: 4+5+10+18, SEQ ID NO: 4+5+11+18, SEQ ID NO: 4+5+12+18, SEQ ID NO: 4+5+12+18, SEQ ID NO: 4+5+8+19, SEQ ID NO: 4+5+9+19, SEQ ID NO: 4+5+10+19, SEQ ID NO: 4+5+11+19, SEQ ID NO: 4+5+12+19, SEQ ID NO: 4+5+12+19, SEQ ID NO: 4+5+8+20, SEQ ID NO: 4+5+9+20, SEQ ID NO: 4+5+10+20, SEQ ID NO: 4+5+11+20, SEQ ID NO: 4+5+12+20, SEQ ID NO: 4+5+12+20, SEQ ID NO: 4+5+8+21, SEQ ID NO: 4+5+9+21, SEQ ID NO: 4+5+10+21, SEQ ID NO: 4+5+11+21, SEQ ID NO: 4+5+12+21, SEQ ID NO: 4+5+12+21, SEQ ID NO: 4+5+8+22, SEQ ID NO: 4+5+9+22, SEQ ID NO: 4+5+10+22, SEQ ID NO: 4+5+11+22, SEQ ID NO: 4+5+12+22, SEQ ID NO: 4+5+12+22, SEQ ID NO: 4+5+8+23, SEQ ID NO: 4+5+9+23, SEQ ID NO: 4+5+10+23, SEQ ID NO: 4+5+11+23, SEQ ID NO: 4+5+12+23, SEQ ID NO: 4+5+12+23, SEQ ID NO: 4+6+8+14, SEQ ID NO: 4+6+9+14, SEQ ID NO: 4+6+10+14, SEQ ID NO: 4+6+11+14, SEQ ID NO: 4+6+12+14, SEQ ID NO: 4+6+12+14, SEQ ID NO: 4+6+8+15, SEQ ID NO: 4+6+9+15, SEQ ID NO: 4+6+10+15, SEQ ID NO: 4+6+11+15, SEQ ID NO: 4+6+12+15, SEQ ID NO: 4+6+12+15, SEQ ID NO: 4+6+8+16, SEQ ID NO: 4+6+9+16, SEQ ID NO: 4+6+10+16, SEQ ID NO: 4+6+11+16, SEQ ID NO: 4+6+12+16, SEQ ID NO: 4+6+12+16, SEQ ID NO: 4+6+8+17, SEQ ID NO: 4+6+9+17, SEQ ID NO: 4+6+10+17, SEQ ID NO: 4+6+11+17, SEQ ID NO: 4+6+12+17, SEQ ID NO: 4+6+12+17, SEQ ID NO: 4+6+8+18, SEQ ID NO: 4+6+9+18, SEQ ID NO: 4+6+10+18, SEQ ID NO: 4+6+11+18, SEQ ID NO: 4+6+12+18, SEQ ID NO: 4+6+12+18, SEQ ID NO: 4+6+8+19, SEQ ID NO: 4+6+9+19, SEQ ID NO: 4+6+10+19, SEQ ID NO: 4+6+11+19, SEQ ID NO: 4+6+12+19, SEQ ID NO: 4+6+12+19, SEQ ID NO: 4+6+8+20, SEQ ID NO: 4+6+9+20, SEQ ID NO: 4+6+10+20, SEQ ID NO: 4+6+11+20, SEQ ID NO: 4+6+12+20, SEQ ID NO: 4+6+12+20, SEQ ID NO: 4+6+8+21, SEQ ID NO: 4+6+9+21, SEQ ID NO: 4+6+10+21, SEQ ID NO: 4+6+11+21, SEQ ID NO: 4+6+12+21, SEQ ID NO: 4+6+12+21, SEQ ID NO: 4+6+8+22, SEQ ID NO: 4+6+9+22, SEQ ID NO: 4+6+10+22, SEQ ID NO: 4+6+11+22, SEQ ID NO: 4+6+12+22, SEQ ID NO: 4+6+12+22, SEQ ID NO: 4+6+8+23, SEQ ID NO: 4+6+9+23, SEQ ID NO: 4+6+10+23, SEQ ID NO: 4+6+11+23, SEQ ID NO: 4+6+12+23, SEQ ID NO: 4+6+12+23, SEQ ID NO: 4+7+8+14, SEQ ID NO: 4+7+9+14, SEQ ID NO: 4+7+10+14, SEQ ID NO: 4+7+11+14, SEQ ID NO: 4+7+12+14, SEQ ID NO: 4+7+12+14, SEQ ID NO: 4+7+8+15, SEQ ID NO: 4+7+9+15, SEQ ID NO: 4+7+10+15, SEQ ID NO: 4+7+11+15, SEQ ID NO: 4+7+12+15, SEQ ID NO: 4+7+12+15, SEQ ID NO: 4+7+8+16, SEQ ID NO: 4+7+9+16, SEQ ID NO: 4+7+10+16, SEQ ID NO: 4+7+11+16, SEQ ID NO: 4+7+12+16, SEQ ID NO: 4+7+12+16, SEQ ID NO: 4+7+8+17, SEQ ID NO: 4+7+9+17, SEQ ID NO: 4+7+10+17, SEQ ID NO: 4+7+11+17, SEQ ID NO: 4+7+12+17, SEQ ID NO: 4+7+12+17, SEQ ID NO: 4+7+8+18, SEQ ID NO: 4+7+9+18, SEQ ID NO: 4+7+10+18, SEQ ID NO: 4+7+11+18, SEQ ID NO: 4+7+12+18, SEQ ID NO: 4+7+12+18, SEQ ID NO: 4+7+8+19, SEQ ID NO: 4+7+9+19, SEQ ID NO: 4+7+10+19, SEQ ID NO: 4+7+11+19, SEQ ID NO: 4+7+12+19, SEQ ID NO: 4+7+12+19, SEQ ID NO: 4+7+8+20, SEQ ID NO: 4+7+9+20, SEQ ID NO: 4+7+10+20, SEQ ID NO: 4+7+11+20, SEQ ID NO: 4+7+12+20, SEQ ID NO: 4+7+12+20, SEQ ID NO: 4+7+8+21, SEQ ID NO: 4+7+9+21, SEQ ID NO: 4+7+10+21, SEQ ID NO: 4+7+11+21, SEQ ID NO: 4+7+12+21, SEQ ID NO: 4+7+12+21, SEQ ID NO: 4+7+8+22, SEQ ID NO: 4+7+9+22, SEQ ID NO: 4+7+10+22, SEQ ID NO: 4+7+11+22, SEQ ID NO: 4+7+12+22, SEQ ID NO: 4+7+12+22, SEQ ID NO: 4+7+8+23, SEQ ID NO: 4+7+9+23, SEQ ID NO: 4+7+10+23, SEQ ID NO: 4+7+11+23, SEQ ID NO: 4+7+12+23 and SEQ ID NO: 4+7+12+23.

For instance, the prognosis method according to the invention can be carried out by determining the expression status of the above combinations SEQ ID NO: 1 + SEQ ID NO: 5 + SEQ ID NO: 13 + SEQ ID NO: 23, wherein
a. if neither SEQ ID NO: 1 nor SEQ ID NO : 5, nor SEQ ID NO: 13 nor SEQ ID NO: 23 is expressed , the patient survival rate at 30 months, 60 months or 120 months following the diagnosis is from about 19% to about 88% or more, and
b. if either SEQ ID NO: 1 or SEQ ID NO : 5, or SEQ ID NO: 13 or SEQ ID NO: 23 is expressed , the patient survival rate at 30 months, 60 months or 120 months following the diagnosis is from about 0% to about 73%.

According to the invention, the terms "about X%" means that the percentage of survival proposed for the prognosis method have to be considered with a standard deviation corresponding to individual variability. This standard deviation is 5%

To summarise, the invention relates to the use of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
   a. at least one gene belongs to a first set A of 4 genes comprising or consisting of the nucleic acid sequences SEQ ID NO: 1 to 4,
   b. at least one gene belongs to a second set B of 3 genes comprising or consisting of the nucleic acid sequences SEQ ID NO: 5 to 7
   c. at least one gene belongs to a third set C of 6 genes comprising or consisting of the nucleic acid sequences SEQ ID NO: 8 to 13, and
   d. at least one gene belongs to a fourth set D of 9 genes comprising or consisting of the nucleic acid sequences SEQ ID NO: 14-23,
◆ at least 4 proteins being such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30, and
   c. at least one protein belongs to a third set CP of 6 proteins comprising or consisting of the amino acid sequences SEQ ID NO: 31-36,
   d. at least one protein belongs to a fourth set BP of 9 proteins comprising or consisting of the amino acid sequences SEQ ID NO: 37-46,
◆ at least 4 antibodies directed against said 4 proteins being such that
   a. at least one antibody that ineract with at least one protein that belongs to a first set AP of 4 proteins comprising or consisting of the amino acid sequences SEQ ID NO: 24-27,
   b. at least one antibody that ineract with at least one protein that belongs a second set BP of 3 proteins comprising or consisting of the amino acid sequences SEQ ID NO: 28-30,
   c. at least one antibody that ineract with at least one protein that belongs a third set CP of 6 proteins comprising or consisting of the amino acid sequences SEQ ID NO: 31-36, and
   d. at least one antibody that ineract with at least one protein that belongs a fourth set BP of 9 proteins comprising or consisting of the amino acid sequences SEQ ID NO: 37-46,
for the implementation of a prognosis method, preferably *in vitro,* of the survival rate of a patient afflicted by a lung cancer, said lung cancer being previously classified as a lung tumour belonging from the group consisting of ADK, SQC, BAS et LCNE,
wherein
- the nucleic acid sequences SEQ ID NO: 1 to 4 and/or the amino acid sequences SEQ ID NO: 24 to 27 are expressed in BAS,
- the nucleic acid sequences SEQ ID NO: 5 to 7 and/or the amino acid sequences SEQ ID NO: 28 to 30 are expressed in ADK,
- the nucleic acid sequences SEQ ID NO: 8 to 13 and/or the amino acid sequences SEQ ID NO: 31 to 36 are expressed in SQC,
- the nucleic acid sequences SEQ ID NO: 14 to 23 and/or the amino acid sequences SEQ ID NO: 37 to 46 are expressed in LCNE,
said prognosis being such that:
either
   ■ if none of the 23 genes of said set is expressed, the patient survival rate during a period of time from 30 to 120 months after the diagnosis of said lung cancer is from about 59% to about 78% or more, and
   ■ if at least one gene of at least one set A, B, C or D is expressed , the patient survival rate during a period of time from 30 to 120 months after the diagnosis of said lung cancer is about from about 3% to about 70%,
or
   ■ if none of the 23 proteins of said set is expressed, the patient survival rate during a period of time from 30 to 120 months after the diagnosis of said lung cancer is from about 59% to about 78% or more, and
   ■ if at least one protein of at least one set AP, BP, CP or DP is expressed, the patient survival rate during a period of time from 30 to 120 months after the diagnosis of said lung cancer is about from about 3% to about 70%,
or
   ■ if none of the antibodies directed against said 23 proteins of said set is expressed, the patient survival rate during a period of time from 30 to 120 months after the diagnosis of said lung cancer is from about 59% to about 78% or more, and
   ■ if at least one antibody directed against one protein of of at least one set AP, BP, CP or DP is expressed, the patient survival rate during a period of time from 30 to 120 months after the diagnosis of said lung cancer is about from about 3% to about 70%,
   wherein said gene, protein or antibody is determined as being expressed when:
   - either it is expressed in a sample of a patient afflicted by a lung cancer but not in a control sample of an healthy individual,
   - or it is expressed at a level above a threshold corresponding to a background signal observed in a series of reference healthy tissues for each detection method, and
   said gene, protein or antibody is determined as being not expressed when:
   - it is neither expressed in a sample of a patient afflicted by a lung cancer nor in a control sample of an healthy individual,
   - or it is expressed in a sample of a patient afflicted by a lung cancer at a level substancially equal or inferior to the level in a control sample of an healthy individual.

According to the invention, "a control sample of an healthy individual" corresponds to a somatic tissue in which the CT gene is not expressed or weakly expressed as defined above.

In the invention "is expressed at a level above a threshold corresponding to a background signal observed in a series of reference healthy tissues for each detection method" means that the threshold, which corresponds to the key step of the invention, is determined by measuring the background signal in negative controle samples of healthy tissues, in which there is no expression of CT genes.

This background signal depends upon the method used to carry out the invention. However, it is easy for a skilled person to measure such threshold whatever the method used, i.e.:
- if the number of control samples is significantly statistically representative, e.g. at least 30 independent control samples, and the background signal of these control sample follows a normal distributation (Gaussian distribution), the threshold is determined by the mean + 2 standard deviations of the background signal measured in the control samples,
- If the number of control sample is not statically representative, e.g. less than 30 independent control samples, and the background signal of these control sample does not follow a normal distributation, the threshold is determined as being the maximal value of the background signal measured in the control samples.

According to the invention, the gene comprising or consisting of the nucleic acid sequence SEQ ID NO : 1 codes for the protein comprising or consisting of the amino acid sequence SEQ ID NO: 24, the gene comprising or consisting of the nucleic acid sequence SEQ ID NO : 2 codes for the protein comprising or consisting of the amino acid sequence SEQ ID NO: 25, the gene comprising or consisting of the nucleic acid sequence SEQ ID NO : 3 codes for the protein comprising or consisting of the amino acid sequence SEQ ID NO: 26, the gene comprising or consisting of the nucleic acid sequence SEQ ID NO : 4 codes for the protein comprising or consisting of the amino acid sequence SEQ ID NO: 27, the gene comprising or consisting of the nucleic acid sequence SEQ ID NO : 5 codes for the protein comprising or consisting of the amino acid sequence SEQ ID NO : 28, the gene comprising or consisting of the nucleic acid sequence SEQ ID NO : 6 codes for the protein comprising or consisting of the amino acid sequence SEQ ID NO : 29, the gene comprising or consisting of the nucleic acid sequence SEQ ID NO : 7 codes for the protein comprising or consisting of the amino acid sequence SEQ ID NO : 30, the gene comprising or consisting of the nucleic acid sequence SEQ ID NO : 8 codes for the protein comprising or consisting of the amino acid sequence SEQ ID NO : 31, the gene comprising or consisting of the nucleic acid sequence SEQ ID NO : 9 codes for the protein comprising or consisting of the amino acid sequence SEQ ID NO : 32, the gene comprising or consisting of the nucleic acid sequence SEQ ID NO : 10 codes for the protein comprising or consisting of the amino acid sequence SEQ ID NO : 33, the gene comprising or consisting of the nucleic acid sequence SEQ ID NO : 11 codes for the protein comprising or consisting of the amino acid sequence SEQ ID NO : 34, the gene comprising or consisting of the nucleic acid sequence SEQ ID NO : 12 codes for the protein comprising or consisting of the amino acid sequence SEQ ID NO : 35, the gene comprising or consisting of the nucleic acid sequence SEQ ID NO : 13 codes for the protein comprising or consisting of the amino acid sequence SEQ ID NO : 36, the gene comprising or consisting of the nucleic acid sequence SEQ ID NO : 14 codes for the protein comprising or consisting of the amino acid sequence SEQ ID NO : 37, the gene comprising or consisting of the nucleic acid sequence SEQ ID NO : 15 codes for the protein comprising or consisting of the amino acid sequence SEQ ID NO : 38, the gene comprising or consisting of the nucleic acid sequence SEQ ID NO : 16 codes for the protein comprising or consisting of the amino acid sequence SEQ ID NO : 39, the gene comprising or consisting of the nucleic acid sequence SEQ ID NO : 17 codes for the protein comprising or consisting of the amino acid sequence SEQ ID NO : 40, the gene comprising or consisting of the nucleic acid sequence SEQ ID NO : 18 codes for the protein comprising or consisting of the amino acid sequence SEQ ID NO : 41, the gene comprising or consisting of the nucleic acid sequence SEQ ID NO : 19 codes for the protein comprising or consisting of the amino acid sequence SEQ ID NO : 42, the gene comprising or consisting of the nucleic acid sequence SEQ ID NO : 20 codes for the protein comprising or consisting of the amino acid sequence SEQ ID NO : 43, the gene comprising or consisting of the nucleic acid sequence SEQ ID NO : 21 codes for the protein comprising or consisting of the amino acid sequence SEQ ID NO : 44, the gene comprising or consisting of the nucleic acid sequence SEQ ID NO : 22 codes for the protein comprising or consisting of the amino acid sequence SEQ ID NO : 45 and the gene comprising or consisting of the nucleic acid sequence SEQ ID NO : 23 codes for the protein comprising or consisting of the amino acid sequence SEQ ID NO : 46.

The 23 genes according to the invention code for 23 proteins. The 23 proteins have been classified by the Inventors in two sets:
a. a first set of 4 proteins, expressed or present in expressed in BAS,
b. a second set of 3 proteins, expressed in ADK,
c. a third set of 6 proteins, expressed in SQC, and
d. a fourth set of 10 proteins expressed in LCNE.

The first set, also called in the invention set AP, of 4 proteins consists of the proteins comprising or constituted by the amino acid sequences SEQ ID NO: 24, SEQ ID NO: 25, SEQ ID NO: 26 and SEQ ID NO: 27.

The second set, also called in the invention set BP, of 3 proteins consists of the proteins comprising or constituted by the amino acid sequences SEQ ID NO: 28, SEQ ID NO: 29 and SEQ ID NO: 30.

The third set, also called in the invention set BP, of 6 proteins consists of the proteins comprising or constituted by the amino acid sequences SEQ ID NO: 31, SEQ ID NO: 32, SEQ ID NO: 33, SEQ ID NO: 34, SEQ ID NO: 35 and SEQ ID NO: 36.

The fourth set, also called in the invention set BP, of 10 proteins consists of the proteins comprising or constituted by the amino acid sequences SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

The inventors have also defined subsets of each of the above sets AP and BP as follows: Set AP is divided into 4 subsets DP1, AP5, and AP6, said subset being such that :
a. subset AP1 consists of the proteins comprising or being constituted by the amino acid sequences SEQ ID NO: 24 and SEQ ID NO: 25,
b. subset AP5 consists of the protein comprising or being constituted by the amino acid sequence SEQ ID NO: 26, and
c. subset AP6 consists of the proteins comprising or being constituted by the amino acid sequences SEQ ID NO: 27.

Set AP can also be divided into the following subsets :
a. subset AP1 consists of the proteins comprising or being constituted by the amino acid sequences SEQ ID NO: 24 and SEQ ID NO: 25,
b. subset AP2 consists of the proteins comprising or being constituted by the amino acid sequences SEQ ID NO: 24, SEQ ID NO: 25 and SEQ ID NO: 26,
c. subset AP3 consists of the proteins comprising or being constituted by the amino acid sequences SEQ ID NO: 24, SEQ ID NO: 25, SEQ ID NO: 26 and SEQ ID NO: 27.

Set BP is divided into 3 subsets BP1 and BP3, said subset being such that :
a. subset BP1 consists of the proteins comprising or being constituted by the amino acid sequences SEQ ID NO: 28 and SEQ ID NO: 29, and
b. subset BP3 consists of the proteins comprising or being constituted by the amino acid sequences SEQ ID NO: 30.

Set BP can also be divided into the following subsets :
a. subset BP1 consists of the proteins comprising or being constituted by the amino acid sequences SEQ ID NO: 28 and SEQ ID NO: 29, and
b. subset BP2 consists of the proteins comprising or being constituted by the amino acid sequences SEQ ID NO: 28, SEQ ID NO: 29SEQ ID NO: 30.

Set CP is divided into 3 subsets CP1, CP4 and CP5, said subset being such that:
a. subset CP1 consists of the proteins comprising or being constituted by the amino acid sequences SEQ ID NO: 31 and SEQ ID NO: 32,
b. subset CP4 consists of the proteins comprising or being constituted by the amino acid sequences SEQ ID NO: 33 and SEQ ID NO: 34,
c. subset CP5 consists of the proteins comprising or being constituted by the amino acid sequences SEQ ID NO: 35 and SEQ ID NO: 36.

Set CP can also be divided into the following subsets :
a. subset CP1 consists of the proteins comprising or being constituted by the amino acid sequences SEQ ID NO: 31 and SEQ ID NO: 32,
b. subset CP2 consists of the proteins comprising or being constituted by the amino acid sequences SEQ ID NO: 31, SEQ ID NO: 32, SEQ ID NO: 33 and SEQ ID NO: 34, and
c. subset CP3 consists of the proteins comprising or being constituted by the amino acid sequences SEQ ID NO: 31, SEQ ID NO: 32, SEQ ID NO: 33, SEQ ID NO: 34, SEQ ID NO: 35 and SEQ ID NO: 36.

Set PP is divided into 4 subsets DP1, DP5, DP6, DP7, said subset being such that :
a. subset DP1 consists of the proteins comprising or being constituted by the amino acid sequences SEQ ID NO: 37 and SEQ ID NO: 38,
b. subset DP5 consists of the proteins comprising or being constituted by the amino acid sequences SEQ ID NO: 39, SEQ ID NO: 40, SEQ ID NO: 41, SEQ ID NO: 42 and SEQ ID NO: 43,
c. subset DP6 consists of the proteins comprising or being constituted by the amino acid sequences SEQ ID NO: 44, and
d. subset DP7 consists of the proteins comprising or being constituted by the amino acid sequences SEQ ID NO: 45 and SEQ ID NO: 46

Set DP can also be divided into the following subsets :
a. subset DP1 consists of the proteins comprising or being constituted by the amino acid sequences SEQ ID NO: 37 and SEQ ID NO: 38,
b. subset DP2 consists of the proteins comprising or being constituted by the amino acid sequences SEQ ID NO: 37, SEQ ID NO: 38, SEQ ID NO: 39, SEQ ID NO: 40, SEQ ID NO: 41, SEQ ID NO: 42 and SEQ ID NO: 43,
c. subset DP3 consists of the proteins comprising or being constituted by the amino acid sequences SEQ ID NO: 37, SEQ ID NO: 38, SEQ ID NO: 39, SEQ ID NO: 40, SEQ ID NO: 41, SEQ ID NO: 42, SEQ ID NO: 43 and SEQ ID NO: 44.
d. subset DP4 consists of the proteins comprising or being constituted by the amino acid sequences SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

In one advantageous embodiment, the invention relates to the use as defined above of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
   a. at least one gene belongs to a first subset A1 of 2 genes comprising or consisting of the nucleic acid sequences SEQ ID NO: 1 to 2
   b. at least one gene belongs to a second subset B1 of 2 genes comprising or consisting of the nucleic acid sequences SEQ ID NO: 5 to 6
   c. at least one gene belongs to a third subset C1 of 2 genes comprising or consisting of the nucleic acid sequences SEQ ID NO: 8 to 9, and
   d. at least one gene belongs to a fourth subset D1 of 2 genes comprising or consisting of the nucleic acid sequences SEQ ID NO: 14 to 15
◆ at least 4 proteins being such that
   a. at least one protein belongs to a first subset AP1 of 2proteins comprising or consisting of the amino acid sequences SEQ ID NO: 24 to 25,
   b. at least one protein belongs to a second subset BP1 of 2 proteins comprising or consisting of the amino acid sequences SEQ ID NO: 28 to 29
   c. at least one protein belongs to a second subset CP1 of 2 proteins comprising or consisting of the amino acid sequences SEQ ID NO: 31 to 32,
   d. at least one protein belongs to a second set DP1 of 2 proteins comprising or consisting of the amino acid sequences SEQ ID NO: 37 to 38,
◆ at least 4 antibodies directed against said 4 proteins being such that
   a. at least one antibody specifically recognises one protein that belongs to a first subset AP1 of 2 proteins comprising or consisting of the amino acid sequences SEQ ID NO: 24 to 25,
   b. at least one antibody specifically recognises one protein that belongs to a second set BP1 of 2 proteins comprising or consisting of the amino acid sequences SEQ ID NO: 27 to 28,
   c. at least one antibody specifically recognises one protein that belongs to a first subset CP1 of 2 proteins comprising or consisting of the amino acid sequences SEQ ID NO: 31 to 32,
   d. at least one antibody specifically recognises one protein that belongs to a first subset DP1 of 2 proteins comprising or consisting of the amino acid sequences SEQ ID NO: 37 to 38,
for the implementation of a prognosis method, preferably *in vitro,* of the survival rate of a patient afflicted by a lung cancer, said prognosis being such that:
either
   ■ if none of the genes of said subset A1, B1, C1 and D1 is expressed, the patient survival rate during a period of time from 30 to 120 months after the diagnosis of said lung cancer is from about 59% to about 78% or more, and
   ■ if at least one gene of at least one subset A1, B, C1 or D1 is expressed , the patient survival rate during a period of time from 30 to 120 months after the diagnosis of said lung cancer is about from about 3% to about 70%,
or
   ■ if none of the proteins of said subset AP1, BP1, CP1 and DP1 is expressed, the patient survival rate during a period of time from 30 to 120 months after the diagnosis of said lung cancer is from about 59% to about 78% or more, and
   ■ if at least one protein of at least one subset AP1, BP1, CP1 and DP1 is expressed, the patient survival rate during a period of time from 30 to 120 months after the diagnosis of said lung cancer is about from about 3% to about 70%,
or
   ■ if none of the antibodies directed against the proteins of said subset AP1, BP1, CP1 and DP1 is expressed, the patient survival rate during a period of time from 30 to 120 months after the diagnosis of said lung cancer is from about 59% to about 78% or more, and
   ■ if at least one antibody directed against one protein of said subset AP1, BP1, CP1 and DP1 is expressed, the patient survival rate during a period of time from 30 to 120 months after the diagnosis of said lung cancer is about from about 3% to about 70%.

In this advantageous embodiment, the prognosis method according to the invention can be carried out as by measuring at least the expression of the following 6 combinations of proteins: SEQ ID NO : 24 + 28 + 31 + 37, SEQ ID NO : 24 + 28 + 31 + 38, SEQ ID NO : 24 + 28 + 32 + 37, SEQ ID NO: 24 + 28 + 32 + 38, SEQ ID NO 24 + 29 + 31 + 37, SEQ ID NO : 24 + 29 + 31 + 38, SEQ ID NO : 24 + 29 + 32 + 37, SEQ ID NO : 24 + 29 + 32 + 38, SEQ ID NO : 25 + 28 + 31 + 37, SEQ ID NO : 25 + 28 + 31 + 38, SEQ ID NO : 25 + 28 + 32 + 37, SEQ ID NO : 25 + 28 + 32 + 38, SEQ ID NO : 25 + 29 + 31 + 37, SEQ ID NO : 25 + 29 + 31 + 38, SEQ ID NO : 25 + 29 + 32 + 37 and SEQ ID NO : 25 + 29+32+38.

For instance, the prognosis method according to the invention can be carried out by determining the expression status of the above combination SEQ ID NO: 24 + SEQ ID NO: 28 + SEQ ID NO: 32 + SEQ ID NO: 37, wherein:
■ if none of the genes SEQ ID NO: 1, SEQ ID NO: 5, SEQ ID NO: 8 and SEQ ID NO: 15 is expressed, the patient survival rate during a period of time from 30 to 120 months after the diagnosis of said lung cancer is from about 59% to about 78% or more, and
■ if at least one gene SEQ ID NO: 1, SEQ ID NO: 5, SEQ ID NO: 8 or SEQ ID NO: 15 is expressed , the patient survival rate during a period of time from 30 to 120 months after the diagnosis of said lung cancer is about from about 3% to about 70%.

Moreover, the prognosis method according to the invention can be carried out as by measuring at least the expression of the following 16 combinations of proteins: SEQ ID NO : 24 + 25 + 31 + 37, SEQ ID NO : 24 + 25 + 31 + 38, SEQ ID NO : 24 + 25 + 32 + 37, SEQ ID NO : 24 + 25 + 32 + 38, SEQ ID NO : 24 + 29 + 31 + 37, SEQ ID NO : 24 + 29 + 31 + 38, SEQ ID NO : 24 + 29 + 32 + 37, SEQ ID NO : 24 + 29 + 32 + 38, SEQ ID NO : 25 + 25 + 31 + 37, SEQ ID NO : 25 + 25 + 31 + 38, SEQ ID NO : 25 + 25 + 32 + 37, SEQ ID NO : 25 + 25 + 32 + 38, SEQ ID NO : 25 + 29 + 31 + 37, SEQ ID NO : 25 + 29 + 31 + 38, SEQ ID NO : 25 + 29 + 32 + 37 and SEQ ID NO : 25 + 29 + 32 + 38.

For instance, the prognosis method according to the invention can be carried out by determining the expression status of the above combination SEQ ID NO: 24 + SEQ ID NO: 5 + SEQ ID NO: 8 + SEQ ID NO: 15, wherein:
■ if none of the proteins SEQ ID NO: 24, SEQ ID NO: 28, SEQ ID NO: 32 and SEQ ID NO: 37 is expressed, the patient survival rate during a period of time from 30 to 120 months after the diagnosis of said lung cancer is from about 59% to about 78% or more, and
■ if at least one protein SEQ ID NO: 24, SEQ ID NO: 28, SEQ ID NO: 32 or SEQ ID NO: 37 is expressed , the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first subset A1 of 2 genes comprising or consisting of the nucleic acid sequences SEQ ID NO: 1 to 2
   b. at least one gene belongs to a second subset B1 of 2 genes comprising or consisting of the nucleic acid sequences SEQ ID NO: 5 to 6
   c. at least one gene belongs to a third subset C1 of 2 genes comprising or consisting of the nucleic acid sequences SEQ ID NO: 8 to 9, and
   d. at least one gene belongs to a fourth subset D1 of 2 genes comprising or consisting of the nucleic acid sequences SEQ ID NO: 14 to 15
for the implementation of a prognosis method, preferably *in vitro,* of the survival rate of a patient afflicted by a lung cancer, said prognosis being such that:
■ if none of the genes of said subset A1, B1, C1 and D1 is expressed, the patient survival rate during a period of time from 30 to 120 months after the diagnosis of said lung cancer is from about 59% to about 78% or more, and
■ if at least one gene of at least one subset A1, B, C1 or D1 is expressed , the patient survival rate during a period of time from 30 to 120 months after the diagnosis of said lung cancer is about from about 3% to about 70%.

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 proteins being such that
   a. at least one protein belongs to a first subset AP1 of 2proteins comprising or consisting of the amino acid sequences SEQ ID NO: 24 to 25,
   b. at least one protein belongs to a second subset BP1 of 2 proteins comprising or consisting of the amino acid sequences SEQ ID NO: 28 to 29
   c. at least one protein belongs to a second subset CP1 of 2 proteins comprising or consisting of the amino acid sequences SEQ ID NO: 31 to 32,
   d. at least one protein belongs to a second set DP1 of 2 proteins comprising or consisting of the amino acid sequences SEQ ID NO: 37 to 38,
for the implementation of a prognosis method, preferably *in vitro,* of the survival rate of a patient afflicted by a lung cancer, said prognosis being such that:
■ if none of the proteins of said subset AP1, BP1, CP1 and DP1 is expressed, the patient survival rate during a period of time from 30 to 120 months after the diagnosis of said lung cancer is from about 59% to about 78% or more, and
■ if at least one protein of at least one subset AP1, BP1, CP1 and DP1 is expressed, the patient survival rate during a period of time from 30 to 120 months after the diagnosis of said lung cancer is about from about 3% to about 70%.

In one advantageous embodiment, the invention relates to the use as defined above of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
   a. at least one gene belongs to a first subset A4 comprising or consisting of the nucleic acid sequences SEQ ID NO: 3
   b. at least one gene belongs to a second subset B1 of 2 genes comprising or consisting of the nucleic acid sequences SEQ ID NO: 5 to 6
   c. at least one gene belongs to a third subset C4 of 2 genes comprising or consisting of the nucleic acid sequences SEQ ID NO: 10 to 11, and
   d. at least one gene belongs to a fourth subset D5 of 2 genes comprising or consisting of the nucleic acid sequences SEQ ID NO: 16 to 19
◆ at least 4 proteins being such that
   a. at least one protein belongs to a first subset AP4 comprising or consisting of the amino acid sequences SEQ ID NO: 26,
   b. at least one protein belongs to a second subset BP1 of 2 proteins comprising or consisting of the amino acid sequences SEQ ID NO: 33 to 34
   c. at least one protein belongs to a second subset CP4 of 5 proteins comprising or consisting of the amino acid sequences SEQ ID NO: 37 to 38,
   d. at least one protein belongs to a second set DP5 of 2 proteins comprising or consisting of the amino acid sequences SEQ ID NO: 39 to 43,
◆ at least 4 antibodies directed against said 4 proteins being such that
   a. at least one antibody specifically recognises one protein that belongs to a first subset AP4 comprising or consisting of the amino acid sequences SEQ ID NO: 26,
   b. at least one antibody specifically recognises one protein that belongs to a second set BP1 of 2 proteins comprising or consisting of the amino acid sequences SEQ ID NO: 33 to 34,
   c. at least one antibody specifically recognises one protein that belongs to a first subset CP4 of 2 proteins comprising or consisting of the amino acid sequences SEQ ID NO: 31 to 32,
   d. at least one antibody specifically recognises one protein that belongs to a first subset DP1 of 2 proteins comprising or consisting of the amino acid sequences SEQ ID NO: 39 to 43,
for the implementation of a prognosis method, preferably *in vitro,* of the survival rate of a patient afflicted by a lung cancer, said prognosis being such that:
either
   ■ if none of the genes of said subset A4, B1, C4 and D5 is expressed, the patient survival rate during a period of time from 30 to 120 months after the diagnosis of said lung cancer is from about 59% to about 78% or more, and
   ■ if at least one gene of at least one subset A4, B, C4 or D5 is expressed , the patient survival rate during a period of time from 30 to 120 months after the diagnosis of said lung cancer is about from about 3% to about 70%,
or
   ■ if none of the proteins of said subset AP4, BP1, CP4 and DP5 is expressed, the patient survival rate during a period of time from 30 to 120 months after the diagnosis of said lung cancer is from about 59% to about 78% or more, and
   ■ if at least one protein of at least one subset AP4, BP1, CP4 and DP5 is expressed, the patient survival rate during a period of time from 30 to 120 months after the diagnosis of said lung cancer is about from about 3% to about 70%,
or
   ■ if none of the antibodies directed against the proteins of said subset AP4, BP1, CP4 and DP5 is expressed, the patient survival rate during a period of time from 30 to 120 months after the diagnosis of said lung cancer is from about 59% to about 78% or more, and
   ■ if at least one antibody directed against one protein of said subset AP4, BP1, CP4 and DP5 is expressed, the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first subset A4 comprising or consisting of the nucleic acid sequences SEQ ID NO: 3
   b. at least one gene belongs to a second subset B1 of 2 genes comprising or consisting of the nucleic acid sequences SEQ ID NO: 5 to 6
   c. at least one gene belongs to a third subset C4 of 2 genes comprising or consisting of the nucleic acid sequences SEQ ID NO: 10 to 11, and
   d. at least one gene belongs to a fourth subset D5 of 2 genes comprising or consisting of the nucleic acid sequences SEQ ID NO: 16 to 19
for the implementation of a prognosis method, preferably *in vitro,* of the survival rate of a patient afflicted by a lung cancer, said prognosis being such that:
■ if none of the genes of said subset A4, B1, C4 and D5 is expressed, the patient survival rate during a period of time from 30 to 120 months after the diagnosis of said lung cancer is from about 59% to about 78% or more, and
■ if at least one gene of at least one subset A4, B1, C4 or D5 is expressed , the patient survival rate during a period of time from 30 to 120 months after the diagnosis of said lung cancer is about from about 3% to about 70%,

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 proteins being such that
◆ at least 4 proteins being such that
   e. at least one protein belongs to a first subset AP4 comprising or consisting of the amino acid sequences SEQ ID NO: 26,
   f. at least one protein belongs to a second subset BP1 of 2 proteins comprising or consisting of the amino acid sequences SEQ ID NO: 33 to 34
   g. at least one protein belongs to a second subset CP4 of 5 proteins comprising or consisting of the amino acid sequences SEQ ID NO: 37 to 38,
   h. at least one protein belongs to a second set DP5 of 2 proteins comprising or consisting of the amino acid sequences SEQ ID NO: 39 to 43,
for the implementation of a prognosis method, preferably *in vitro,* of the survival rate of a patient afflicted by a lung cancer, said prognosis being such that:
■ if none of the proteins of said subset AP4, BP1, CP4 and DP5 is expressed, the patient survival rate during a period of time from 30 to 120 months after the diagnosis of said lung cancer is from about 59% to about 78% or more, and
■ if at least one protein of at least one subset AP4, BP1, CP4 and DP5 is expressed, the patient survival rate during a period of time from 30 to 120 months after the diagnosis of said lung cancer is about from about 3% to about 70%.

In one advantageous embodiment, the invention relates to the use of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
   a. at least one gene belongs to a first subset A2 of 3 genes comprising or consisting of the nucleic acid sequences SEQ ID NO: 1 to 3
   b. at least one gene belongs to a second subset B1 of 2 genes comprising or consisting of the nucleic acid sequences SEQ ID NO: 5 to 6
   c. at least one gene belongs to a third subset C2 of 4 genes comprising or consisting of the nucleic acid sequences SEQ ID NO: 8 to 11, and
   d. at least one gene belongs to a fourth subset D2 of 7 genes comprising or consisting of the nucleic acid sequences SEQ ID NO: 14 to 20
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP2 of 4 proteins comprising or consisting of the amino acid sequences SEQ ID NO: 31 to 34,
   d. at least one protein belongs to a second set DP2 of 7 proteins comprising or consisting of the amino acid sequences SEQ ID NO: 37 to 43,
◆ at least 4 antibodies directed against said 4 proteins being such that
   a. at least one antibody specifically recognises one protein that belongs to a first subset AP2 of 3 proteins comprising or consisting of the amino acid sequences SEQ ID NO: 24 to 26,
   b. at least one antibody specifically recognises one protein that belongs to a second set BP1 of 4 proteins comprising or consisting of the amino acid sequences SEQ ID NO: 27 to 28,
   c. at least one antibody specifically recognises one protein that belongs to a first subset CP2 of 4 proteins comprising or consisting of the amino acid sequences SEQ ID NO: 31 to 34,
   d. at least one antibody specifically recognises one protein that belongs to a first subset DP2 of 7 proteins comprising or consisting of the amino acid sequences SEQ ID NO: 37 to 43,
for the implementation of a prognosis method, preferably *in vitro,* of the survival rate of a patient afflicted by a lung cancer, said prognosis being such that:
either
   ■ if none of the genes of said subset A2, B1, C2 and D2 is expressed, the patient survival rate during a period of time from 30 to 120 months after the diagnosis of said lung cancer is from about 59% to about 78% or more, and
   ■ if at least one gene of at least one subset A2, B1, C2 or D2 is expressed , the patient survival rate during a period of time from 30 to 120 months after the diagnosis of said lung cancer is about from about 3% to about 70%,
or
   ■ if none of the proteins of said subset AP2, BP1, CP2 and DP2 is expressed, the patient survival rate during a period of time from 30 to 120 months after the diagnosis of said lung cancer is from about 59% to about 78% or more, and
   ■ if at least one protein of at least one subset AP2, BP1, CP2 and DP2 is expressed, the patient survival rate during a period of time from 30 to 120 months after the diagnosis of said lung cancer is about from about 3% to about 70%,
or
   ■ if none of the antibodies directed against the proteins of said subset AP2, BP1, CP2 and DP2 is expressed, the patient survival rate during a period of time from 30 to 120 months after the diagnosis of said lung cancer is from about 59% to about 78% or more, and
   ■ if at least one antibody directed against one protein of said subset AP2, BP1, CP2 and DP2 is expressed, the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first subset **A2** of 3 genes comprising or consisting of the nucleic acid sequences SEQ ID NO: 1 to 3
   b. at least one gene belongs to a second subset B1 of 2 genes comprising or consisting of the nucleic acid sequences SEQ ID NO: 5 to 6
   c. at least one gene belongs to a third subset C2 of 4 genes comprising or consisting of the nucleic acid sequences SEQ ID NO: 8 to 11, and
   d. at least one gene belongs to a fourth subset D2 of 7 genes comprising or consisting of the nucleic acid sequences SEQ ID NO: 14 to 20
   for the implementation of a prognosis method, preferably *in vitro,* of the survival rate of a patient afflicted by a lung cancer, said prognosis being such that:
   ■ if none of the genes of said subset A2, B1, C2 and D21 is expressed, the patient survival rate during a period of time from 30 to 120 months after the diagnosis of said lung cancer is from about 59% to about 78% or more, and
   ■ if at least one gene of at least one subset A2, B1, C2 or D2 is expressed , the patient survival rate during a period of time from 30 to 120 months after the diagnosis of said lung cancer is about from about 3% to about 70%.

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP2 of 4 proteins comprising or consisting of the amino acid sequences SEQ ID NO: 31 to 34,
   d. at least one protein belongs to a second set DP2 of 7 proteins comprising or consisting of the amino acid sequences SEQ ID NO: 37 to 43,
for the implementation of a prognosis method, preferably *in vitro,* of the survival rate of a patient afflicted by a lung cancer, said prognosis being such that:
■ if none of the proteins of said subset AP2, BP1, CP2 and DP2 is expressed, the patient survival rate during a period of time from 30 to 120 months after the diagnosis of said lung cancer is from about 59% to about 78% or more, and
■ if at least one protein of at least one subset AP2, BP1, CP2 and DP2 is expressed, the patient survival rate during a period of time from 30 to 120 months after the diagnosis of said lung cancer is about from about 3% to about 70%.

In one advantageous embodiment, the invention relates to the use of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
   a. at least one gene belongs to a first subset A2 of 3 genes comprising or consisting of the nucleic acid sequences SEQ ID NO: 1 to 3
   b. at least one gene belongs to a second subset B1 of 2 genes comprising or consisting of the nucleic acid sequences SEQ ID NO: 5 to 6
   c. at least one gene belongs to a third subset C3 of 6 genes comprising or consisting of the nucleic acid sequences SEQ ID NO: 8 to 13, and
   d. at least one gene belongs to a fourth subset D3 of 8 genes comprising or consisting of the nucleic acid sequences SEQ ID NO: 14 to 21,
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP3 of 6 proteins comprising or consisting of the amino acid sequences SEQ ID NO: 31 to 36,
   d. at least one protein belongs to a second set DP3 of 8 proteins comprising or consisting of the amino acid sequences SEQ ID NO: 37 to 44,
◆ at least 4 antibodies directed against said 4 proteins being such that
   a. at least one antibody specifically recognises one protein that belongs to a first subset AP2 of 3 proteins comprising or consisting of the amino acid sequences SEQ ID NO: 24 to 26,
   b. at least one antibody specifically recognises one protein that belongs to a second set BP1 of 4 proteins comprising or consisting of the amino acid sequences SEQ ID NO: 27 to 28,
   c. at least one antibody specifically recognises one protein that belongs to a first subset CP3 of 6 proteins comprising or consisting of the amino acid sequences SEQ ID NO: 31 to 36,
   d. at least one antibody specifically recognises one protein that belongs to a first subset DP3 of 8 proteins comprising or consisting of the amino acid sequences SEQ ID NO: 37 to 44,
for the implementation of a prognosis method, preferably *in vitro,* of the survival rate of a patient afflicted by a lung cancer, said prognosis being such that:
either
   ■ if none of the genes of said subset A2, B1, C3 and D3 is expressed, the patient survival rate during a period of time from 30 to 120 months after the diagnosis of said lung cancer is from about 59% to about 78% or more, and
   ■ if at least one gene of at least one subset A2, B1, C3 or D3 is expressed , the patient survival rate during a period of time from 30 to 120 months after the diagnosis of said lung cancer is about from about 3% to about 70%,
or
   ■ if none of the proteins of said subset AP2, BP1, CP3 and DP3 is expressed, the patient survival rate during a period of time from 30 to 120 months after the diagnosis of said lung cancer is from about 59% to about 78% or more, and
   ■ if at least one protein of at least one subset AP2, BP1, CP3 and DP3 is expressed, the patient survival rate during a period of time from 30 to 120 months after the diagnosis of said lung cancer is about from about 3% to about 70%,
or
   ■ if none of the antibodies directed against the proteins of said subset AP2, BP1, CP3 and DP3 is expressed, the patient survival rate during a period of time from 30 to 120 months after the diagnosis of said lung cancer is from about 59% to about 78% or more, and
   ■ if at least one antibody directed against one protein of said subset AP2, BP1, CP3 and DP3 is expressed, the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
a. at least one gene belongs to a first subset A2 of 3 genes comprising or consisting of the nucleic acid sequences SEQ ID NO: 1 to 3
b. at least one gene belongs to a second subset B1 of 2 genes comprising or consisting of the nucleic acid sequences SEQ ID NO: 5 to 6
c. at least one gene belongs to a third subset C3 of 6 genes comprising or consisting of the nucleic acid sequences SEQ ID NO: 8 to 13, and
d. at least one gene belongs to a fourth subset D3 of 8 genes comprising or consisting of the nucleic acid sequences SEQ ID NO: 14 to 21,
for the implementation of a prognosis method, preferably *in vitro,* of the survival rate of a patient afflicted by a lung cancer, said prognosis being such that:
■ if none of the genes of said subset A2, B1, C3 and D3 is expressed, the patient survival rate during a period of time from 30 to 120 months after the diagnosis of said lung cancer is from about 59% to about 78% or more, and
■ if at least one gene of at least one subset A2, B1, C3 or D3 is expressed , the patient survival rate during a period of time from 30 to 120 months after the diagnosis of said lung cancer is about from about 3% to about 70%.

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP3 of 6 proteins comprising or consisting of the amino acid sequences SEQ ID NO: 31 to 36,
   d. at least one protein belongs to a second set DP3 of 8 proteins comprising or consisting of the amino acid sequences SEQ ID NO: 37 to 44,
for the implementation of a prognosis method, preferably *in vitro,* of the survival rate of a patient afflicted by a lung cancer, said prognosis being such that:
■ if none of the proteins of said subset AP2, BP1, CP3 and DP3 is expressed, the patient survival rate during a period of time from 30 to 120 months after the diagnosis of said lung cancer is from about 59% to about 78% or more, and
■ if at least one protein of at least one subset AP2, BP1, CP3 and DP3 is expressed, the patient survival rate during a period of time from 30 to 120 months after the diagnosis of said lung cancer is about from about 3% to about 70%,

In one other embodiment, the invention relates to the use according as defined above, wherein said prognosis is such that
■ when said tumor is ADK :
   o if none of the genes of said set B or of the subset B1 is expressed, the patient survival rate is from about 76% to about 86%
   o if at least one of the genes of the set B or of the subset B1 is expressed, the patient survival rate is from about 39% to about 63%,
      or
   o if none of the proteins of said set BP or of the subset BP1 is expressed, the patient survival rate is from about 76% to about 86%
   o if at least one of the proteins of the set BP1 or of the subset BP1 is expressed, the patient survival rate is from about 39% to about 63%,
      or
   o if none of antibodies directed against the proteins of said set BP or of the subset BP1 is expressed, the patient survival rate is from about 76% to about 86%
   o if at least one antibody directed against at least one of the proteins of the set BP or of the subset BP1 is expressed, the patient survival rate is from about 39% to about 63%,
■ when said tumor is BAS :
   o if none of the genes of the set A or of subsets A1 or A2 is expressed, the patient survival rate is about 19% to about 65%
   o if at least one of the genes of the set A or of subsets A1 or A2 is expressed, the patient survival rate is about 0% to about 33%, or
   o if none of the proteins of the set AP or of subsets AP1 or AP2 is expressed, the patient survival rate is about 19% to about 65%
   o if at least one of the proteins of the set AP or of subsets AP1 or AP2 is expressed, the patient survival rate is about 0% to about 33%,
      or
   o if none of antibodies directed against the proteins of said set AP or of subsets AP1 or AP2 is expressed, the patient survival rate is about 19% to about 65%
   o if at least one antibody directed against at least one of the proteins of the set AP or of subsets AP1 or AP2 is expressed, the patient survival rate is about 0% to about 33%,
■ when said tumor is SQC :
   o if none of the genes of the set C or of the subsets C1 or C2 is expressed, the patient survival rate is about 58% to about 88%
   o if at least one of the genes of the set C or of the subsets C1 or C2 is expressed, the patient survival rate is from about 33% to about 64%,
      or
   o if none of the proteins of the set CP or of the subsets CP1 or CP2 is expressed, the patient survival rate is about 58% to about 88%
   o if at least one of the proteins of the set CP or of the subsets CP1 or CP2 is expressed, the patient survival rate is from about 33% to about 64%,
      or
   o if none of antibodies directed against the proteins of said set CP or of the subsets CP1 or CP2 is expressed, the patient survival rate is about 58% to about 88%
   o if at least one antibody directed against at least one of the proteins of the set CP or of the subsets CP1 or CP2 is expressed, the patient survival rate is from about 33% to about 64%,
■ when said tumor is LCNE :
   o if none of the genes of the set D or of the subsets D1, D2 or D3 is expressed, the patient survival rate is from about 40% to about 65%
   o if at least one of the genes of the set D or of the subsets D1, D2 or D3 is expressed, the patient survival rate is from about 0% to 7%,
      or
   o if none of the proteins of the set DP or of the subsets DP1, DP2 or DP3 is expressed, the patient survival rate is from about 40% to about 65%
   o if at least one of the proteins of the set DP or of the subsets DP1, DP2 or DP3 is expressed, the patient survival rate is from about 0% to 7%,
      or
   o if none of antibodies directed against the proteins of said set DP or of the subsets DP1, DP2 or DP3 is expressed, the patient survival rate is from about 40% to about 65%,
   o if at least one antibody directed against at least one of the proteins of the set DP or of the subsets DP1, DP2 or DP3 is expressed, the patient survival rate is from about 0% to 7%.

In one other embodiment, the invention relates to the use according as defined above, wherein said prognosis is such that
■ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
      or
   o if none of the proteins of the subset BP1 is expressed, the patient survival rate is from about 76% to about 86%
   o if at least one of the subset BP1 is expressed, the patient survival rate is from about 39% to about 63%,
      or
   o if none of antibodies directed against the proteins of the subset BP1 is expressed, the patient survival rate is from about 76% to about 86%
   o if at least one antibody directed against at least one of the proteins of the subset BP1 is expressed, the patient survival rate is from about 39% to about 63%,
■ when said tumor is BAS :
   o if none of the genes of subset A1 is expressed, the patient survival rate is about 19% to about 65%
   o if at least one of the genes of subsets A1 is expressed, the patient survival rate is about 0% to about 33%,
      or
   o if none of the proteins of subsets AP1 is expressed, the patient survival rate is about 19% to about 65%
   o if at least one of the proteins of the subsets AP1 or is expressed, the patient survival rate is about 0% to about 33%,
      or
   o if none of antibodies directed against the proteins of subset AP1 is expressed, the patient survival rate is about 19% to about 65%
   o if at least one antibody directed against at least one of the proteins of the subset AP1 is expressed, the patient survival rate is about 0% to about 33%,
■ when said tumor is SQC :
   o if none of the genes of the subset C1 is expressed, the patient survival rate is about 58% to about 88%
   o if at least one of the genes of the subset C1 is expressed, the patient survival rate is from about 33% to about 64%,
      or
   o if none of the proteins of the subset CP1 is expressed, the patient survival rate is about 58% to about 88%
   o if at least one of the proteins of the subset CP1 is expressed, the patient survival rate is from about 33% to about 64%,
      or
   o if none of antibodies directed against the proteins of the subsets CP1 is expressed, the patient survival rate is about 58% to about 88%
   o if at least one antibody directed against at least one of the proteins of the subset CP1 is expressed, the patient survival rate is from about 33% to about 64%,
■ when said tumor is LCNE :
   o if none of the genes of the subset D1 is expressed, the patient survival rate is from about 40% to about 65%
   o if at least one of the genes of the subset D1 is expressed, the patient survival rate is from about 0% to 7%,
      or
   o if none of the proteins of the subset **DP1** is expressed, the patient survival rate is from about 40% to about 65%
   o if at least one of the proteins of the subset **DP1** is expressed, the patient survival rate is from about 0% to 7%,
      or
   o if none of antibodies directed against the proteins of the subset **DP1** is expressed, the patient survival rate is from about 40% to about 65%,
   o if at least one antibody directed against at least one of the proteins of the the subset **DP1** is expressed, the patient survival rate is from about 0% to 7%.

In one other embodiment, the invention relates to the use according as defined above, wherein said prognosis is such that
■ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
■ when said tumor is BAS :
   o if none of the genes of subset A1 is expressed, the patient survival rate is about 19% to about 65%
   o if at least one of the genes of subsets A1 is expressed, the patient survival rate is about 0% to about 33%,
■ when said tumor is SQC :
   o if none of the genes of the subset C1 is expressed, the patient survival rate is about 58% to about 88%
   o if at least one of the genes of the subset C1 is expressed, the patient survival rate is from about 33% to about 64%,
■ when said tumor is LCNE :
   o if none of the genes of the subset D1 is expressed, the patient survival rate is from about 40% to about 65%
   o if at least one of the genes of the subset D1 is expressed, the patient survival rate is from about 0% to 7%.

In one other embodiment, the invention relates to the use according as defined above, wherein said prognosis is such that
■ when said tumor is ADK :
   o if none of the proteins of the subset BP1 is expressed, the patient survival rate is from about 76% to about 86%
   o if at least one of the subset BP1 is expressed, the patient survival rate is from about 39% to about 63%,
■ when said tumor is BAS :
   o if none of the proteins of subsets AP1 is expressed, the patient survival rate is about 19% to about 65%
   o if at least one of the proteins of the subsets AP1 or is expressed, the patient survival rate is about 0% to about 33%,
■ when said tumor is SQC :
   o if none of the proteins of the subset CP1 is expressed, the patient survival rate is about 58% to about 88%
   o if at least one of the proteins of the subset CP1 is expressed, the patient survival rate is from about 33% to about 64%,
■ when said tumor is LCNE :
   o if none of the proteins of the subset DP1 is expressed, the patient survival rate is from about 40% to about 65%
   o if at least one of the proteins of the subset DP1 is expressed, the patient survival rate is from about 0% to 7%,

In one other embodiment, the invention relates to the use according as defined above, wherein said prognosis is such that
■ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
      or
   o if none of the proteins of the subset BP1 is expressed, the patient survival rate is from about 76% to about 86%
   o if at least one of the subset BP1 is expressed, the patient survival rate is from about 39% to about 63%,
      or
   o if none of antibodies directed against the proteins of the subset BP1 is expressed, the patient survival rate is from about 76% to about 86%
   o if at least one antibody directed against at least one of the proteins of the subset BP1 is expressed, the patient survival rate is from about 39% to about 63%,
■ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
      or
   o if none of the proteins of subsets AP2 is expressed, the patient survival rate is about 19% to about 65%
   o if at least one of the proteins of the subsets AP2 or is expressed, the patient survival rate is about 0% to about 33%,
      or
   o if none of antibodies directed against the proteins of subset AP2 is expressed, the patient survival rate is about 19% to about 65%
   o if at least one antibody directed against at least one of the proteins of the subset AP2 is expressed, the patient survival rate is about 0% to about 33%,
■ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
      or
   o if none of the proteins of the subset CP2 is expressed, the patient survival rate is about 58% to about 88%
   o if at least one of the proteins of the subset CP2 is expressed, the patient survival rate is from about 33% to about 64%,
      or
   o if none of antibodies directed against the proteins of the subsets CP2 is expressed, the patient survival rate is about 58% to about 88%
   o if at least one antibody directed against at least one of the proteins of the subset CP2 is expressed, the patient survival rate is from about 33% to about 64%,
■ when said tumor is LCNE :
   o if none of the genes of the subset D2 is expressed, the patient survival rate is from about 40% to about 65%
   o if at least one of the genes of the subset D2 is expressed, the patient survival rate is from about 0% to 7%,
      or
   o if none of the proteins of the subset DP2 is expressed, the patient survival rate is from about 40% to about 65%
   o if at least one of the proteins of the subset DP2 is expressed, the patient survival rate is from about 0% to 7%,
      or
   o if none of antibodies directed against the proteins of the subset DP2 is expressed, the patient survival rate is from about 40% to about 65%,
   o if at least one antibody directed against at least one of the proteins of the the subset DP2 is expressed, the patient survival rate is from about 0% to 7%.

In one other embodiment, the invention relates to the use according as defined above, wherein said prognosis is such that
■ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
■ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
■ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
■ when said tumor is LCNE :
   o if none of the genes of the subset D2 is expressed, the patient survival rate is from about 40% to about 65%
   o if at least one of the genes of the subset D2 is expressed, the patient survival rate is from about 0% to 7%.

In one other embodiment, the invention relates to the use according as defined above, wherein said prognosis is such that
■ when said tumor is ADK :
   o if none of the proteins of the subset BP1 is expressed, the patient survival rate is from about 76% to about 86%
   o if at least one of the subset BP1 is expressed, the patient survival rate is from about 39% to about 63%,
■ when said tumor is BAS :
   o if none of the proteins of subsets AP2 is expressed, the patient survival rate is about 19% to about 65%
   o if at least one of the proteins of the subsets AP2 or is expressed, the patient survival rate is about 0% to about 33%,
■ when said tumor is SQC :
   o if none of the proteins of the subset CP2 is expressed, the patient survival rate is about 58% to about 88%
   o if at least one of the proteins of the subset CP2 is expressed, the patient survival rate is from about 33% to about 64%,
■ when said tumor is LCNE :
   o if none of the proteins of the subset DP2 is expressed, the patient survival rate is from about 40% to about 65%
   o if at least one of the proteins of the subset DP2 is expressed, the patient survival rate is from about 0% to 7%.

In one other embodiment, the invention relates to the use according as defined above, wherein said prognosis is such that
■ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
      or
   o if none of the proteins of the subset BP1 is expressed, the patient survival rate is from about 76% to about 86%
   o if at least one of the subset BP1 is expressed, the patient survival rate is from about 39% to about 63%,
      or
   o if none of antibodies directed against the proteins of the subset BP1 is expressed, the patient survival rate is from about 76% to about 86%
   o if at least one antibody directed against at least one of the proteins of the subset BP1 is expressed, the patient survival rate is from about 39% to about 63%,
■ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
      or
   o if none of the proteins of subsets AP2 is expressed, the patient survival rate is about 19% to about 65%
   o if at least one of the proteins of the subsets AP2 or is expressed, the patient survival rate is about 0% to about 33%,
      or
   o if none of antibodies directed against the proteins of subset AP21 is expressed, the patient survival rate is about 19% to about 65%
   o if at least one antibody directed against at least one of the proteins of the subset AP2 is expressed, the patient survival rate is about 0% to about 33%,
■ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
      or
   o if none of the proteins of the subset CP2 is expressed, the patient survival rate is about 58% to about 88%
   o if at least one of the proteins of the subset CP2 is expressed, the patient survival rate is from about 33% to about 64%,
      or
   o if none of antibodies directed against the proteins of the subsets CP2 is expressed, the patient survival rate is about 58% to about 88%
   o if at least one antibody directed against at least one of the proteins of the subset CP2 is expressed, the patient survival rate is from about 33% to about 64%,
■ when said tumor is LCNE :
   o if none of the genes of the subset D3 is expressed, the patient survival rate is from about 40% to about 65%
   o if at least one of the genes of the subset D3 is expressed, the patient survival rate is from about 0% to 7%,
      or
   o if none of the proteins of the subset DP3 is expressed, the patient survival rate is from about 40% to about 65%
   o if at least one of the proteins of the subset DP3 is expressed, the patient survival rate is from about 0% to 7%,
      or
   o if none of antibodies directed against the proteins of the subset DP3 is expressed, the patient survival rate is from about 40% to about 65%,
   o if at least one antibody directed against at least one of the proteins of the the subset DP3 is expressed, the patient survival rate is from about 0% to 7%.

In one other embodiment, the invention relates to the use according as defined above, wherein said prognosis is such that
■ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
■ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
■ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
■ when said tumor is LCNE :
   o if none of the genes of the subset D3 is expressed, the patient survival rate is from about 40% to about 65%
   o if at least one of the genes of the subset D3 is expressed, the patient survival rate is from about 0% to 7%.

In one other embodiment, the invention relates to the use according as defined above, wherein said prognosis is such that
■ when said tumor is ADK :
   o if none of the proteins of the subset BP1 is expressed, the patient survival rate is from about 76% to about 86%
   o if at least one of the subset BP1 is expressed, the patient survival rate is from about 39% to about 63%,
■ when said tumor is BAS :
   o if none of the proteins of subsets AP2 is expressed, the patient survival rate is about 19% to about 65%
   o if at least one of the proteins of the subsets AP2 or is expressed, the patient survival rate is about 0% to about 33%,
■ when said tumor is SQC :
   o if none of the proteins of the subset CP2 is expressed, the patient survival rate is about 58% to about 88%
   o if at least one of the proteins of the subset CP2 is expressed, the patient survival rate is from about 33% to about 64%,
■ when said tumor is LCNE :
   o if none of the proteins of the subset DP3 is expressed, the patient survival rate is from about 40% to about 65%
   o if at least one of the proteins of the subset DP3 is expressed, the patient survival rate is from about 0% to 7%.

In one particular embodiment, the invention relates to the use as defined above, wherein
■ when said tumor is SQC :
   o if none of the genes of said set C or of subsets C1 and C2 is expressed, the patient survival rate is about 58% to about 88%
   o if one of the genes of the set C or of subsets C1 and C2 is expressed, the patient survival rate is from about 45% to about 73%,
   o if at least two of the genes of the set C or of subsets C1 and C2 are expressed, the patient survival rate is from about 14%, to about 50%,
      or
   o if none of the proteins of said set CP or of subsets CP1 and CP2 is expressed, the patient survival rate is about 58% to about 88%
   o if one of the proteins of the set CP or of subsets CP1 and CP2 is expressed, the patient survival rate is from about 45% to about 73%,
   o if at least two of the proteins of the set CP or of subsets CP1 and CP2 are expressed, the patient survival rate is from about 14%, to about 50%,
      or
   o if none of the antibodies directed against proteins said set CP or of subsets CP1 and CP2 is expressed, the patient survival rate is about 58% to about 88%,
   o if one of antibody directed agains one protein of the set CP or of subsets CP1 and CP2 is expressed, the patient survival rate is from about 45% to about 73%,
   o if antibibodies directed against at least two proteins of the set CP or of subsets CP1 and CP2 are expressed, the patient survival rate is from about 14%, to about 50%.

In one particular embodiment, the invention relates to the use as defined above, wherein
■ when said tumor is SQC :
   o if none of the genes of said set C or of subsets C1 and C2 is expressed, the patient survival rate is about 58% to about 88%
   o if one of the genes of the set C or of subsets C1 and C2 is expressed, the patient survival rate is from about 45% to about 73%,
   o if at least two of the genes of the set C or of subsets C1 and C2 are expressed, the patient survival rate is from about 14%, to about 50%,

In one particular embodiment, the invention relates to the use as defined above, wherein
■ when said tumor is SQC :
   o if none of the genes of said subset is expressed, the patient survival rate is about 58% to about 88%
   o if one of the genes of the subset C1 is expressed, the patient survival rate is from about 45% to about 73%,
   o if at least two of the genes of the subset C1 are expressed, the patient survival rate is from about 14%, to about 50%.

In one particular embodiment, the invention relates to the use as defined above, wherein
■ when said tumor is SQC :
   o if none of the genes of said subset is expressed, the patient survival rate is about 58% to about 88%
   o if one of the genes of the subset C2 is expressed, the patient survival rate is from about 45% to about 73%,
   o if at least two of the genes of the subset C2 are expressed, the patient survival rate is from about 14%, to about 50%.

In one particular embodiment, the invention relates to the use as defined above, wherein
■ when said tumor is SQC :
   o if none of the proteins of said set CP or of subsets CP1 and CP2 is expressed, the patient survival rate is about 58% to about 88%
   o if one of the proteins of the set CP or of subsets CP1 and CP2 is expressed, the patient survival rate is from about 45% to about 73%,
   o if at least two of the proteins of the set CP or of subsets CP1 and CP2 are expressed, the patient survival rate is from about 14%, to about 50%.

In one particular embodiment, the invention relates to the use as defined above, wherein
■ when said tumor is SQC :
   o if none of the proteins of said subset CP1 is expressed, the patient survival rate is about 58% to about 88%
   o if one of the proteins of the subset CP1 is expressed, the patient survival rate is from about 45% to about 73%,
   o if at least two of the proteins of the subset CP1 are expressed, the patient survival rate is from about 14%, to about 50%.

In one particular embodiment, the invention relates to the use as defined above, wherein
■ when said tumor is SQC :
   o if none of the proteins of said subset CP2 is expressed, the patient survival rate is about 58% to about 88%
   o if one of the proteins of the subset CP2 is expressed, the patient survival rate is from about 45% to about 73%,
   o if at least two of the proteins of the subset CP2 are expressed, the patient survival rate is from about 14%, to about 50%.

In another advantageaous embodiment, the invention relates to the use as defined above, wherein during a period of time of 120 months from the diagnosis of said lung cancer, wherein
■ when said tumor is ADK :
   o if none of the genes of said set B or of the subset B1 is expressed, the patient survival rate is about 76%
   o if at least one of the genes of the set B or of the subset B1 is expressed, the patient survival rate is about 39%,
      or
   o if none of the proteins of said set BP or of the subset BP1 is expressed, the patient survival rate is about 76% ,
   o if at least one of the proteins of the set BP or of the subset BP1 is expressed, the patient survival rate is about 39%,
      or
   o if none of antibodies directed against the proteins of said set BP or of the subset BP1 is expressed, the patient survival rate is about 76% ,
   o if at least one antibody directed against at least one of the proteins of the set BP or of the subset BP1 is expressed, the patient survival rate is about 39%,
■ when said tumor is BAS :
   o if none of the genes of the set A or of subsets A1 or A2 is expressed, the patient survival rate is about 19%
   o if at least one of the genes of the set A or of subsets A1 or A2 is expressed, the patient survival rate is about 0%,
      or
   o if none of the proteins of the set AP or of subsets AP1 or AP2 is expressed, the patient survival rate is about 19%
   o if at least one of the proteins of the set AP or of subsets AP1 or AP2 is expressed, the patient survival rate is about 0%,
      or
   o if none of antibodies directed against the proteins of said set AP or of subsets AP1 or AP2 is expressed, the patient survival rate is about 19%
   o if at least one antibody directed against at least one of the proteins of the set AP or of subsets AP1 or AP2 is expressed, the patient survival rate is about 0%,
■ when said tumor is SQC :
   o if none of the genes of the set C or of the subsets C1 or C2 is expressed, the patient survival rate is about 58%
   o if at least one of the genes of the set C or of the subsets C1 or C2 is expressed, the patient survival rate is about 33%,
      or
   o if none of the proteins of the set CP or of the subsets CP1 or CP2 is expressed, the patient survival rate is about 58%
   o if at least one of the proteins of the set CP or of the subsets CP1 or CP2 is expressed, the patient survival rate is about 33%, or
   o if none of antibodies directed against the proteins of said set CP or of the subsets CP1 or CP2 is expressed, the patient survival rate is about 58%
   o if at least one antibody directed against at least one of the proteins of the set CP or of the subsets CP1 or CP2 is expressed, the patient survival rate is about 33%,
■ when said tumor is LCNE :
   o if none of the genes of the set D or of the subsets D1, D2 or D3 is expressed, the patient survival rate is about 40%
   o if at least one of the genes of the set D or of the subsets D1, D2 or D3 is expressed, the patient survival rate is about 0%,
      or
   o if none of the proteins of the set DP or of the subsets DP1, DP2 or DP3 is expressed, the patient survival rate is about 40%
   o if at least one of the proteins of the set DP or of the subsets DP1, DP2 or DP3 is expressed, the patient survival rate is about 0%,
      or
   o if none of antibodies directed against the proteins of said set DP or of the subsets DP1, DP2 or DP3 is expressed, the patient survival rate is about 40% ,
   o if at least one antibody directed against at least one of the proteins of the set DP or of the subsets DP1, DP2 or DP3 is expressed, the patient survival rate is about 0%.

In one particular embodiment, the invention relates to the use as defined above, où
■ when said tumor is SQC :
   o if none of the genes of the set C or of the subsets C1 or C2 is expressed, the patient survival rate is about 58%
   o if one of the genes the set C or of the subsets C1 or C2 is expressed, the patient survival rate is about 45%,
   o if at least two of the genes of the set C or of the subsets C1 or C2 is expressed, the patient survival rate is about 14%,
      or
   o if none of the protein of the set CP or of the subsets CP1 or CP2 is expressed, the patient survival rate is about 58%
   o if one of the genes the set C or of the subsets CP1 or CP2 is expressed, the patient survival rate is about 45%,
   o if at least two of the genes of the set C or of the subsets CP1 or CP2 is expressed, the patient survival rate is about 14%,
      or
   o if none of antibodies directed against the proteins of said set CP or of the subsets CP1 or CP2 is expressed, the patient survival rate is about 58%
   o if one antibody directed against at least one of the proteins of the set CP or of the subsets CP1, CP2 or CP3, the patient survival rate is about 45%,
   o if at least two antibody directed against at least one of the proteins of the set CP or of the subsets CP1, CP2 or CP3, the patient survival rate is about 14%.

In another advantageous embodiment, the invention relates to the use as defined above, wherein during a period of time of 60 months from the diagnosis of said lung cancer, wherein
■ when said tumor is ADK :
   o if none of the genes of said set B or of the subset B1 is expressed, the patient survival rate is about 82%
   o if at least one of the genes of the set B or of the subset B1 is expressed, the patient survival rate is about 46%,
      or
   o if none of the proteins of said set BP or of the subset BP1 is expressed, the patient survival rate is about 82% ,
   o if at least one of the proteins of the set BP or of the subset BP1 is expressed, the patient survival rate is about 46%,
      or
   o if none of antibodies directed against the proteins of said set BP or of the subset BP1 is expressed, the patient survival rate is about 82%,
   o if at least one antibody directed against at least one of the proteins of the set BP or of the subset BP1 is expressed, the patient survival rate is about 46%,
■ when said tumor is BAS :
   o if none of the genes of the set A or of subsets A1 or A2 is expressed, the patient survival rate is about 35%
   o if at least one of the genes of the set A or of subsets A1 or A2 is expressed, the patient survival rate is about 7%,
      or
   o if none of the proteins of the set AP or of subsets AP1 or AP2 is expressed, the patient survival rate is about 35%
   o if at least one of the proteins of the set AP or of subsets AP1 or AP2 is expressed, the patient survival rate is about 7%,
      or
   o if none of antibodies directed against the proteins of said set AP or of subsets AP1 or AP2 is expressed, the patient survival rate is about 35%
   o if at least one antibody directed against at least one of the proteins of the set AP or of subsets AP1 or AP2 is expressed, the patient survival rate is about 7%,
■ when said tumor is SQC :
   o if none of the genes of the set C or of the subsets C1 or C2 is expressed, the patient survival rate is about 77%
   o if at least one of the genes of the set C or of the subsets C1 or C2 is expressed, the patient survival rate is about 42%,
      or
   o if none of the proteins of the set CP or of the subsets CP1 or CP2 is expressed, the patient survival rate is about 77%
   o if at least one of the proteins of the set CP or of the subsets CP1 or CP2 is expressed, the patient survival rate is about 42%, or
   o if none of antibodies directed against the proteins of said set CP or of the subsets CP1 or CP2 is expressed, the patient survival rate is about 77%
   o if at least one antibody directed against at least one of the proteins of the set CP or of the subsets CP1 or CP2 is expressed, the patient survival rate is about 42%,
■ when said tumor is LCNE :
   o if none of the genes of the set D or of the subsets D1, D2 or D3 is expressed, the patient survival rate is about 55%
   o if at least one of the genes of the set D or of the subsets D1, D2 or D3 is expressed, the patient survival rate is about 4%,
      or
   o if none of the proteins of the set DP or of the subsets DP1, DP2 or DP3 is expressed, the patient survival rate is about 55%
   o if at least one of the proteins of the set DP or of the subsets DP1, DP2 or DP3 is expressed, the patient survival rate is about 4%,
      or
   o if none of antibodies directed against the proteins of said set DP or of the subsets DP1, DP2 or DP3 is expressed, the patient survival rate is about 55% ,
   o if at least one antibody directed against at least one of the proteins of the set DP or of the subsets DP1, DP2 or DP3 is expressed, the patient survival rate is about 4%.

In one particular embodiment, the invention relates to the use as defined above, où
■ when said tumor is SQC :
   o if none of the genes of the set C or of the subsets C1 or C2 is expressed, the patient survival rate is about 77%
   o if one of the genes the set C or of the subsets C1 or C2 is expressed, the patient survival rate is about 59%,
   o if at least two of the genes of the set C or of the subsets C1 or C2 is expressed, the patient survival rate is about 14%,
      or
   o if none of the protein of the set CP or of the subsets CP1 or CP2 is expressed, the patient survival rate is about 77%
   o if one of the genes the set C or of the subsets CP1 or CP2 is expressed, the patient survival rate is about 59%,
   o if at least two of the genes of the set C or of the subsets CP1 or CP2 is expressed, the patient survival rate is about 14%,
      or
   o if none of antibodies directed against the proteins of said set CP or of the subsets CP1 or CP2 is expressed, the patient survival rate is about 77%
   o if one antibody directed against at least one of the proteins of the set CP or of the subsets CP1 or CP2, the patient survival rate is about 59%,
   o if at least two antibody directed against at least one of the proteins of the set CP or of the subsets CP1 or CP2, the patient survival rate is about 14%.

In another advantageous embodiment, the invention relates to the use as defined above, wherein during a period of time of 30 months from the diagnosis of said lung cancer, wherein
■ when said tumor is ADK :
   o if none of the genes of said set B or of the subset B1 is expressed, the patient survival rate is about 86%
   o if at least one of the genes of the set B or of the subset B1 is expressed, the patient survival rate is about 63%,
      or
   o if none of the proteins of said set BP or of the subset BP1 is expressed, the patient survival rate is about 86% ,
   o if at least one of the proteins of the set BP or of the subset BP1 is expressed, the patient survival rate is about 63%,
      or
   o if none of antibodies directed against the proteins of said set BP or of the subset BP1 is expressed, the patient survival rate is about 86% ,
   o if at least one antibody directed against at least one of the proteins of the set BP or of the subset BP1 is expressed, the patient survival rate is about 63%,
■ when said tumor is BAS :
   o if none of the genes of the set A or of subsets A1 or A2 is expressed, the patient survival rate is about 65%
   o if at least one of the genes of the set A or of subsets A1 or A2 is expressed, the patient survival rate is about 33%,
      or
   o if none of the proteins of the set AP or of subsets AP1 or AP2 is expressed, the patient survival rate is about 63%
   o if at least one of the proteins of the set AP or of subsets AP1 or AP2 is expressed, the patient survival rate is about 33%,
      or
   o if none of antibodies directed against the proteins of said set AP or of subsets AP1 or AP2 is expressed, the patient survival rate is about 65%
   o if at least one antibody directed against at least one of the proteins of the set AP or of subsets AP1 or AP2 is expressed, the patient survival rate is about 33%,
■ when said tumor is SQC :
   o if none of the genes of the set C or of the subsets C1 or C2 is expressed, the patient survival rate is about 88%
   o if at least one of the genes of the set C or of the subsets C1 or C2 is expressed, the patient survival rate is about 64%,
      or
   o if none of the proteins of the set CP or of the subsets CP1 or CP2 is expressed, the patient survival rate is about 88%
   o if at least one of the proteins of the set CP or of the subsets CP1 or CP2 is expressed, the patient survival rate is about 64%,
      or
   o if none of antibodies directed against the proteins of said set CP or of the subsets CP1 or CP2 is expressed, the patient survival rate is about 88%
   o if at least one antibody directed against at least one of the proteins of the set CP or of the subsets CP1 or CP2 is expressed, the patient survival rate is about 64%,
■ when said tumor is LCNE :
   o if none of the genes of the set D or of the subsets D1, D2 or D3 is expressed, the patient survival rate is about 65%
   o if at least one of the genes of the set D or of the subsets D1, D2 or D3 is expressed, the patient survival rate is about 7%,
      or
   o if none of the proteins of the set DP or of the subsets DP1, DP2 or DP3 is expressed, the patient survival rate is about 65%
   o if at least one of the proteins of the set DP or of the subsets DP1, DP2 or DP3 is expressed, the patient survival rate is about 7%,
      or
   o if none of antibodies directed against the proteins of said set DP or of the subsets DP1, DP2 or DP3 is expressed, the patient survival rate is about 65% ,
   o if at least one antibody directed against at least one of the proteins of the set DP or of the subsets DP1, DP2 or DP3 is expressed, the patient survival rate is about 7%.

In one particular embodiment, the invention relates to the use as defined above, où
■ when said tumor is SQC :
   o if none of the genes of the set C or of the subsets C1 or C2 is expressed, the patient survival rate is about 88%
   o if one of the genes the set C or of the subsets C 1 or C2 is expressed, the patient survival rate is about 73%,
   o if at least two of the genes of the set C or of the subsets C 1 or C2 is expressed, the patient survival rate is about 50%,
      or
   o if none of the protein of the set CP or of the subsets CP1 or CP2 is expressed, the patient survival rate is about 88%
   o if one of the genes the set C or of the subsets CP1 or CP2 is expressed, the patient survival rate is about 73%,
   o if at least two of the genes of the set C or of the subsets CP1 or CP2 is expressed, the patient survival rate is about 50%,
      or
   o if none of antibodies directed against the proteins of said set CP or of the subsets CP1 or CP2 is expressed, the patient survival rate is about 88%
   o if one antibody directed against at least one of the proteins of the set CP or of the subsets CP1 or CP2, the patient survival rate is about 73%,
   o if at least two antibody directed against at least one of the proteins of the set CP or of the subsets CP1 or CP2, the patient survival rate is about 50%.

The invention also relates to a prognosis method, preferably *in vitro,* of the survival rate of a patient afflicted by a lung tumour, from a biological sample containing said lung tumor, at a time from 30 to 120 months after the diagnosis of said lung cancer, as defined above, said lung cancer being previously classified as a lung tumour belonging from the group consisting of ADK, SQC, BAS et LCNE,
said method comprising a step of measuring, in said biological sample, the expression of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
   a. at least one gene belongs to a first set A of 4 genes comprising or consisting of the nucleic acid sequences SEQ ID NO: 1 to 4, or a subset A1 as defined above,
   b. at least one gene belongs to a second set B of 3 genes comprising or consisting of the nucleic acid sequences SEQ ID NO: 5 to 7, or a subset B1 or B2 as defined above
   c. at least one gene belongs to a third set C of 6 genes comprising or consisting of the nucleic acid sequences SEQ ID NO: 8 to 13 , or a subset C1 or C2 as defined above, and
   d. at least one gene belongs to a fourth set D of 9 genes comprising or consisting of the nucleic acid sequences SEQ ID NO: 14-23, or a subset D1, D2 or D3 as defined above,
◆ at least 4 proteins being such that
   a. at least one protein belongs to a first set AP of 4 proteins comprising or consisting of the amino acid sequences SEQ ID NO: 24-27, or a subset AP1 as defined above
   b. at least one protein belongs to a second set BP of 3 proteins comprising or consisting of the amino acid sequences SEQ ID NO: 28-30, or a subset BP1 or BP2 as defined above
   c. at least one protein belongs to a second set CP of 6 proteins comprising or consisting of the amino acid sequences SEQ ID NO: 31-36, or a subset CP1 or CP2 as defined above,
   d. at least one protein belongs to a second set BP of 9 proteins comprising or consisting of the amino acid sequences SEQ ID NO: 37-46, or a subset DP1, DP2 or DP3 as defined above,
◆ at least 4 antibodies directed against said 4 proteins being such that
   a. at least one antibody that ineract with at least one protein that belongs to a first set AP of 4 proteins comprising or consisting of the amino acid sequences SEQ ID NO: 24-27, or a subset AP1 as defined above
   b. at least one antibody that ineract with at least one protein that belongs a second set BP of 3 proteins comprising or consisting of the amino acid sequences SEQ ID NO: 28-30, or a subset BP1 or BP2 as defined above
   c. at least one antibody that ineract with at least one protein that belongs a third set CP of 6 proteins comprising or consisting of the amino acid sequences SEQ ID NO: 31-36, or a subset CP1 or CP2 as defined above,
   d. at least one antibody that ineract with at least one protein that belongs a fourth set BP of 9 proteins comprising or consisting of the amino acid sequences SEQ ID NO: 37-46, or a subset DP1, DP2 or DP3 as defined above,
wherein
- the nucleic acid sequences SEQ ID NO: 1 to 4 and/or the amino acid sequences SEQ ID NO: 24 to 27 are expressed in BAS,
- the nucleic acid sequences SEQ ID NO: 5 to 7 and/or the amino acid sequences SEQ ID NO: 28 to 30 are expressed in ADK,
- the nucleic acid sequences SEQ ID NO: 8 to 13 and/or the amino acid sequences SEQ ID NO: 31 to 36 are expressed in SQC,
- the nucleic acid sequences SEQ ID NO: 14 to 23 and/or the amino acid sequences SEQ ID NO: 37 to 46 are expressed in LCNE,
said prognosis being such that:
either
   ■ if none of the genes of said set A, B, C and D is expressed, the patient survival rate during a period of time from 30 to 120 months after the diagnosis of said lung cancer is from about 59% to about 78% or more, and
   ■ if at least one gene of at least one set A, B, C or D is expressed , the patient survival rate during a period of time from 30 to 120 months after the diagnosis of said lung cancer is about from about 3% to about 70%,
or
   ■ if none of the proteins of said set AP, BP, CP and DP is expressed, the patient survival rate during a period of time from 30 to 120 months after the diagnosis of said lung cancer is from about 59% to about 78% or more, and
   ■ if at least one protein of at least one set AP, BP, CP and DP is expressed, the patient survival rate during a period of time from 30 to 120 months after the diagnosis of said lung cancer is about from about 3% to about 70%,
or
   ■ if none of the antibodies directed against the proteins of said set AP, BP, CP and DP is expressed, the patient survival rate during a period of time from 30 to 120 months after the diagnosis of said lung cancer is from about 59% to about 78% or more, and
   ■ if at least one antibody directed against one protein of said set AP, BP, CP and DP is expressed, the patient survival rate during a period of time from 30 to 120 months after the diagnosis of said lung cancer is about from about 3% to about 70%.

The invention also relates to a prognosis method, preferably *in vitro,* of the survival rate of a patient afflicted by a lung tumour, from a biological sample containing said lung tumor, at a time from 30 to 120 months after the diagnosis of said lung cancer, as defined above,
said method comprising a step of measuring, in said biological sample, the expression of
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
   said
   ◆ at least 4 genes being such that
      a. at least one gene belongs to a first set A of 4 genes comprising or consisting of the nucleic acid sequences SEQ ID NO: 1 to 4, or a subset A1 as defined above,
      b. at least one gene belongs to a second set B of 3 genes comprising or consisting of the nucleic acid sequences SEQ ID NO: 5 to 7, or a subset B1 or B2 as defined above
      c. at least one gene belongs to a third set C of 6 genes comprising or consisting of the nucleic acid sequences SEQ ID NO: 8 to 13 , or a subset C1 or C2 as defined above, and
      d. at least one gene belongs to a fourth set D of 9 genes comprising or consisting of the nucleic acid sequences SEQ ID NO: 14-23, or a subset D1, D2 or D3 as defined above,
wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
■ if none of the genes of said set A, B, C and D is expressed, the patient survival rate during a period of time from 30 to 120 months after the diagnosis of said lung cancer is from about 59% to about 78% or more, and
■ if at least one gene of at least one set A, B, C or D is expressed , the patient survival rate during a period of time from 30 to 120 months after the diagnosis of said lung cancer is about from about 3% to about 70%.

In another advantageous embodiment, the invention relates to a prognosis method previously defined, wherein the step of measuring is carried out by using a technique chosen among the set consisting of:
a. Quantitative PCR,
b. DNA CHIP, and
c. Northern blot.

In another advantageous embodiment, the invention relates to a prognosis method as previously defined, wherein the step of measuring is carried out by using nucleic acid molecules consisting of from 15 to 100 nucleotides molecules being complementary to said at least 4 genes.

In one advantageous embodiment, the invention relates to a prognosis method as defined above, wherein the step of measuring is carried out by DNA CHIP using
- at least one nucleic acid probe comprising or being constituted by the nucleic acid sequence SEQ ID NO: 47 to SEQ ID NO: 50,
- at least one nucleic acid probe comprising or being constituted by the nucleic acid sequence SEQ ID NO: 51 to SEQ ID NO: 53,
- at least one nucleic acid probe comprising or being constituted by the nucleic acid sequence SEQ ID NO: 54 to SEQ ID NO: 59, and
- at least one nucleic acid probe comprising or being constituted by the nucleic acid sequence SEQ ID NO: 60 to SEQ ID NO: 69.

In another advantageous embodiment, the invention relates to the method as defined above, wherein the step of measuring is carried out by DNA CHIP using at least 2, preferably at least 3 nucleic acid probe as defined above.

An advantageous embodiment of the invention relates to the above method wherein the nucleic acid probes comprising or being constituted by the nucleic acid sequences SEQ ID NO: 47 to 69 are used, together.

In the invention the correspondence between the genes and the nucleic acid probes are as follows: SEQ ID NO:1 is able to be detected by the nucleic acid probe comprising or being constituted by the nucleic acid sequence SEQ ID NO: 47, SEQ ID NO:2 is able to be detected by the nucleic acid probe comprising or being constituted by the nucleic acid sequence SEQ ID NO: 48, SEQ ID NO:3 is able to be detected by the nucleic acid probe comprising or being constituted by the nucleic acid sequence SEQ ID NO: 49, SEQ ID NO:4 is able to be detected by the nucleic acid probe comprising or being constituted by the nucleic acid sequence SEQ ID NO: 50, SEQ ID NO:5 is able to be detected by the nucleic acid probe comprising or being constituted by the nucleic acid sequence SEQ ID NO: 51, SEQ ID NO:6 is able to be detected by the nucleic acid probe comprising or being constituted by the nucleic acid sequence SEQ ID NO: 52, SEQ ID NO:7 is able to be detected by the nucleic acid probe comprising or being constituted by the nucleic acid sequence SEQ ID NO: 53, SEQ ID NO:8 is able to be detected by the nucleic acid probe comprising or being constituted by the nucleic acid sequence SEQ ID NO: 54, SEQ ID NO:9 is able to be detected by the nucleic acid probe comprising or being constituted by the nucleic acid sequence SEQ ID NO: 55, SEQ ID NO:10 is able to be detected by the nucleic acid probe comprising or being constituted by the nucleic acid sequence SEQ ID NO: 56, SEQ ID NO:11 is able to be detected by the nucleic acid probe comprising or being constituted by the nucleic acid sequence SEQ ID NO: 57, SEQ ID NO:12 is able to be detected by the nucleic acid probe comprising or being constituted by the nucleic acid sequence SEQ ID NO: 58, SEQ ID NO:13 is able to be detected by the nucleic acid probe comprising or being constituted by the nucleic acid sequence SEQ ID NO: 59, SEQ ID NO:14 is able to be detected by the nucleic acid probe comprising or being constituted by the nucleic acid sequence SEQ ID NO: 60, SEQ ID NO:15 is able to be detected by the nucleic acid probe comprising or being constituted by the nucleic acid sequence SEQ ID NO: 61, SEQ ID NO:16 is able to be detected by the nucleic acid probe comprising or being constituted by the nucleic acid sequence SEQ ID NO: 62, SEQ ID NO:17 is able to be detected by the nucleic acid probe comprising or being constituted by the nucleic acid sequence SEQ ID NO: 63, SEQ ID NO:18 is able to be detected by the nucleic acid probe comprising or being constituted by the nucleic acid sequence SEQ ID NO: 64, SEQ ID NO:19 is able to be detected by the nucleic acid probe comprising or being constituted by the nucleic acid sequence SEQ ID NO: 65, SEQ ID NO:20 is able to be detected by the nucleic acid probe comprising or being constituted by the nucleic acid sequence SEQ ID NO: 66, SEQ ID NO:21 is able to be detected by the nucleic acid probe comprising or being constituted by the nucleic acid sequence SEQ ID NO: 67, SEQ ID NO:22 is able to be detected by the nucleic acid probe comprising or being constituted by the nucleic acid sequence SEQ ID NO: 68, SEQ ID NO:23 is able to be detected by the nucleic acid probe comprising or being constituted by the nucleic acid sequence SEQ ID NO: 69.

The invention also relates to a prognosis method, preferably *in vitro,* of the survival rate of a patient afflicted by a lung tumour, from a biological sample containing said lung tumor, at a time from 30 to 120 months after the diagnosis of said lung cancer, said method comprising a step of measuring, in said biological sample, the expression of
- at least 4 proteins chosen among a set of 23 proteins coded by 23 genes comprising or consisting of the nucleic acid sequences SEQ ID NO 1 to 23,
- or fragments of said proteins,
said at least 4 proteins being such that
◆ at least 4 proteins being such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30,
   c. at least one protein belongs to a third set CP of 6 proteins comprising or consisting of the amino acid sequences SEQ ID NO: 31-36,
   d. at least one protein belongs to a fourth set BP of 9 proteins comprising or consisting of the amino acid sequences SEQ ID NO: 37-46,
wherein
- the amino acid sequences SEQ ID NO: 24 to 27 are expressed in BAS,
- the amino acid sequences SEQ ID NO: 28 to 30 are expressed in ADK,
- the amino acid sequences SEQ ID NO: 31 to 36 are expressed in SQC,
- the amino acid sequences SEQ ID NO: 37 to 46 are expressed in LCNE,
said prognosis method being such that
■ if none of the proteins is expressed, the patient survival rate is from about 59% to about 78%, or more, and
■ if at least one protein of said set AP, BP, CP and DP is expressed, the patient survival rate from about 3% to about 70%.

In one advantageous embodiment, the invention relates to the prognosis methodas defined above wherein the step of measuring is carried out by using a technique chosen among the set consisting of:
a. western Blot,
b. ELISA,
c. Immunofluorescence, and
d. Immunohistochemistry.

In one other advantageous embodiment, the invention relates to a prognosis method, as defined above, wherein the step of measuring is carried out by using antibodies directed against said at least 4 proteins coded by said at least two genes.

Another advantageaous embodiment of the invention relates to a prognosis method, as defined above, further comprising a step of comparison of said measured expression to the expression in at least one control sample.

The invention also concern a kit comprising a DNA CHIP comprising at least the nucleic acid probes comprising or being constituted by the nucleic acid sequences SEQ ID NO : 47 to 69.

### FIGURES

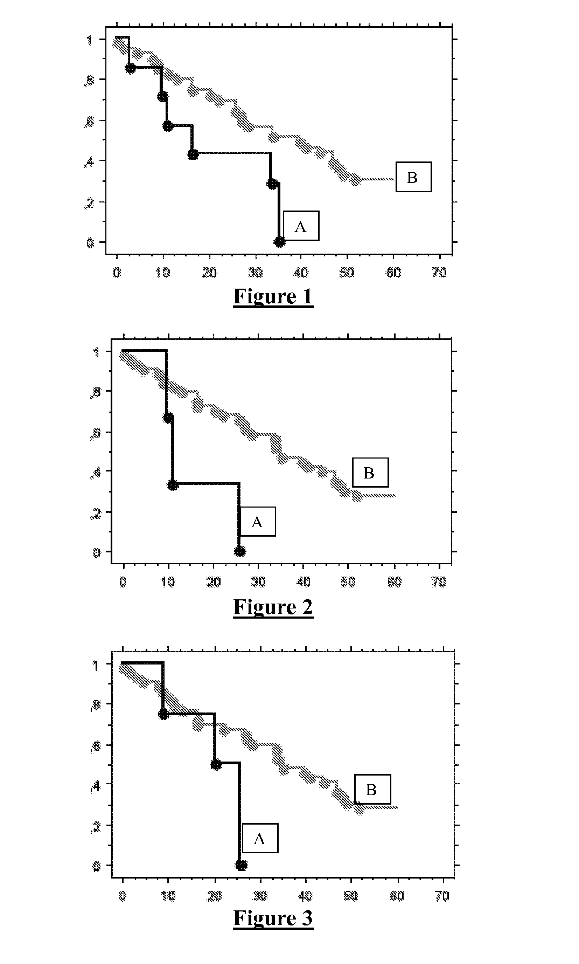

**Figure 1** represents the cumulative survival rate (Kaplan Mayer curve) over 60 months of patients expressing (A) or not (B) the gene SEQ ID NO: 4 (gene 391). Y- axis represents cumulative survival in %, X-axis represents time in months.
**Figure 2** represents the cumulative survival rate (Kaplan Mayer curve) over 60 months of patients expressing (A) or not (B) the gene SEQ ID NO: 3 (gene 417). Y- axis represents cumulative survival in %, X-axis represents time in months.
**Figure 3** represents the cumulative survival rate (Kaplan Mayer curve) over 60 months of patients expressing (A) or not (B) the gene SEQ ID NO: 2 (gene 1261). Y- axis represents cumulative survival in %, X-axis represents time in months.

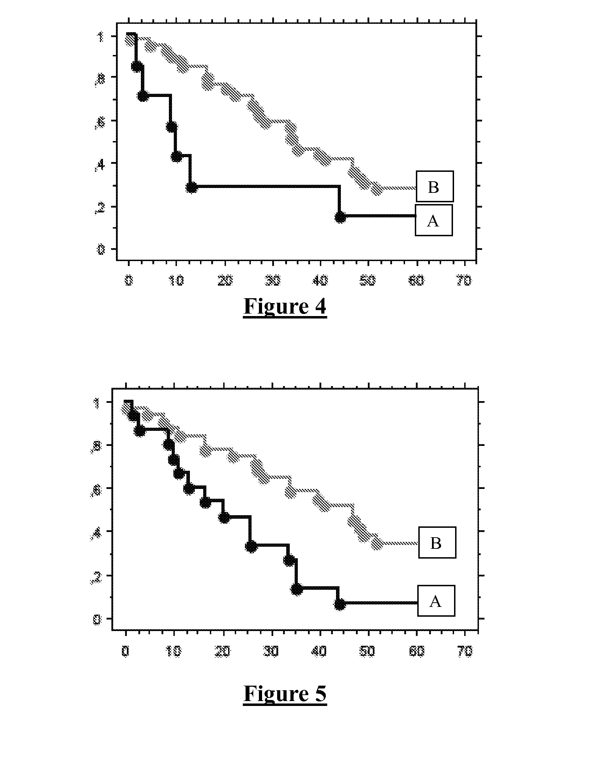

**Figure 4** represents the cumulative survival rate (Kaplan Mayer curve) over 60 months of patients expressing (A) or not (B) the following genes: SEQ ID NO: 1 (gene 1292). Y-axis represents cumulative survival in %, X-axis represents time in months.
**Figure 5** represents the cumulative survival rate (Kaplan Mayer curve) over 60 months of patients expressing (A) or not (B) at least one of the following genes: SEQ ID NO: 1 (gene 1292), SEQ ID NO: 2 (gene 1261), SEQ ID NO: 3 (gene 417) and SEQ ID NO: 4 (gene 391). Y- axis represents cumulative survival in %, X-axis represents time in months.

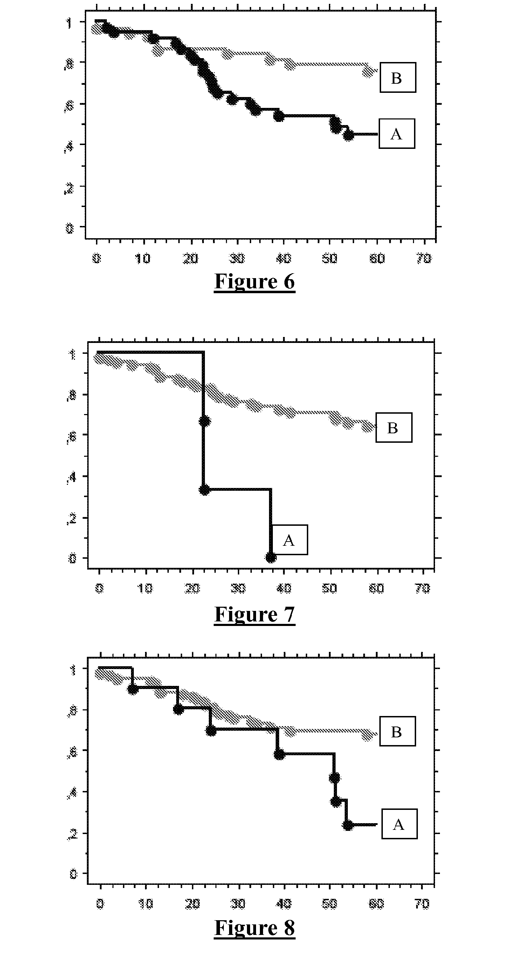

**Figure 6** represents the cumulative survival rate (Kaplan Mayer curve) over 60 months of patients expressing (A) or not (B) the following genes: SEQ ID NO: 7 (gene 59). Y- axis represents cumulative survival in %, X-axis represents time in months.
**Figure 7** represents the cumulative survival rate (Kaplan Mayer curve) over 60 months of patients expressing (A) or not (B) the following genes: SEQ ID NO: 6 (gene 222). Y-axis represents cumulative survival in %, X-axis represents time in months.
**Figure 8** represents the cumulative survival rate (Kaplan Mayer curve) over 60 months of patients expressing (A) or not (B) the following genes: SEQ ID NO: 5 (gene 1371). Y-axis represents cumulative survival in %, X-axis represents time in months.

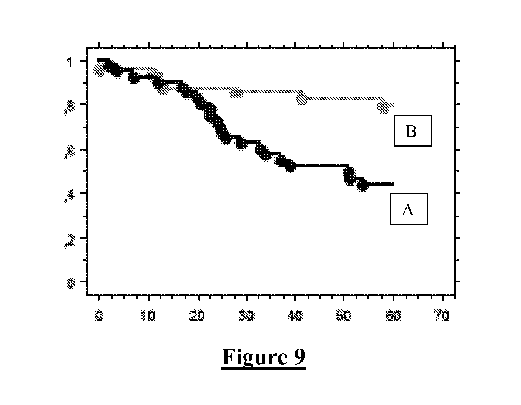

**Figure 9** represents the cumulative survival rate (Kaplan Mayer curve) over 60 months of patients expressing (A) or not (B) at least one of the following genes: SEQ ID NO: 5 (gene 1371), SEQ ID NO: 6 (gene 222) and SEQ ID NO: 7 (gene 59). Y- axis represents cumulative survival in %, X-axis represents time in months.

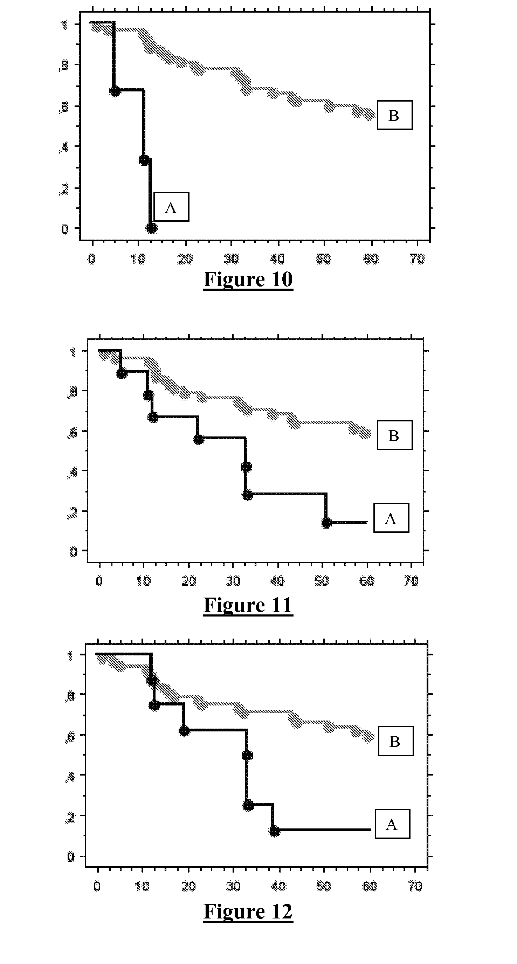

**Figure 10** represents the cumulative survival rate (Kaplan Mayer curve) over 60 months of patients expressing (A) or not (B) the gene SEQ ID NO: 12 (gene 440). Y- axis represents cumulative survival in %, X-axis represents time in months.
**Figure 11** represents the cumulative survival rate (Kaplan Mayer curve) over 60 months of patients expressing (A) or not (B) the gene SEQ ID NO: 9 (gene 442). Y- axis represents cumulative survival in %, X-axis represents time in months.
**Figure 12** represents the cumulative survival rate (Kaplan Mayer curve) over 60 months of patients expressing (A) or not (B) the gene SEQ ID NO: 8 (gene 266). Y- axis represents cumulative survival in %, X-axis represents time in months.

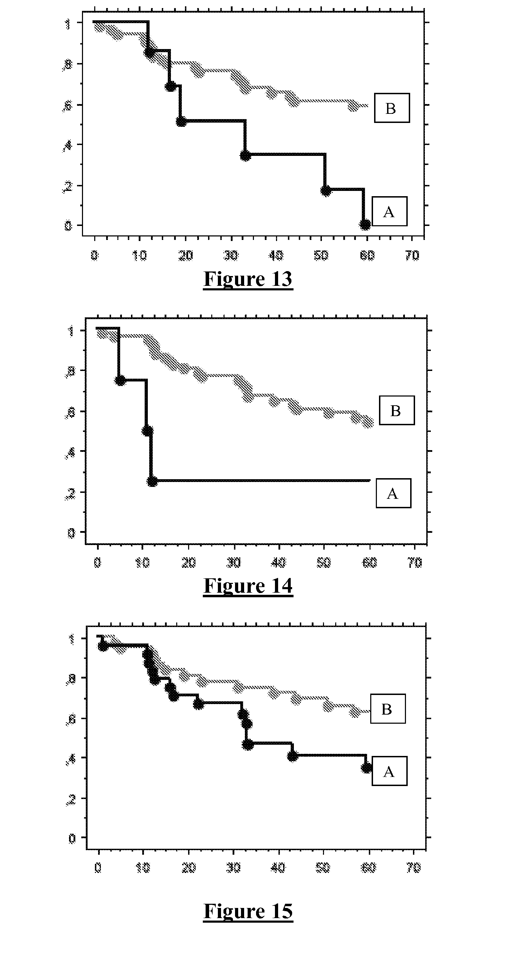

**Figure 13** represents the cumulative survival rate (Kaplan Mayer curve) over 60 months of patients expressing (A) or not (B) the gene SEQ ID NO: 13 (gene 1161). Y- axis represents cumulative survival in %, X-axis represents time in months.
**Figure 14** represents the cumulative survival rate (Kaplan Mayer curve) over 60 months of patients expressing (A) or not (B) the gene SEQ ID NO: 11 (gene 356). Y- axis represents cumulative survival in %, X-axis represents time in months.
F**igure 15** represents the cumulative survival rate (Kaplan Mayer curve) over 60 months of patients expressing (A) or not (B) the gene SEQ ID NO: 10 (gene 160). Y- axis represents cumulative survival in %, X-axis represents time in months.

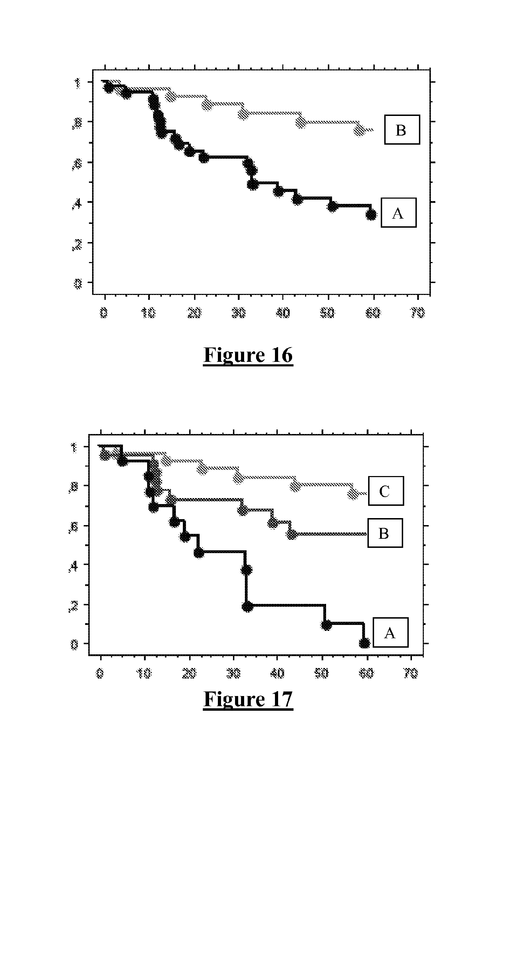

**Figure 16** represents the cumulative survival rate (Kaplan Mayer curve) over 60 months of patients expressing (A) or not (B) at least one of the following genes: SEQ ID NO: 8 (gene 266), SEQ ID NO: 9 (gene 442), SEQ ID NO: 10 (gene 160), SEQ ID NO: 11 (gene 356), SEQ ID NO: 12 (gene 440) and SEQ ID NO : 13 (gene 1161). Y- axis represents cumulative survival in %, X-axis represents time in months.
**Figure 17** represents the cumulative survival rate (Kaplan Mayer curve) over 60 months of patients expressing one (B), or two or more (A) or no (C) genes chosen among SEQ ID NO: 8 (gene 266), SEQ ID NO: 9 (gene 442), SEQ ID NO: 10 (gene 160), SEQ ID NO: 11 (gene 356), SEQ ID NO: 12 (gene 440) and SEQ ID NO : 13 (gene 1161). Y- axis represents cumulative survival in %, X-axis represents time in months.

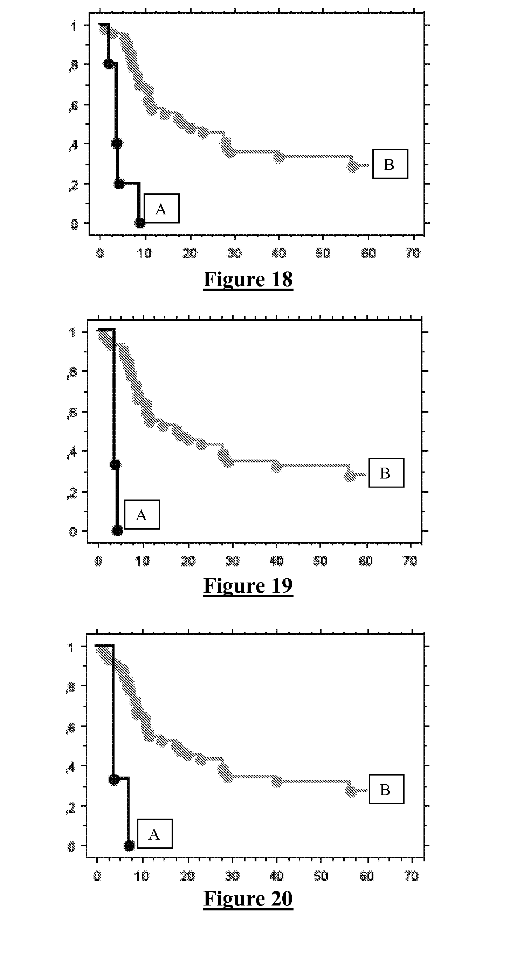

**Figure 18** represents the cumulative survival rate (Kaplan Mayer curve) over 60 months of patients expressing (A) or not (B) the gene SEQ ID NO: 22 (gene 117). Y- axis represents cumulative survival in %, X-axis represents time in months.
**Figure 19** represents the cumulative survival rate (Kaplan Mayer curve) over 60 months of patients expressing (A) or not (B) the gene SEQ ID NO: 21 (gene 118). Y- axis represents cumulative survival in %, X-axis represents time in months.
**Figure 20** represents the cumulative survival rate (Kaplan Mayer curve) over 60 months of patients expressing (A) or not (B) the gene SEQ ID NO: 18 (gene 144). Y- axis represents cumulative survival in %, X-axis represents time in months.

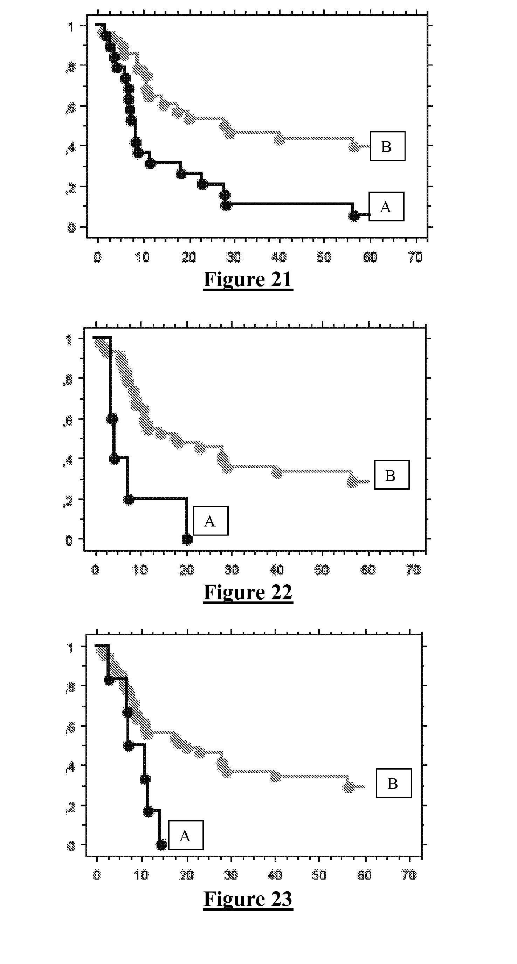

**Figure 21** represents the cumulative survival rate (Kaplan Mayer curve) over 60 months of patients expressing (A) or not (B) the gene SEQ ID NO: 16 (gene 424). Y- axis represents cumulative survival in %, X-axis represents time in months.
**Figure 22** represents the cumulative survival rate (Kaplan Mayer curve) over 60 months of patients expressing (A) or not (B) the gene SEQ ID NO: 19 (gene 437). Y- axis represents cumulative survival in %, X-axis represents time in months.
**Figure 23** represents the cumulative survival rate (Kaplan Mayer curve) over 60 months of patients expressing (A) or not (B) the gene SEQ ID NO: 17 (gene 125). Y- axis represents cumulative survival in %, X-axis represents time in months.

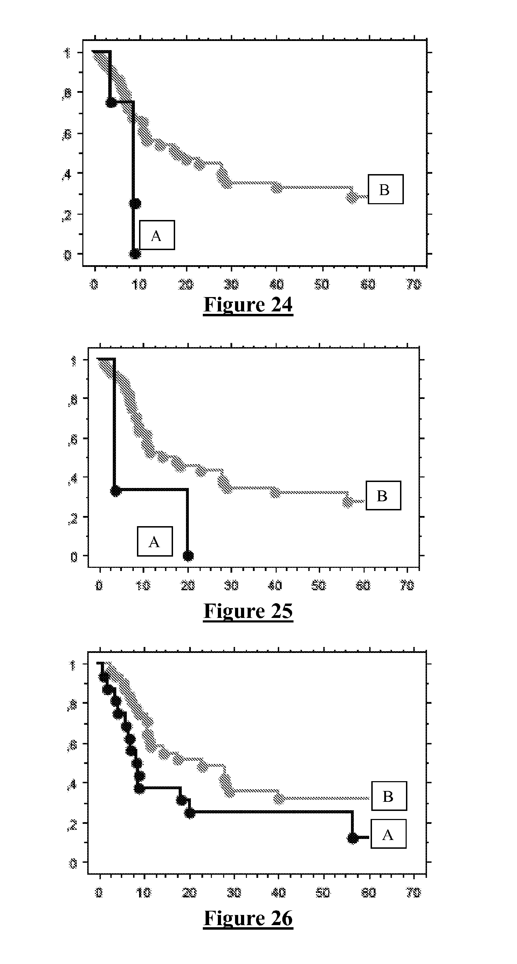

**Figure 24** represents the cumulative survival rate (Kaplan Mayer curve) over 60 months of patients expressing (A) or not (B) the gene SEQ ID NO: 20 (gene 766). Y- axis represents cumulative survival in %, X-axis represents time in months.
**Figure 25** represents the cumulative survival rate (Kaplan Mayer curve) over 60 months of patients expressing (A) or not (B) the gene SEQ ID NO: 14 (gene 71). Y- axis represents cumulative survival in %, X-axis represents time in months.
**Figure 26** represents the cumulative survival rate (Kaplan Mayer curve) over 60 months of patients expressing (A) or not (B) the gene SEQ ID NO: 23 (gene 390). Y- axis represents cumulative survival in %, X-axis represents time in months.

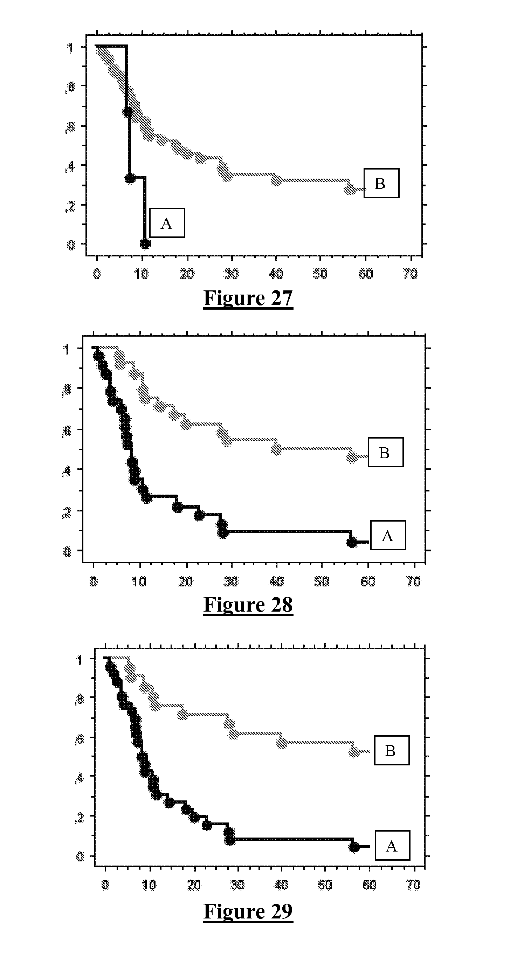

**Figure 27** represents the cumulative survival rate (Kaplan Mayer curve) over 60 months of patients expressing (A) or not (B) the gene SEQ ID NO: 15 (gene 1165). Y- axis represents cumulative survival in %, X-axis represents time in months.
**Figure 28** represents the cumulative survival rate (Kaplan Mayer curve) over 60 months of patients expressing (A) or not (B) at least one of the following genes: SEQ ID NO: 16 (gene 424), SEQ ID NO: 18 (gene 144), SEQ ID NO: 21 (gene 118) and SEQ ID NO: 22 (gene 117). Y- axis represents cumulative survival in %, X-axis represents time in months.
**Figure 29** represents the cumulative survival rate (Kaplan Mayer curve) over 60 months of patients expressing (A) or not (B) at least one of the following genes: SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.

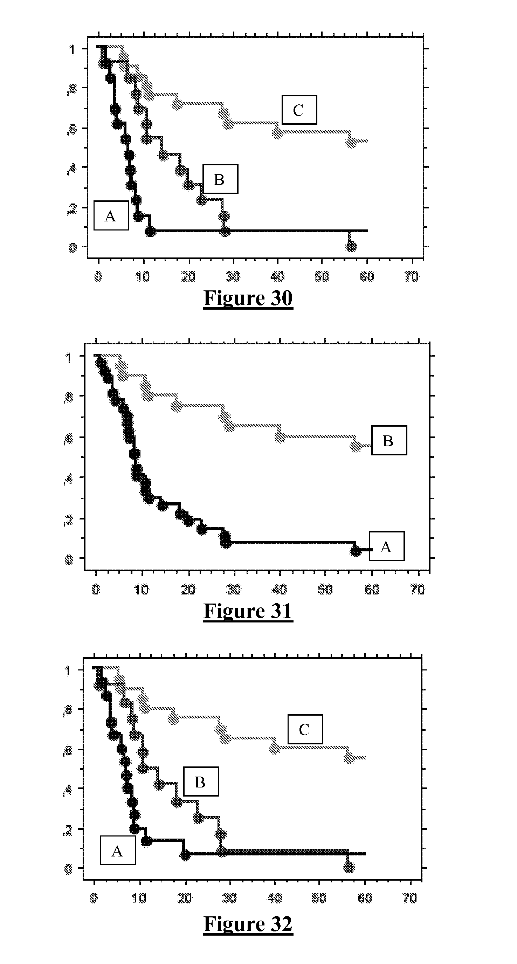

**Figure 30** represents the cumulative survival rate (Kaplan Mayer curve) over 60 months of patients expressing one (B), or two or more (A) or no (C) genes chosen among SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.
**Figure 31** represents the cumulative survival rate (Kaplan Mayer curve) over 60 months of patients expressing (A) or not (B) at least one of the following genes: SEQ ID NO : 14 (gene 71), SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.
**Figure 32** represents the cumulative survival rate (Kaplan Mayer curve) over 60 months of patients expressing one (B), or two or more (A) or no (C) genes chosen among SEQ ID NO : 14 (gene 71), SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.

### EXAMPLE

This work is based on a group of **23 genes**, which can be used to establish the **survival prognosis of lung tumour patients.** All these genes are actively repressed and silent in normal adult somatic cells, since their expression in strictly restricted to placenta or male germinal cells. The inventors have demonstrated that the aberrant expression in malignant cells of at least one of these genes is associated with significantly poorer prognosis for lung cancer patients. Moreover, the detection of the expression of several combinations of these genes allows predicting prognosis in lung tumour patients with higher significance and accuracy than with individual genes.

### I- Methodological approach

The following procedure allowed identifying the genes mentioned above.

### Overview

The expression of the 497 testis- and placenta- specific genes was studied in a series of 271 lung cancer samples by extracting the corresponding expression data from genome-wide transcriptomic data (the latter were obtained by C. and E. Brambilla supported by the Ligue CIT program (Carte d'Identite des Tumeurs)).

For each gene, the patients were divided into two groups,
- those expressing the gene,
- and those not expressing the gene.

For each of the 497 genes of the list, the global and disease free survival probabilities were compared between the patients expressing the gene and those not expressing the gene.

This was done considering the whole period of the study, as well as for 10 years, 5 years and 2.5 years of follow-up.

This was performed considering patients of the main histological subtypes of this population (ADK=adenocarcinoma; BAS= basaloid; LCNE=Large cell neuroendocrine; SQC=squamous cell tumour).

The genes discriminating the patients with good or bad prognosis with a significance corresponding to a p value =<0.05 (Logrank p value, obtained when comparing cumulative global survival and/or disease free survival over 5 years between patients expressing or not expressing the gene) were selected as candidate prognosis markers.

For all these genes, the correlation between their expression and prognosis was validated in at least one of the two following lung published cancer transcriptomic studies with survival clinical data using the same Affymetrix technology (website GEO: http://www.ncbi.nlm.nih.gov/geo, respectively GSE4576 and GSE8894).

These studies were selected as external populations of lung cancer patients in order to validate the survival data obtained by analysing the transcriptomic data of the Brambilla study.

### Detailed procedure

### 1- Establishment of a list of placenta and testis restricted genes and analysis of their aberrant expression in lung cancer patients

1a- A list of 497 human genes whose expression was restricted to placenta or male germ cells was established as defined in W02009/121878 . These genes are never expressed in normal adult somatic tissues (adult somatic tissues comprise all tissues except germinal cells, foetal tissues and placenta).
1b- Expression data were extracted from a series of 112 normal adult somatic tissues randomly selected from a genome wide study of normal human tissues (GSE3526 on GEO, this study was chosen because it uses the same probes and measurement technology to detect gene expression as the Brambilla study: Affymetrix Human Genome U133 Plus 2.0 Array). The CEL files (raw data) from the control samples were downloaded from GEO. They were entered in the Genespring software and normalized (RMA algorithm) simultaneously with the CEL files from the Brambilla study.
1c- For each of the 497 genes/probes, the mean hybridization intensity signal value of the 112 control samples and 2 standard deviation was defined as the threshold for expression. This threshold was used to distinguish between the cancer samples expressing the gene and those not expressing the gene.

The measurement of expression of the genes using Affymetrix microarrays involves the hybridization of fluorescence labeled cDNAs from each tissue sample on microarrays containing gene-specific probes, the fluorescence intensity signal corresponding to each probe of the microarray is measured and changed into a raw value. The absolute value of the fluorescence intensity signal is highly variable and probe-dependent (different probes corresponding to the same gene can give different intensities of fluorescence). Therefore, on the basis of these absolute fluorescence intensity values it is generally not possible to determine whether a gene is expressed or not, and commonly people use this technique to assess variations of expression between samples.

The definition of a precise threshold for expression was possible because the selected 497 genes are NOT expressed in any normal adult somatic tissue. Therefore the signal values obtained in the 112 normal adult somatic control samples give a high confidence set of values corresponding to the background noise signal, which allow further analyses.

A threshold signal value for expression could not have been defined for genes, which do not have a restricted expression pattern.

Indeed in all these types of transcriptomic experiments the background noise signal value is highly dependent on the sequence of the probe. For instance several probes representative of the same gene generally give different signal values. In the case of non-restricted genes (most genes have a pattern of expression, which is not restricted to germinal cells or placenta), it is therefore impossible to use these signal values as "absolute" indicators of the presence or absence of expression. However, one can compare expression levels between two groups of tissues.

Therefore, in this particular study, since the Inventors have previously demonstrated that all the studied 497 genes are NOT expressed in normal adult somatic tissues, the Inventors were able to define a threshold differentiating expression (ON) and non-expression (OFF). This is a specific key feature of their approach.
1d- Based on this threshold, the expression of each of the 497 genes in each of the samples was defined as negative (OFF) or positive (ON) as follow. In each cancer sample:
   - if the normalised signal value was above this threshold, the gene was considered as aberrantly expressed in this sample (gene ON),
   - if it was under this threshold, it was considered as not expressed (gene OFF).
1e- From the Brambilla study (271 cases of lung cancer), the Inventors found that 130 of the 497 genes were aberrantly expressed in at least 1% of these lung cancer cases.

### 2- Correlation between the expression of each individual gene (of the list of 130 genes) and the prognosis for survival in the lung cancer patients, and selection of 23 genes individually associated with the prognosis of a particular histological subtype (these subtypes being either ADK or BAS or LCNE or SQC).

2a- As a first step, using each of the 130 genes individually, we compared the global survival over a period of five years between the groups of patients expressing the gene (yes) versus those not expressing the gene (no). A Logrank Mantel-Cox test was performed and a p value was calculated. This analysis was performed with each one of the following populations: ADK cases (n=91), BAS cases (n=46), LCNE cases (n=47), SQC cases (n=62).
2b- **A total of 23 genes were selected, whose individual expression was significantly associated with a poorer prognosis (as measured by the cumulative global survival and/or disease-free survival over five years; p<0.05) in the Brambilla lung cancer study, as well as in at least one of the external validation populations (see table 1). The following table 1 lists the 23 genes associated with poor prognosis of specific histological subtypes of lung cancer, confirmed by external studies.**

Gene ID corresponds to the nomenclature of the Inventors, Type corresponds to the histological subtype, SEQ ID NO gene correspond to the nucleic acid sequence according to the invention, SEQ ID NO prot corresponds to the corresponding amino acid sequence, Gene name corresponds to the name of the genes, ADK_, SQC_ BAS_ and LCNE_ corresponds to the Logrank p value for each histological type : i.e. significance of difference in cumulative global survival probabilities over 5 years between patients expressing the gene and those not expressing the gene, nb of ADK_, SQC_ BAS_ and LCNE_ corresponds to the number of patients expressing the genes.

**Table 1.**

| **Gene ID** | **type** | **SEQ ID gene** | **SEQ ID prot** | **Gene Name** | **ADK_** | **nbADK+ (/91)** | **BAS_** | **nb BAS+(/46)** | **LCNE_** | **nb LCNE+ (/47)** | **SQC_** | **nb SQC+ (/62)** |
|---|---|---|---|---|---|---|---|---|---|---|---|---|
| 1292 | **BAS** | **1** | **24** | KRTAP21-1 | | | **0,068** | 7 | | | | |
| 1261 | **BAS** | **2** | **25** | C12orf37 | | | **0,033** | 4 | | | | |
| 417 | **BAS** | **3** | **26** | WBSCR28 | | | **0,021** | 3 | | | | |
| 391 | **BAS** | **4** | **27** | RBM46 | | | 0,017 | 7 | | | | |
| 1371 | **ADK** | **5** | **28** | DKFZp761D1918 | **0,033** | 10 | | | | | | |
| 222 | **ADK** | **6** | **29** | NBPF4 | **0,007** | 3 | | | | | | |
| 59 | **ADK** | **7** | **30** | ADAM12 | **0,007** | 38 | | | | | | |
| 266 | **SQC** | **8** | **31** | CDNA clone IMAGE:5299000 | | | | | | | **0,01** | 9 |
| 442 | **SQC** | **9** | **32** | SOX30 | | | | | | | **0,009** | 9 |
| 160 | **SQC** | **10** | **33** | CDNA clone IMAGE:5296886 | | | | | | | **0,048** | 25 |
| 356 | **SQC** | **11** | **34** | ASZ1 | | | | | | | **0,021** | 4 |
| 440 | **SQC** | **12** | **35** | SLC2A14 | | | | | | | **<0,0001** | 4 |
| 1161 | **SQC** | **13** | **36** | COX8C | | | | | | | **0,017** | 7 |
| 71 | **LCNE** | **14** | **37** | TKTL2 | | | | | **0,052** | 3 | | |
| 1165 | **LCNE** | **15** | **38** | TPTEps1 | | | | | **0,07** | 3 | | |
| 424 | **LCNE** | **16** | **39** | C9orf11 | | | | | **0,002** | 19 | | |
| 125 | **LCNE** | **17** | **40** | PIWIL1 | | | | | **0,02** | 6 | | |
| 144 | **LCNE** | **18** | **41** | C19orf41 | | | | | | | **0,0003** | 3 |
| 437 | **LCNE** | **19** | **42** | RNF17 | | | | | | | **0,003** | 5 |
| 766 | **LCNE** | **20** | **43** | GALNTL5 | | | | | | | **0,028** | 4 |
| 118 | **LCNE** | **21** | **44** | KISS1 | | | | | | | **<0,0001** | 3 |
| 117 | **LCNE** | **22** | **45** | IGFBP1 | | | | | | | **<0,0001** | 5 |
| 390 | **LCNE** | **23** | **46** | MAGEB6 | | | | | | | **0,068** | 16 |

2c- A detailed **quantitative evaluation of the prognosis** is given in **table 2A-D,** using each of the 23 genes associated with poor prognosis in all lung cancer histological types. The corresponding Kaplan Meyer survival curves obtained using each of these genes are represented in figures.

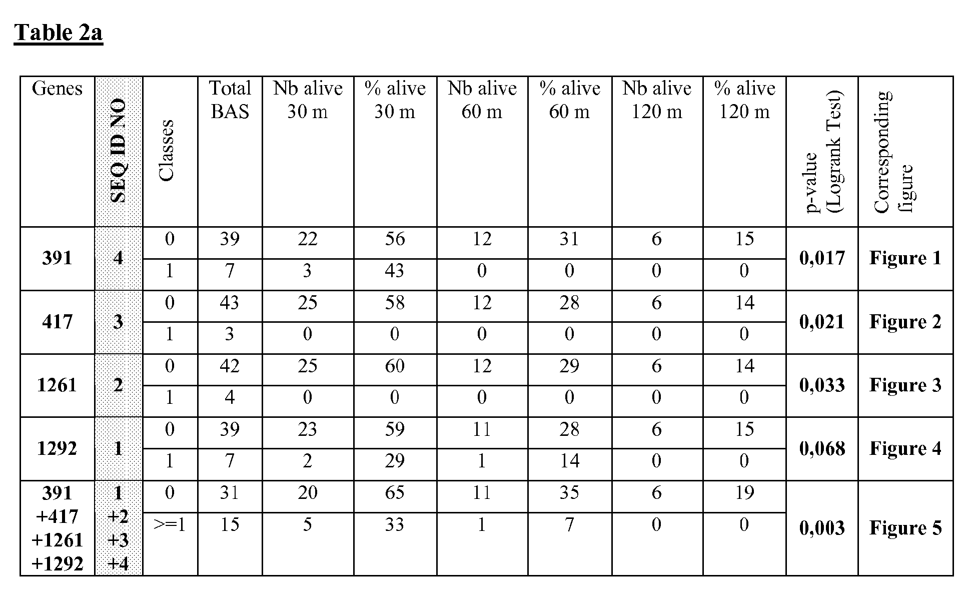

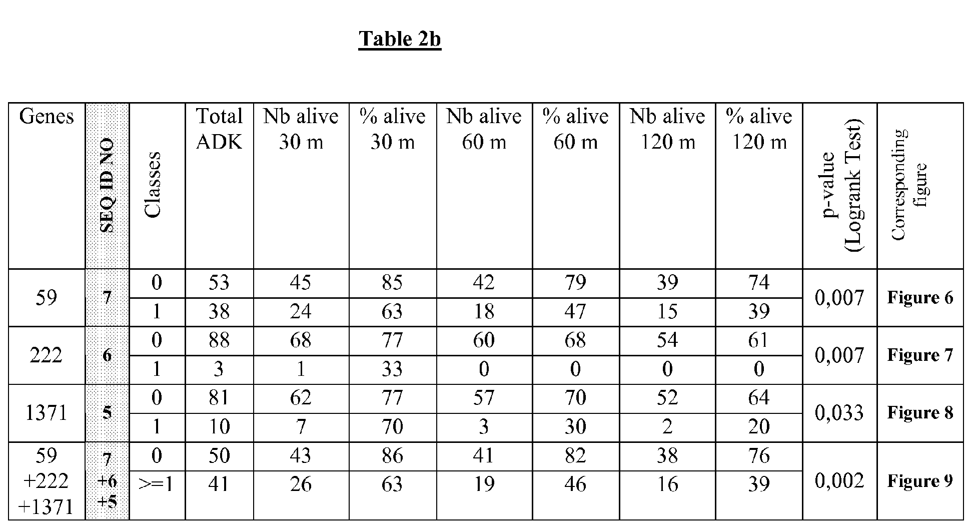

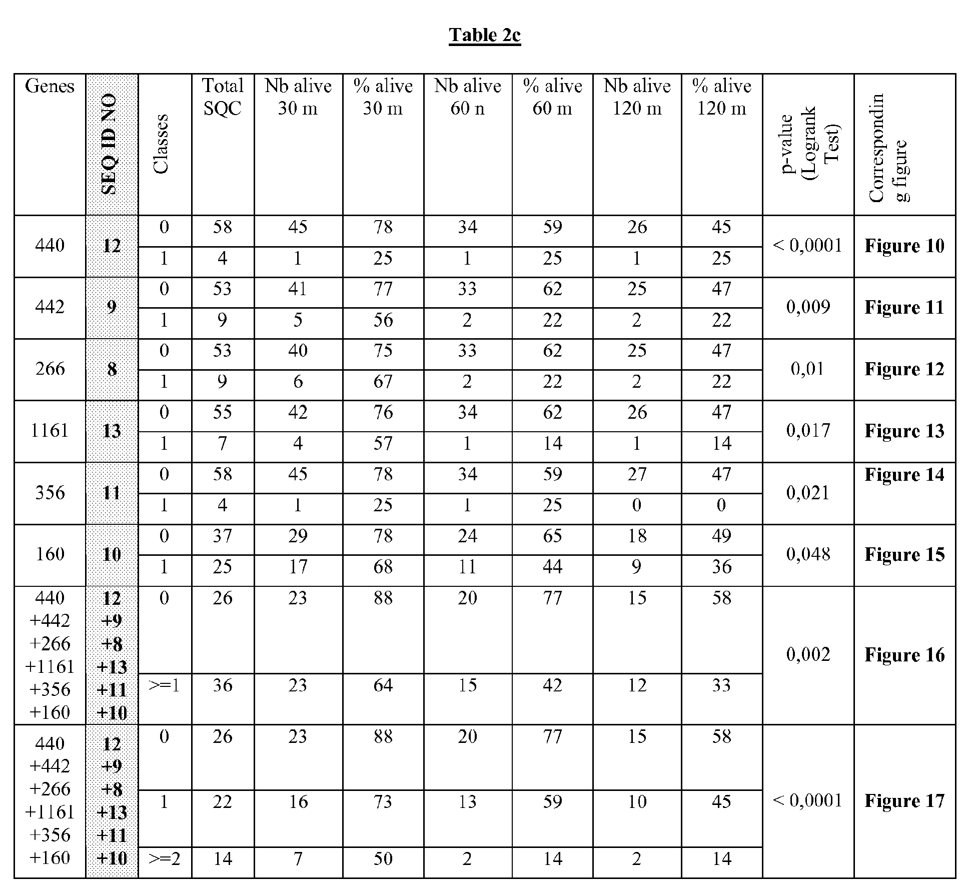

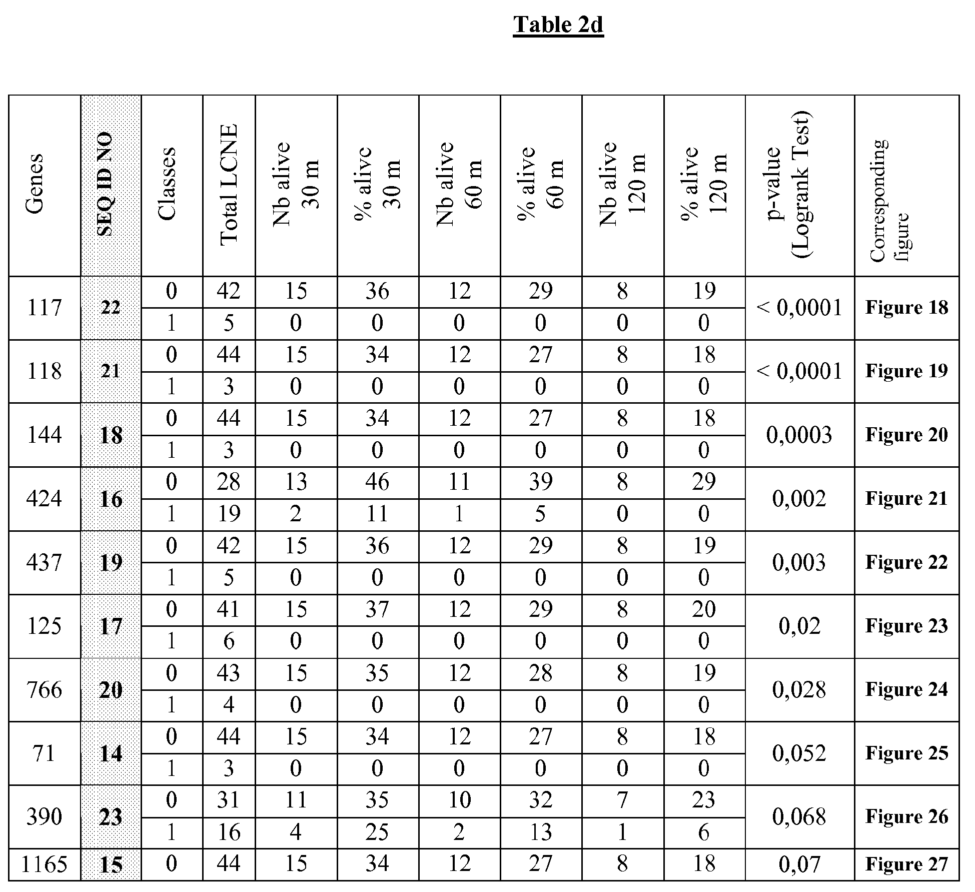

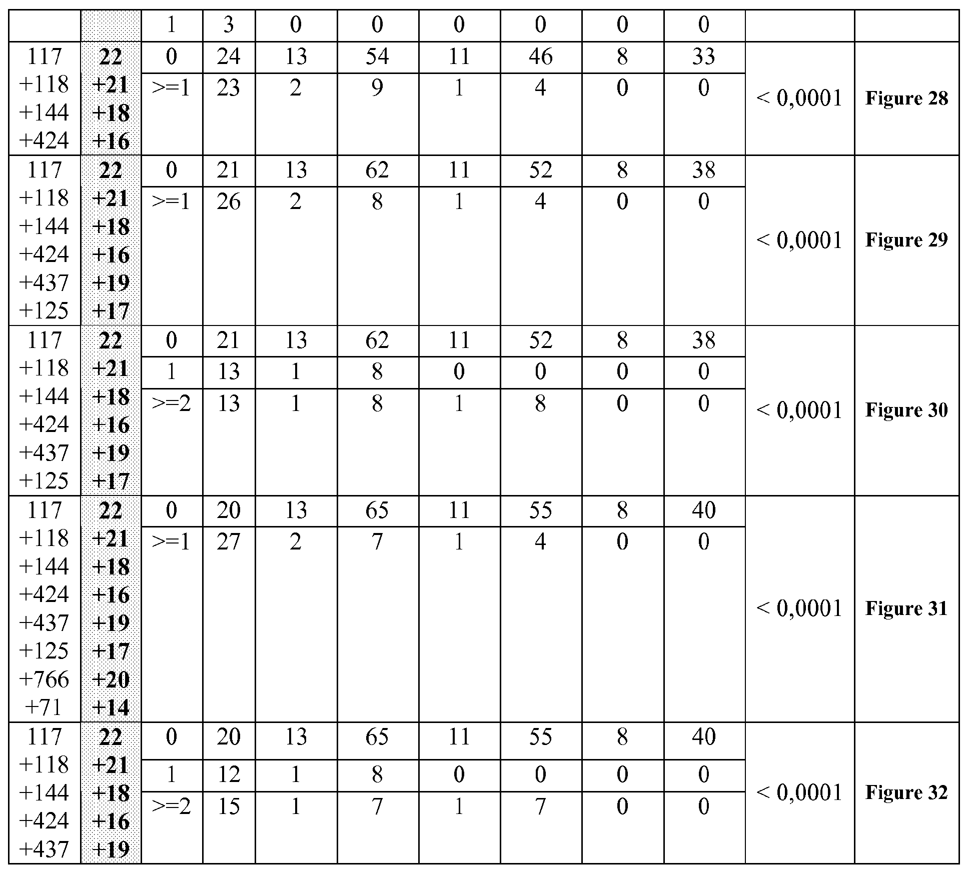

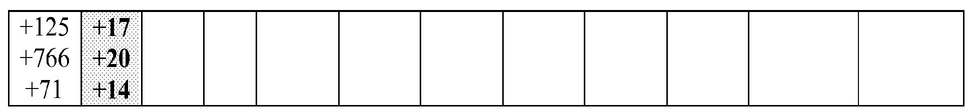

Legend of tables 2 A-D
Gene represents the Inventors nomenclature of genes
SEQ ID NO corresponds to the nucleic acid molecules according to the invention
Classes correspond to Lung cancer patients not expressing (=0) or expressing (=1) the gene. In the case of combinations of several genes, some patients could express only one gene or at least one gene of the combination (=1), only 2 genes or 2 genes or more of the combination (=2) etc...
Total BAS/ADK/SQC or LCNE corresponds to the number of patients of this class (considering the whole Brambilla study with 271 cases, including all histological types)
% or nb alive 30, 60, 120 month corresponds to percentage or number of patients of this class alive after 30, 60, 120 month p-value corresponds to the significance of the difference in cumulative global survival probabilities between the different classes of patients over 2.5, 5 and 10 years (Logrank Test, considering survival curves of all the classes of patients). Corresponding figures corresponds to the Kaplan Meyer curves.

### 4- Twenty three genes (allow an accurate prognosis in lung cancer patients with specific histological subtypes, either individually or in association

4a- Three genes, individually or in association, allow defining groups of different prognosis among ADK patients (table 2B).
4b- Four genes, individually or in association, allow defining groups of different prognosis among BAS patients (table 2A).
4c- Eleven genes, individually or in association, allow defining groups of different prognosis among LCNE patients (table 2C).
4d- Six genes, individually or in association, allow defining groups of different prognosis among SQC patients (table 2D).

### CONCLUSIONS

Using 23 genes aberrantly expressed in lung cancer, the Inventors are able to assess prognosis four lung cancer patients diagnosed with one of the four main histological subtypes of non-small cell lung cancer, including adenocarcinoma, squamous cell carcinoma, large cell neuro endocrine and basaloid tumours. These 23 genes therefore provide a basis for developing easy to use diagnosis tests for histologically classified lung tumours, in order to assess the survival probability of lung cancer patients independently from other prognosis parameters (histology, tumour size and stage...) and adapt therapeutic strategies accordingly.

## Claims

1. Use of at least one element chosen among:
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
a. at least one gene belongs to a first set A of 4 genes comprising or consisting of the nucleic acid sequences SEQ ID NO: 1 to 4,
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13, and
d. at least one gene belongs to a fourth set D of 9 genes comprising or consisting of the nucleic acid sequences SEQ ID NO: 14-23,
◆ at least 4 proteins being such that
a. at least one protein belongs to a first set AP of 4 proteins comprising or consisting of the amino acid sequences SEQ ID NO: 24-27,
b. at least one protein belongs to a second set BP of 3 proteins comprising or consisting of the amino acid sequences SEQ ID NO: 28-30, and
c. at least one protein belongs to a third set CP of 6 proteins comprising or consisting of the amino acid sequences SEQ ID NO: 31-36,
d. at least one protein belongs to a fourth set BP of 9 proteins comprising or consisting of the amino acid sequences SEQ ID NO: 37-46,
◆ at least 4 antibodies directed against said 4 proteins being such that
a. at least one antibody that ineract with at least one protein that belongs to a first set AP of 4 proteins comprising or consisting of the amino acid sequences SEQ ID NO: 24-27,
b. at least one antibody that ineract with at least one protein that belongs a second set BP of 3 proteins comprising or consisting of the amino acid sequences SEQ ID NO: 28-30,
c. at least one antibody that ineract with at least one protein that belongs a third set CP of 6 proteins comprising or consisting of the amino acid sequences SEQ ID NO: 31-36, and
d. at least one antibody that ineract with at least one protein that belongs a fourth set BP of 9 proteins comprising or consisting of the amino acid sequences SEQ ID NO: 37-46,
for the implementation of a prognosis method, preferably *in vitro,* of the survival rate of a patient afflicted by a lung cancer, said lung cancer being previously classified as a lung tumour belonging from the group consisting of ADK, SQC, BAS et LCNE,
wherein
- the nucleic acid sequences SEQ ID NO: 1 to 4 and/or the amino acid sequences SEQ ID NO: 24 to 27 are expressed in BAS,
- the nucleic acid sequences SEQ ID NO: 5 to 7 and/or the amino acid sequences SEQ ID NO: 28 to 30 are expressed in ADK,
- the nucleic acid sequences SEQ ID NO: 8 to 13 and/or the amino acid sequences SEQ ID NO: 31 to 36 are expressed in SQC,
- the nucleic acid sequences SEQ ID NO: 14 to 23 and/or the amino acid sequences SEQ ID NO: 37 to 46 are expressed in LCNE,
said prognosis being such that:
either
■ if none of the 23 genes of said set is expressed, the patient survival rate during a period of time from 30 to 120 months after the diagnosis of said lung cancer is from about 59% to about 78% or more, and
■ if at least one gene of at least one set A, B, C or D is expressed , the patient survival rate during a period of time from 30 to 120 months after the diagnosis of said lung cancer is about from about 3% to about 70%,
or
■ if none of the 23 proteins of said set is expressed, the patient survival rate during a period of time from 30 to 120 months after the diagnosis of said lung cancer is from about 59% to about 78% or more, and
■ if at least one protein of at least one set AP, BP, CP or DP is expressed, the patient survival rate during a period of time from 30 to 120 months after the diagnosis of said lung cancer is about from about 3% to about 70%,
or
■ if none of the antibodies directed against said 23 proteins of said set is expressed, the patient survival rate during a period of time from 30 to 120 months after the diagnosis of said lung cancer is from about 59% to about 78% or more, and
■ if at least one antibody directed against one protein of of at least one set AP, BP, CP or DP is expressed, the patient survival rate during a period of time from 30 to 120 months after the diagnosis of said lung cancer is about from about 3% to about 70%,
wherein said gene, protein or antibody is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method , and said gene, protein or antibody is determined as being not expressed when:
- it is neither expressed in a sample of a patient afflicted by a lung cancer nor in a control sample of an healthy individual,
- or it is expressed in a sample of a patient afflicted by a lung cancer at a level substancially equal or inferior to the level in a control sample of an healthy individual.

2. Use of at least one element chosen among:
- at least 4 genes chosen among a group of 23 genes comprising or consisting of the nucleic acid sequences SEQ ID NO 1 to 23,
- or fragments of said genes
- or complementary sequences of said genes
- or sequences having at least 80% homology with said genes or fragment thereof,
- or protein coded by said genes, said proteins comprising or consisting of the nucleic acid sequences SEQ ID NO 24 to 46
- or fragments of said proteins,
- or antibodies directed against said proteins,
said at least 4 genes being such that
a. at least one gene belongs to a first set A of 4 genes comprising or consisting of the nucleic acid sequences SEQ ID NO: 1 to 4,
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13 , and
d. at least one gene belongs to a fourth set D of 9 genes comprising or consisting of the nucleic acid sequences SEQ ID NO: 14-23,
for the implementation of a prognosis method, preferably *in vitro,* of the survival rate of a patient afflicted by a lung cancer, said lung cancer being previously classified as a lung tumour belonging from the group consisting of ADK, SQC, BAS et LCNE,
wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
■ if none of the **23** genes of said set is expressed, the patient survival rate during a period of time from 30 to 120 months after the diagnosis of said lung cancer is from about 19% to about 88% , and
• if at least one gene of one of said sets A, B, C D is expressed, the patient survival rate during a period of time from 30 to 120 months after the diagnosis of said lung cancer is from about 0% to about 73%.

3. Use according to claim 2, wherein said prognosis is such that
■ when said tumor is ADK :
o if none of the 23 genes of said set is expressed, the patient survival rate is from about 76% to about 86%
o if at least one of the genes of the set B is expressed, the patient survival rate is from about 39% to about 63%,
■ when said tumor is BAS :
o if none of the 23 genes of said set is expressed, the patient survival rate is about 19% to about 65%
o if at least one of the genes of the set A is expressed, the patient survival rate is about 0% to about 33%,
■ when said tumor is SQC :
o if none of the 23 genes of said set is expressed, the patient survival rate is about 58% to about 88%
o if at least one of the genes of the set C is expressed, the patient survival rate is from about 33% to about 64%
■ when said tumor is LCNE :
o if none of the 23 genes of said set is expressed, the patient survival rate is from about 40% to about 65%
o if at least one of the genes of the set D is expressed, the patient survival rate is from about 0% to 7%.

4. Use according to claim 2 or 3, wherein
■ when said tumor is SQC :
o if none of the 23 genes of said set is expressed, the patient survival rate is about 58% to about 88%
o if one of the genes of the set C is expressed, the patient survival rate is from about 45% to about 73%,
o if at least two of the genes of the set C are expressed, the patient survival rate is from about 14%, to about 50%.

5. Use according to anyone of claims 2 to 4, wherein during a period of time of 120 months from the diagnosis of said lung cancer, wherein
■ when said tumor is ADK :
o if none of the 23 genes of said set is expressed, the patient survival rate is about 76%
o if at least one of the genes of the set B is expressed, the patient survival rate is about 39%,
■ when said tumor is BAS :
o if none of the 23 genes of said set is expressed, the patient survival rate is about 19%
o if at least one of the genes of the set A is expressed, the patient survival rate is about 0%,
■ when said tumor is SQC :
o if none of the 23 genes of said set is expressed, the patient survival rate is about 58%
o if at least one of the genes of the set C is expressed, the patient survival rate is about 33%,
■ when said tumor is LCNE :
o if none of the 23 genes of said set is expressed, the patient survival rate is about 40%
o if at least one of the genes of the set D is expressed, the patient survival rate is about 0%.

6. Use according to of claims 5, wherein
■ when said tumor is SQC :
o if none of the 23 genes of said set is expressed, the patient survival rate is about 58%
o if one of the genes of the set C is expressed, the patient survival rate is about 45%,
o if at least two of the genes of the set C are expressed, the patient survival rate is about 14%.

7. Use according to anyone of claims 2 to 4, wherein during a period of time of 60 months from the diagnosis of said lung cancer, wherein
■ when said tumor is ADK :
o if none of the 23 genes of said set is expressed, the patient survival rate is about 82%
o if at least one of the genes of the set B is expressed, the patient survival rate is about 46%,
■ when said tumor is BAS :
o if none of the 23 genes of said set is expressed, the patient survival rate is about 35%
o if at least one of the genes of the set A is expressed, the patient survival rate is about 7%,
■ when said tumor is SQC :
o if none of the 23 genes of said set is expressed, the patient survival rate is about 77%
o if at least one of the genes of the set C is expressed, the patient survival rate is about 42%
■ when said tumor is LCNE :
o if none of the 23 genes of said set is expressed, the patient survival rate is about 55%
o if at least one of the genes of the set D is expressed, the patient survival rate is about 4%.

8. Use according to claim 7, wherein
■ when said tumor is SQC :
o if none of the 23 genes of said set is expressed, the patient survival rate is about 77%
o if one of the genes of the set C is expressed, the patient survival rate is about 59%,
o if at least two of the genes of the set C are expressed, the patient survival rate is about 14%.

9. Use according to anyone of claims 2 to 4, wherein during a period of time of 30 months from the diagnosis of said lung cancer, wherein
■ when said tumor is ADK :
o if none of the 23 genes of said set is expressed, the patient survival rate is about 86%
o if at least one of the genes of the set B is expressed, the patient survival rate is about 63%,
■ when said tumor is BAS :
o if none of the 23 genes of said set is expressed, the patient survival rate is about 65%
o if at least one of the genes of the set A is expressed, the patient survival rate is about 33%,
■ when said tumor is SQC :
o if none of the 23 genes of said set is expressed, the patient survival rate is about 88%
o if at least one of the genes of the set C is expressed, the patient survival rate is about 64%
■ when said tumor is LCNE :
o if none of the 23 genes of said set is expressed, the patient survival rate is about 65%
o if at least one of the genes of the set D is expressed, the patient survival rate is about 7%.

10. Use according to of claim 9, wherein
■ when said tumor is SQC :
o if none of the 23 genes of said set is expressed, the patient survival rate is about 88%
o if one of the genes of the set C is expressed, the patient survival rate is about 73%,
o if at least two of the genes of the set C are expressed, the patient survival rate is about 50%.

11. Prognosis method, preferably in vitro, of the survival rate of a patient afflicted by a lung tumour, from a biological sample containing said lung tumor belonging from the group consisting of ADK, SQC, BAS et LCNE, at a time from 30 to 120 months after the diagnosis of said lung cancer,
said method comprising a step of measuring, in said biological sample, the expression of
- at least 4 genes chosen among a group of 23 genes comprising or consisting of the nucleic acid sequences SEQ ID NO 1 to 23,
- or fragments of said genes
- or complementary sequences of said genes
said at least 4 genes being such that
a. at least one gene belongs to a first set A of 4 genes comprising or consisting of the nucleic acid sequences SEQ ID NO: 1 to 4
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13, and
d. at least one gene belongs to a fourth set D of 9 genes comprising or consisting of the nucleic acid sequences SEQ ID NO: 14-23,
wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
■ if none of the genes of said sets is expressed, the patient survival rate during a period of time from 30 to 120 months after the diagnosis of said lung cancer is from about 19% to about 88% , and
if at least one gene of one of said sets A, B, C D is expressed, the patient survival rate during a period of time from 30 to 120 months after the diagnosis of said lung cancer is from about 0% to about 73%.

12. Prognosis method, according to claim 11, wherein the step of measuring is carried out by using a technique chosen among the set consisting of:
a. Quantitative PCR,
b. DNA CHIP, and
c. Northern blot.

13. Prognosis method, according to claim 11 or 12, wherein the step of measuring is carried out by using nucleic acid molecules consisting of from 15 to 100 nucleotides molecules being complementary to said at least 4 genes.

14. Prognosis method, preferably *in vitro,* of the survival rate of a patient afflicted by a lung tumour, from a biological sample containing said lung tumor, at a time from 30 to 120 months after the diagnosis of said lung cancer,
said method comprising a step of measuring, in said biological sample, the expression of
- at least 4 proteins chosen among a set of 23 proteins coded by 23 genes comprising or consisting of the nucleic acid sequences SEQ ID NO 1 to 23,
- or fragments of said proteins,
said at least 4 proteins being such that
◆ at least 4 proteins being such that
a. at least one protein belongs to a first set AP of 4 proteins comprising or consisting of the amino acid sequences SEQ ID NO: 24-27,
b. at least one protein belongs to a second set BP of 3 proteins comprising or consisting of the amino acid sequences SEQ ID NO: 28-30,
c. at least one protein belongs to a third set CP of 6 proteins comprising or consisting of the amino acid sequences SEQ ID NO: 31-36,
d. at least one protein belongs to a fourth set BP of 9 proteins comprising or consisting of the amino acid sequences SEQ ID NO: 37-46,
wherein
- the amino acid sequences SEQ ID NO: 24 to 27 are expressed in BAS,
- the amino acid sequences SEQ ID NO: 28 to 30 are expressed in ADK,
- the amino acid sequences SEQ ID NO: 31 to 36 are expressed in SQC,
- the amino acid sequences SEQ ID NO: 37 to 46 are expressed in LCNE, said prognosis method being such that
■ if none of the proteins is expressed, the patient survival rate is from about 59% to about 78%, or more, and
■ if at least one protein of said set AP, BP, CP and DP is expressed, the patient survival rate from about 3% to about 70%.

15. Prognosis method, according to claim 14, wherein the step of measuring is carried out by using a technique chosen among the set consisting of:
a. western Blot,
b. ELISA,
c. Immunofluorescence, and
d. Immunohistochemistry.

16. Prognosis method, according to claim 14 or 15, wherein the step of measuring is carried out by using antibodies directed against said at least 2 proteins coded by said at least two genes.

17. Prognosis method, according to anyone of claims 11 to 16, further comprising a step of comparison of said measured expression to the expression in at least one control sample.
